# EUROPEAN PATENT APPLICATION

(11) **EP 4 729 509 A1**
(43) Date of publication of application: **22.04.2026**
(21) Application number: 24822829.8
(22) Date of filing: 14.06.2024
(51) Int. Cl.: C07C 333/04, C07C 229/16, C07C 219/06, C07C 219/08, A61K 9/127, C12N 15/11

(54) **IONIZABLE LIPID FOR MUSCLE-SPECIFIC DELIVERY**

(30) Priority: 16.06.2023 CN 202310720951; 16.06.2023 CN 202310723466; 29.12.2023 WO PCT/CN2023/143111; 26.01.2024 CN 202410115561; 01.03.2024 CN 202410239117; 11.04.2024 WO PCT/CN2024/087231; 12.04.2024 WO PCT/CN2024/087429; 12.04.2024 WO PCT/CN2024/087436
(71) Applicant: Beijing Jitai Life Sciences Ltd, Beijing 100094 (CN); Metis TechBio Co., Ltd., Beijing 102629 (CN)
(72) Inventor: SHI, Feng, Beijing 100094 (CN); ZHANG, Lin, Beijing 100094 (CN); FENG, Ruilu, Beijing 100094 (CN); LIU, Shaoli, Beijing 100094 (CN); LIU, Andong, Beijing 100094 (CN); MENG, Xiangxue, Beijing 100094 (CN); LEI, Jiani, Beijing 100094 (CN); WANG, Xue, Beijing 100094 (CN)
(74) Representative: Cabinet Becker et Associés
(86) International application number: PCT/CN2024/099393
(87) International publication number: WO 2024/255882

(57) **Abstract**

The present invention provides an ionizable lipid for muscle-specific delivery, and specifically relates to a compound of formula (I), or an isotopic variant, tautomer or stereoisomer thereof, or a pharmaceutically acceptable salt thereof. The present invention further provides a nanoparticle pharmaceutical composition comprising the compound, and a use of the compound and the composition thereof in muscle-specific nucleic acid delivery.

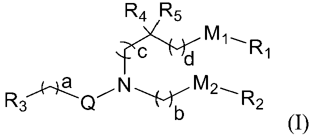

## Description

The present application claims priority to Chinese Application CN202310720951.5 filed on Jun. 16, 2023, Chinese Application CN202310723466.3 filed on Jun. 16, 2023, PCT Application PCT/CN2023/143111 filed on Dec. 29, 2023, Chinese Application CN202410115561.X filed on Jan. 26, 2024, Chinese Application CN202410239117.9 filed on Mar. 01, 2024, PCT Application PCT/CN2024/087231 filed on Apr. 11, 2024, PCT Application PCT/CN2024/087436 filed on Apr. 12, 2024, and PCT Application PCT/CN2024/087429 filed on Apr. 12, 2024, which are incorporated herein by reference in their entirety.

### TECHNICAL FIELD

The present disclosure relates to a class of ionizable cationic lipid compounds for muscle-specific delivery, or isotopic variants, tautomers, or stereoisomers thereof, or pharmaceutically acceptable salts thereof. The present disclosure also relates to a lipid nanoparticle and a pharmaceutical composition comprising the compound, and use of the lipid nanoparticle in muscle-specific delivery of a biologically active substance such as a nucleic acid (e.g., mRNA, siRNA, ASO, DNA, etc.).

### BACKGROUND

Gene therapy refers to introduction of exogenous genes into target cells to correct or compensate for gene defects or abnormalities in the cells, so as to achieve therapeutic goals. Over the past decades, mounting importance has been attached to related studies on the treatment of clinical diseases by means of gene therapy. In particular, siRNA-related drugs and mRNA vaccines have been approved by the FDA for clinical treatment in recent years, which has further boosted the research and related investments in the field of gene therapy. Nucleic acid substances are prone to degradation under the action of nucleases in organisms, and the nucleic acid substances themselves are negatively charged, making it difficult for them to enter cells through cell membranes. Lipid nanoparticles (LNPs), as a nucleic acid delivery material, possess the advantages of simple preparation, good biodegradability, no immunogenicity, good safety, and the like, and are one of the most dominant nucleic acid delivery systems at present. The main ingredients of LNP include a cationic lipid molecule, cholesterol, a neutral lipid, and a polyethylene glycol-conjugated lipid. Among them, the cationic lipid molecule plays a central role in an LNP delivery system, and its molecular structure exerts a determining effect on the aspects of delivery efficiency, targeting property, formulation stability, and the like of the whole liposome nanoparticle.

Currently, there are nearly a thousand monogenic diseases known to cause muscle dysfunction. The design of many LNPs, however, may inadvertently lead to expression of quantities of mRNAs in off-target tissues and organs (e.g., the liver, spleen, or heart), resulting in unnecessary side effects. Therefore, there is an urgent need to design LNPs for muscle-specific mRNA delivery.

### SUMMARY

The present disclosure develops a class of ionizable cationic lipid compounds for muscle-specific delivery, which can be used for delivering a variety of biologically active substances and possess high muscle-specific delivery efficiency. SM102 has been widely recognized in the field of muscle delivery, and LNP drugs for intramuscular administration based on SM102 have been launched. Unexpectedly, the cationic lipid of the present disclosure possesses better *in vivo* muscle delivery specificity than SM102.

The present disclosure provides a compound of formula (I), or an isotopic variant, tautomer, or stereoisomer thereof, or a pharmaceutically acceptable salt thereof: 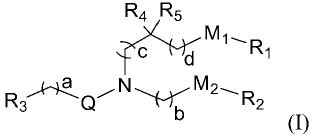 wherein each group is as defined in the present disclosure.

In another aspect, the present disclosure provides a nanoparticle composition comprising a lipid component, and optionally a load, wherein the lipid component comprises the compound of the present disclosure.

In another aspect, the present disclosure provides a pharmaceutical composition comprising the compound of the present disclosure or the nanoparticle composition of the present disclosure, and optionally a pharmaceutically acceptable auxiliary material, such as a carrier, an adjuvant, or a medium.

In another aspect, the present disclosure provides use of the compound of the present disclosure, the nanoparticle composition of the present disclosure, or the pharmaceutical composition of the present disclosure in the manufacture of a medicament for treating, diagnosing, or preventing a disease. In one embodiment, the medicament for treating, diagnosing, or preventing a disease is a therapeutic or prophylactic mRNA vaccine. In another aspect, the present disclosure provides use of the compound of the present disclosure, the nanoparticle composition of the present disclosure, or the pharmaceutical composition of the present disclosure in the manufacture of a medicament for delivering a load.

In another aspect, the present disclosure provides a method for treating, diagnosing, or preventing a disease in a subject, comprising administering to the subject the compound of the present disclosure, the nanoparticle composition of the present disclosure, or the pharmaceutical composition of the present disclosure.

In another aspect, the present disclosure provides the compound of the present disclosure, the nanoparticle composition of the present disclosure, or the pharmaceutical composition of the present disclosure for use in treating, diagnosing, and/or preventing a disease.

In another aspect, the present disclosure provides a method for delivering a load in a subject, comprising administering to the subject the compound of the present disclosure, the nanoparticle composition of the present disclosure, or the pharmaceutical composition of the present disclosure.

In another aspect, the present disclosure provides the compound of the present disclosure, the nanoparticle composition of the present disclosure, or the pharmaceutical composition of the present disclosure for use in delivering a load.

In a specific embodiment, the load is selected from one or more of a therapeutic agent, a prophylactic agent, and a diagnostic agent; preferably, the therapeutic agent, the prophylactic agent, or the diagnostic agent is a nucleic acid.

In a more specific embodiment, the nucleic acid is selected from one or more of ASO, RNA, and DNA.

In a more specific embodiment, the RNA is selected from one or more of a small interfering RNA (siRNA), a short hairpin RNA (shRNA), an antisense RNA (aRNA), a messenger RNA (mRNA), a long non-coding RNA (lncRNA), a micro RNA (miRNA), a small activating RNA (saRNA), a multimeric coding nucleic acid (MCNA), a polymeric coding nucleic acid (PCNA), a guide RNA (gRNA), a CRISPR RNA (crRNA), and a ribozyme, preferably an mRNA, and more preferably a modified mRNA.

### Definitions

### Chemical definitions

Definitions of specific functional groups and chemical terms are described in detail below.

When a range of values is given, it is intended to include each value and every subrange within the range. For example, "C₁₋₆ alkyl" includes C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅, and C₅₋₆ alkyl.

"C₁₋₂₀ alkyl" refers to a linear or branched saturated hydrocarbon group having 1 to 20 carbon atoms. In some embodiments, C₄₋₂₀ alkyl, C₆₋₁₄ alkyl, C₇₋₁₂ alkyl, C₈₋₁₂ alkyl, C₉₋₁₂ alkyl, C₇₋₁₁ alkyl, C₈₋₁₁ alkyl, C₉₋₁₁ alkyl, C₁₀₋₁₁ alkyl, C₄₋₁₀ alkyl, C₆₋₁₀ alkyl, C₇₋₁₀ alkyl, C₈₋₁₀ alkyl, C₉₋₁₀ alkyl, C₁₀ alkyl, C₈₋₉ alkyl, C₆₋₉ alkyl, C₇₋₉ alkyl, C₃₋₉ alkyl, C₄₋₉ alkyl, C₉ alkyl, C₁₀ alkyl, C₁₁ alkyl, C₂₋₈ alkyl, C₃₋₈ alkyl, C₄₋₈ alkyl, C₅₋₈ alkyl, C₆₋₈ alkyl, C₇₋₈ alkyl, C₈ alkyl, C₆₋₇ alkyl, C₇ alkyl, C₄₋₆ alkyl, C₁₋₂₀ alkyl, C₁₋₁₄ alkyl, C₂₋₁₄ alkyl, C₁₋₁₃ alkyl, C₁₋₁₂ alkyl, C₁₋₁₀ alkyl, C₁₋₉ alkyl, C₁₋₈ alkyl, C₁₋₇ alkyl, C₂₋₇ alkyl, C₃₋₇ alkyl, C₄₋₇ alkyl, C₅₋₇ alkyl, C₁₋₆ alkyl, C₂₋₆ alkyl, C₅₋₆ alkyl, C₁₋₅ alkyl, C₄₋₅ alkyl, C₅ alkyl, C₁₋₄ alkyl, C₂₋₄ alkyl, C₁₋₃ alkyl, C₂₋₃ alkyl, C₁₋₂ alkyl, and Me are preferred. Examples of C₁₋₆ alkyl include: methyl (C₁), ethyl (C₂), n-propyl (C₃), isopropyl (C₃), n-butyl (C₄), tert-butyl (C₄), sec-butyl (C₄), isobutyl (C₄), n-pentyl (C₅), 3-pentyl (C₅), pentyl (C₅), neopentyl (C₅), 3-methyl-2-butyl (C₅), tert-pentyl (C₅), and n-hexyl (C₆). The term "C₁₋₆ alkyl" also includes heteroalkyl in which one or more (e.g., 1, 2, 3, or 4) carbon atoms are replaced by a heteroatom (e.g., oxygen, sulfur, nitrogen, boron, silicon, or phosphorus). The alkyl group may be optionally substituted with one or more substituents, e.g., 1 to 5 substituents, 1 to 3 substituents, or 1 substituent. Conventional abbreviations of alkyl include: Me (-CH₃), Et (-CH₂CH₃), iPr (-CH(CH₃)₂), nPr (-CH₂CH₂CH₃), n-Bu (-CH₂CH₂CH₂CH₃), or i-Bu (-CH₂CH(CH₃)₂). In some embodiments, the alkyl is preferably linear alkyl.

"C₂₋₁₃ alkenyl" refers to a linear or branched hydrocarbon group having 2 to 13 carbon atoms and at least one carbon-carbon double bond. "C₄₋₂₀ alkenyl" refers to a linear or branched hydrocarbon group having 4 to 20 carbon atoms and at least one carbon-carbon double bond. In some embodiments, C₄₋₁₄ alkenyl, C₆₋₁₄ alkenyl, C₇₋₁₂ alkenyl, C₄₋₁₀ alkenyl, C₂₋₁₀ alkenyl, C₂₋₉ alkenyl, C₂₋₆ alkenyl, and C₂₋₄ alkenyl are preferred. Examples of C₂₋₆ alkenyl include: vinyl (C₂), 1-propenyl (C₃), 2-propenyl (C₃), 1-butenyl (C₄), 2-butenyl (C₄), butadienyl (C₄), pentenyl (C₅), pentadienyl (C₅), hexenyl (C₆), and the like. The term "C₂₋₆ alkenyl" also includes heteroalkenyl in which one or more (e.g., 1, 2, 3, or 4) carbon atoms are replaced by a heteroatom (e.g., oxygen, sulfur, nitrogen, boron, silicon, or phosphorus). The alkenyl group may be optionally substituted with one or more substituents, e.g., 1 to 5 substituents, 1 to 3 substituents, or 1 substituent.

"C₂₋₁₃ alkynyl" refers to a linear or branched hydrocarbon group having 2 to 13 carbon atoms, at least one carbon-carbon triple bond, and optionally one or more carbon-carbon double bonds. "C₄₋₂₀ alkynyl" refers to a linear or branched hydrocarbon group having 4 to 20 carbon atoms, at least one carbon-carbon triple bond, and optionally one or more carbon-carbon double bonds. In some embodiments, C₄₋₁₄ alkynyl, C₆₋₁₄ alkynyl, C₇₋₁₂ alkynyl, C₄₋₁₀ alkynyl, C₂₋₁₀ alkynyl, C₂₋₉ alkynyl, C₂₋₆ alkynyl, and C₂₋₄ alkynyl are preferred. Examples of C₂₋₆ alkynyl include, but are not limited to: ethynyl (C₂), 1-propynyl (C₃), 2-propynyl (C₃), 1-butynyl (C₄), 2-butynyl (C₄), pentynyl (C₅), hexynyl (C₆), and the like. The term "C₂₋₆ alkynyl" also includes heteroalkynyl in which one or more (e.g., 1, 2, 3, or 4) carbon atoms are replaced by a heteroatom (e.g., oxygen, sulfur, nitrogen, boron, silicon, or phosphorus). The alkynyl group may be optionally substituted with one or more substituents, e.g., 1 to 5 substituents, 1 to 3 substituents, or 1 substituent.

"C₁₋₂₀ alkylene" refers to a divalent group formed by removing another hydrogen from a C₁₋₂₀ alkyl group, which may be substituted or unsubstituted. In some embodiments, C₄₋₂₀ alkylene, C₆₋₁₄ alkylene, C₇₋₁₂ alkylene, C₈₋₁₂ alkylene, C₄₋₁₀ alkylene, C₇₋₁₁ alkylene, C₈₋₁₁ alkylene, C₈₋₁₀ alkylene, C₉₋₁₀ alkylene, C₈₋₉ alkylene, C₄₋₉ alkylene, C₆₋₉ alkylene, C₇₋₉ alkylene, C₉ alkylene, C₂₋₈ alkylene, C₅₋₈ alkylene, C₇₋₈ alkylene, C₄₋₆ alkylene, C₁₋₂₀ alkylene, C₁₋₁₄ alkylene, C₂₋₁₄ alkylene, C₁₋₁₃ alkylene, C₁₋₁₂ alkylene, C₁₋₁₀ alkylene, C₁₋₉ alkyl, C₁₋₈ alkylene, C₁₋₇ alkylene, C₂₋₇ alkylene, C₁₋₆ alkylene, C₂₋₆ alkylene, C₁₋₅ alkylene, C₅ alkylene, C₁₋₄ alkylene, C₂₋₄ alkylene, C₁₋₃ alkylene, C₂₋₃ alkylene, C₁₋₂ alkylene, and methylene are preferred. Unsubstituted alkylene includes, but is not limited to: methylene (-CH₂-), ethylene (-CH₂CH₂-), propylene (-CH₂CH₂CH₂-), butylene (-CH₂CH₂CH₂CH₂-), pentylene (-CH₂CH₂CH₂CH₂CH₂-), hexylene (-CH₂CH₂CH₂CH₂CH₂CH₂-), and the like. Examples of substituted alkylene, for example, an alkylene substituted with one or more alkyl (methyl) groups, include, but are not limited to: substituted methylene (-CH(CH₃)- and -C(CH₃)₂-), substituted ethylene (-CH(CH₃)CH₂-, -CH₂CH(CH₃)-, -C(CH₃)₂CH₂-, and -CH₂C(CH₃)₂-), substituted propylene (-CH(CH₃)CH₂CH₂-, -CH₂CH(CH₃)CH₂-, -CH₂CH₂CH(CH₃)-, -C(CH₃)₂CH₂CH₂-, -CH₂C(CH₃)₂CH₂-, and - CH₂CH₂C(CH₃)₂-), and the like.

"C₂₋₁₃ alkenylene" refers to a divalent group formed by removing another hydrogen from a C₂₋₁₃ alkenyl group, which may be substituted or unsubstituted. "C₄₋₁₄ alkenylene" refers to a divalent group formed by removing another hydrogen from a C₄₋₁₄ alkenyl group, which may be substituted or unsubstituted. In some embodiments, C₆₋₁₄ alkenylene, C₄₋₁₀ alkenylene, C₂₋₁₀ alkenylene, C₂₋₉ alkenylene, C₂₋₆ alkenylene, and C₂₋₄ alkenylene are particularly preferred. Examples of unsubstituted alkenylene include, but are not limited to: vinylene (-CH=CH-) and propenylene (e.g., -CH=CHCH₂- and -CH₂-CH=CH-). Examples of substituted alkenylene, for example, an alkenylene substituted with one or more alkyl (methyl) groups, include, but are not limited to: substituted vinylene (-C(CH₃)=CH- and -CH=C(CH₃)-), substituted propenylene (-C(CH₃)=CHCH₂-, - CH=C(CH₃)CH₂-, -CH=CHCH(CH₃)-, -CH=CHC(CH₃)₂-, -CH(CH₃)-CH=CH-, -C(CH₃)₂-CH=CH-, -CH₂-C(CH₃)=CH-, and -CH₂-CH=C(CH₃)-), and the like.

"C₂₋₁₃ alkynylene" refers to a divalent group formed by removing another hydrogen from a C₂₋₁₃ alkynyl group, which may be substituted or unsubstituted. "C₄₋₁₄ alkynylene" refers to a divalent group formed by removing another hydrogen from a C₄₋₁₄ alkynyl group, which may be substituted or unsubstituted. In some embodiments, C₆₋₁₄ alkynylene, C₄₋₁₀ alkynylene, C₂₋₁₀ alkynylene, C₂₋₉ alkynylene, C₂₋₆ alkynylene, and C₂₋₄ alkynylene are particularly preferred. Examples of alkynylene include, but are not limited to: ethynylene (-C≡C-), substituted or unsubstituted propynylene (-C≡CCH₂-), and the like.

"C₀₋₆ alkylene" refers to a chemical bond and the "C₁₋₆ alkylene" described above, and "C₀₋₄ alkylene" refers to a chemical bond and the "C₁₋₄ alkylene" described above.

The term "variable A and variable B have a total length of x carbon atoms" means that the sum of the number of carbon atoms of the backbone in the group represented by variable A and the number of carbon atoms of the backbone in the group represented by variable B is x.

The term "the substitution site of Rₛ on R₂ is separated from M₂ by x carbon atom(s)" means that the sum of the number of carbon atoms (including the N atom replaced by -NR'-) between the site on the variable R₂ where substitution with the variable Rₛ takes place and M₂. The same applies to other cases. For example: 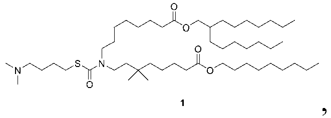 in compound 1, the substitution site of Rₛ on R₂ is separated from M₂ by 1 carbon atom.

"Halo" or "halogen" refers to fluorine (F), chlorine (Cl), bromine (Br), and iodine (I).

Thus, "C₁₋₁₀ haloalkyl" refers to the "C₁₋₁₀ alkyl" described above substituted with one or more halogen groups. In some embodiments, C₁₋₈ haloalkyl, C₁₋₆ haloalkyl, C₁₋₄ haloalkyl, and C₁₋₃ haloalkyl are particularly preferred, and C₁₋₂ haloalkyl is more preferred. Examples of haloalkyl include, but are not limited to: -CF₃, - CH₂F, -CHF₂, -CHFCH₂F, -CH₂CHF₂, -CF₂CF₃, -CCl₃, -CH₂Cl, -CHCl₂, 2,2,2-trifluoro-1,1-dimethyl-ethyl, and the like. The haloalkyl group may be substituted at any available connection site with, for example, 1 to 5 substituents, 1 to 3 substituents, or 1 substituent.

"C₃₋₁₄ cycloalkyl" or "3- to 14-membered cycloalkyl" refers to a non-aromatic cyclic hydrocarbon group having 3 to 14 ring carbon atoms and no heteroatoms and optionally containing 1, 2, or 3 double or triple bonds. In some embodiments, 3- to 10-membered cycloalkyl, 5- to 10-membered cycloalkyl, 3- to 8-membered cycloalkyl, 3- to 7-membered cycloalkyl, and 3- to 6-membered cycloalkyl are particularly preferred; 5- to 7-membered cycloalkyl, 4- to 6-membered cycloalkyl, 3- to 5-membered cycloalkyl, 3- to 4-membered cycloalkyl, and 5- to 6-membered cycloalkyl are more preferred; 5-membered cycloalkyl is more preferred; 6-membered cycloalkyl is more preferred; and cyclopropyl is more preferred. The cycloalkyl also includes ring systems in which the cycloalkyl ring described above is fused to one or more aryl or heteroaryl groups, where the connection site is on the cycloalkyl ring, and in such cases, the number of carbon still represents the number of carbon in the cycloalkyl system. The cycloalkyl also includes those in which substituents on any nonadjacent carbon atoms of the cycloalkyl ring described above are joined to form a bridged ring, together forming polycyclic alkane sharing two or more carbon atoms. The cycloalkyl also includes those in which substituents on the same carbon atom of the cycloalkyl ring described above are joined to form a ring, together forming polycyclic alkane sharing one carbon atom. Examples of cycloalkyl include, but are not limited to: cyclopropyl (C₃), cyclopropenyl (C₃), cyclobutyl (C₄), cyclobutenyl (C₄), cyclopentyl (Cs), cyclopentenyl (C₅), cyclohexyl (C₆), cyclohexenyl (C₆), cyclohexadienyl (C₆), cycloheptyl (C₇), cycloheptenyl (C₇), cycloheptadienyl (C₇), cycloheptatrienyl (C₇), and the like. The cycloalkyl group may be optionally substituted with one or more substituents, e.g., 1 to 5 substituents, 1 to 3 substituents, or 1 substituent.

"C₃₋₁₄ cycloalkylene" refers to a divalent group formed by removing another hydrogen from a C₃₋₁₄ cycloalkyl group, which may be substituted or unsubstituted. In some embodiments, C₃₋₁₀ cycloalkylene, C₃₋₇ cycloalkylene, C₃₋₆ cycloalkylene, C₃₋₅ cycloalkylene, and C₃₋₄ cycloalkylene are particularly preferred, e.g., cyclopropylene, cyclobutylene, cyclopentylene, or cyclohexylene, and cyclopropylene is more preferred.

"3- to 14-membered heterocyclyl" refers to a saturated or unsaturated group of a 3- to 14-membered non-aromatic ring system having ring carbon atoms and 1 to 5 ring heteroatoms, wherein each heteroatom is independently selected from nitrogen, oxygen, sulfur, boron, phosphorus, and silicon, and the group optionally contains 1, 2, or 3 double or triple bonds. In heterocyclyl containing one or more nitrogen atoms, the connection site may be a carbon or nitrogen atom, as long as the valency permits. In some embodiments, 3- to 10-membered heterocyclyl, which is a 3- to 10-membered non-aromatic ring system having ring carbon atoms and 1 to 5 ring heteroatoms, is preferred; in some embodiments, 5- to 10-membered heterocyclyl, which is a 5- to 10-membered non-aromatic ring system having ring carbon atoms and 1 to 5 ring heteroatoms, is preferred; in some embodiments, 3- to 8-membered heterocyclyl, which is a 3- to 8-membered non-aromatic ring system having ring carbon atoms and 1 to 4 ring heteroatoms, is preferred; in some embodiments, 3- to 7-membered heterocyclyl, which is a 3- to 7-membered non-aromatic ring system having ring carbon atoms and 1 to 4 ring heteroatoms, is preferred; 5- to 7-membered heterocyclyl, which is a 5- to 7-membered non-aromatic ring system having ring carbon atoms and 1 to 3 ring heteroatoms, is preferred; 3- to 6-membered heterocyclyl, which is a 3- to 6-membered non-aromatic ring system having ring carbon atoms and 1 to 3 ring heteroatoms, is preferred; 4- to 6-membered heterocyclyl, which is a 4- to 6-membered non-aromatic ring system having ring carbon atoms and 1 to 3 ring heteroatoms, is preferred; 5- to 6-membered heterocyclyl, which is a 5- to 6-membered non-aromatic ring system having ring carbon atoms and 1 to 3 ring heteroatoms, is more preferred; 5-membered heterocyclyl, which is a 5-membered non-aromatic ring system having ring carbon atoms and 1 to 3 ring heteroatoms, is preferred; 6-membered heterocyclyl, which is a 6-membered non-aromatic ring system having ring carbon atoms and 1 to 3 ring heteroatoms, is preferred. The heterocyclyl also includes ring systems in which the heterocyclyl ring described above is fused to one or more cycloalkyl groups, where the connection site is on the heterocyclyl ring, or ring systems in which the heterocyclyl ring described above is fused to one or more aryl or heteroaryl groups, where the connection site is on the heterocyclyl ring; in such cases, the number of ring members still represents the number of ring members in the heterocyclyl ring system. The heterocyclyl also includes those in which substituents on any nonadjacent carbon or nitrogen atoms of the heterocyclyl ring described above are joined to form a bridged ring, together forming polycyclic heteroalkane sharing two or more carbon or nitrogen atoms. The heterocyclyl also includes those in which substituents on the same carbon atom of the heterocyclyl ring described above are joined to form a ring, together forming polycyclic heteroalkane sharing one carbon atom. Examples of 3-membered heterocyclyl containing one heteroatom include, but are not limited to: aziridinyl, oxiranyl, and thiorenyl. Examples of 4-membered heterocyclyl containing one heteroatom include, but are not limited to: azetidinyl, oxetanyl, and thietanyl. Examples of 5-membered heterocyclyl containing one heteroatom include, but are not limited to: tetrahydrofuranyl, dihydrofuranyl, tetrahydrothienyl, dihydrothienyl, pyrrolidinyl, dihydropyrrolyl, and pyrrolyl-2,5-dione. Examples of 5-membered heterocyclyl containing two heteroatoms include, but are not limited to: pyrazolidinyl, dioxolanyl, oxasulfuranyl, disulfuranyl, and oxazolidin-2-one. Examples of 5-membered heterocyclyl containing three heteroatoms include, but are not limited to: triazolinyl, oxadiazolinyl, and thiadiazolinyl. Examples of 6-membered heterocyclyl containing one heteroatom include, but are not limited to: piperidinyl, tetrahydropyranyl, dihydropyridinyl, and thianyl. Examples of 6-membered heterocyclyl containing two heteroatoms include, but are not limited to: piperazinyl, morpholinyl, dithianyl, and dioxanyl. Examples of 6-membered heterocyclyl containing three heteroatoms include, but are not limited to: triazinanyl. Examples of 7-membered heterocyclyl containing one heteroatom include, but are not limited to: azepanyl, oxepanyl, and thiepanyl. Examples of 5-membered heterocyclyl fused to a C₆ aryl ring (also referred to herein as 5,6-bicyclic heterocyclyl) include, but are not limited to: indolinyl, isoindolinyl, dihydrobenzofuranyl, dihydrobenzothienyl, benzoxazolinonyl, and the like. Examples of 6-membered heterocyclyl fused to a C₆ aryl ring (also referred to herein as 6,6-bicyclic heterocyclyl) include, but are not limited to: tetrahydroquinolinyl, tetrahydroisoquinolinyl, and the like. The heterocyclyl also includes those in which the heterocyclyl described above shares one or two atoms with a cycloalkyl, heterocyclyl, aryl, or heteroaryl group to form bridged or spiro rings, where the shared atoms may be carbon or nitrogen atoms, as long as the valency permits. The heterocyclyl also includes those in which the heterocyclyl and heterocyclyl groups described above may be optionally substituted with one or more substituents, e.g., 1 to 5 substituents, 1 to 3 substituents, or 1 substituent.

"C₆₋₁₀ aryl" refers to a group of a monocyclic or polycyclic (e.g., bicyclic) 4n+2 aromatic ring system (e.g., having 6 or 10 π electrons shared in a cyclic arrangement) having 6-10 ring carbon atoms and no heteroatoms. In some embodiments, the aryl has six ring carbon atoms ("C₆ aryl"; e.g., phenyl). In some embodiments, the aryl has ten ring carbon atoms ("C₁₀ aryl"; such as naphthyl, e.g., 1-naphthyl and 2-naphthyl). The aryl also includes ring systems in which the aryl ring described above is fused to one or more cycloalkyl or heterocyclyl groups, and the connection site is on the aryl ring; in such cases, the number of carbon atoms still represents the number of carbon atoms in the aryl ring system. The aryl group may be optionally substituted with one or more substituents, e.g., 1 to 5 substituents, 1 to 3 substituents, or 1 substituent.

"5- to 14-membered heteroaryl" refers to a group of a 5- to 14-membered monocyclic or bicyclic 4n+2 aromatic ring system (e.g., having 6, 10, or 14 π electrons shared in a cyclic arrangement) having ring carbon atoms and 1-4 ring heteroatoms, wherein each heteroatom is independently selected from nitrogen, oxygen, and sulfur. In heteroaryl containing one or more nitrogen atoms, the connection site may be a carbon or nitrogen atom, as long as the valency permits. The heteroaryl bicyclic system may contain one or more heteroatoms in one or both rings. The heteroaryl also includes ring systems in which the heteroaryl ring described above is fused to one or more cycloalkyl or heterocyclyl groups, and the connection site is on the heteroaryl ring; in such cases, the number of carbon atoms still represents the number of carbon atoms in the heteroaryl ring system. In some embodiments, 5- to 10-membered heteroaryl, which is a 5- to 10-membered monocyclic or bicyclic 4n+2 aromatic ring system having ring carbon atoms and 1-4 ring heteroatoms, is preferred. In some other embodiments, 5- to 6-membered heteroaryl, which is a 5- to 6-membered monocyclic or bicyclic 4n+2 aromatic ring system having ring carbon atoms and 1-4 ring heteroatoms, is particularly preferred. Examples of 5-membered heteroaryl containing one heteroatom include, but are not limited to: pyrrolyl, furanyl, and thienyl. Examples of 5-membered heteroaryl containing two heteroatoms include, but are not limited to: imidazolyl, pyrazolyl, oxazolyl, isoxazolyl, thiazolyl, and isothiazolyl. Examples of 5-membered heteroaryl containing three heteroatoms include, but are not limited to: triazolyl, oxadiazolyl (e.g., 1,2,4-oxadiazolyl), and thiadiazolyl. Examples of 5-membered heteroaryl containing four heteroatoms include, but are not limited to: tetrazolyl. Examples of 6-membered heteroaryl containing one heteroatom include, but are not limited to: pyridinyl or pyridonyl. Examples of 6-membered heteroaryl containing two heteroatoms include, but are not limited to: pyridazinyl, pyrimidinyl, and pyrazinyl. Examples of 6-membered heteroaryl containing three or four heteroatoms include, but are not limited to: triazinyl and tetrazinyl. Examples of 7-membered heteroaryl containing one heteroatom include, but are not limited to: azepinyl, oxepinyl, and thiepinyl. Examples of 5,6-bicyclic heteroaryl include, but are not limited to: indolyl, isoindolyl, indazolyl, benzotriazolyl, benzothienyl, isobenzothienyl, benzofuranyl, benzisofuranyl, benzimidazolyl, benzoxazolyl, benzisoxazolyl, benzoxadiazolyl, benzothiazolyl, benzisothiazolyl, benzothiadiazolyl, indolizinyl, and purinyl. Examples of 6,6-bicyclic heteroaryl include, but are not limited to: naphthyridinyl, pteridinyl, quinolinyl, isoquinolinyl, cinnolinyl, quinoxalinyl, phthalazinyl, and quinazolinyl. The heteroaryl group may be optionally substituted with one or more substituents, e.g., 1 to 5 substituents, 1 to 3 substituents, or 1 substituent. "Hydroxyalkyl" refers to an alkyl group substituted with one or more hydroxyl groups.

"Alkoxy" refers to the oxygen ether form of a linear or branched alkyl group, i.e., -O-alkyl. Similarly, "methoxy" refers to -O-CH₃.

"Optionally substituted with ..." means that the compound may be substituted with a specified substituent, or may be unsubstituted.

Divalent groups formed by removing another hydrogen from the above-defined alkyl, alkenyl, alkynyl, cycloalkyl, heterocyclyl, aryl, and heteroaryl groups are collectively referred to as "-ylene". Cyclic groups such as cycloalkyl, heterocyclyl, aryl, and heteroaryl are collectively referred to as "cyclyl".

The alkyl, alkenyl, alkynyl, cycloalkyl, heterocyclyl, aryl, heteroaryl, and the like as defined herein are optionally substituted groups.

Examples of substituents on carbon atoms include, but are not limited to: halogen, -CN, -NO₂, -N₃, -SO₂H, - SO₃H, -OH, -OR^{aa}, -ON(R^{bb})₂, -N(R^{bb})₂, -N(R^{bb})₃⁺X⁻, -N(OR^{cc})R^{bb}, -SH, -SR^{aa}, -SSR^{cc}, -C(=O)R^{aa}, -CO₂H, - CHO, -C(OR^{cc})₂, -CO₂R^{aa}, -OC(=O)R^{aa}, -OCO₂R^{aa}, -C(=O)N(R^{bb})₂, -OC(=O)N(R^{bb})₂, -NR^{bb}C(=O)R^{aa}, - NR^{bb}CO₂R^{aa}, -NR^{bb}C(=O)N(R^{bb})₂, -C(=NR^{bb})R^{aa}, -C(=NR^{bb})OR^{aa}, -OC(=NR^{bb})R^{aa}, -OC(=NR^{bb})OR^{aa}, - C(=NR^{bb})N(R^{bb})₂, -OC(=NR^{bb})N(R^{bb})₂, -NR^{bb}C(=NR^{bb})N(R^{bb})₂, -C(=O)NR^{bb}SO₂R^{aa}, -NR^{bb}SO₂R^{aa}, - SO₂N(R^{bb})₂, -SO₂R^{aa}, -SO₂OR^{aa}, -OSO₂R^{aa}, -S(=O)R^{aa}, -OS(=O)R^{aa}, -Si(R^{aa})₃, -OSi(R^{aa})₃, -C(=S)N(R^{bb})₂, - C(=O)SR^{aa}, -C(=S)SR^{aa}, -SC(=S)SR^{aa}, -SC(=O)SR^{aa}, -OC(=O)SR^{aa}, -SC(=O)OR^{aa}, -SC(=O)R^{aa}, -P(=O)₂R^{aa}, -OP(=O)₂R^{aa}, -P(=O)(R^{aa})₂, -OP(=O)(R^{aa})₂, -OP(=O)(OR^{cc})₂, -P(=O)₂N(R^{bb})₂, -OP(=O)₂N(R^{bb})₂, - P(=O)(NR^{bb})₂, -OP(=O)(NR^{bb})₂, -NR^{bb}P(=O)(OR^{cc})₂, -NR^{bb}P(=O)(NR^{bb})₂, -P(R^{cc})₂, -P(R^{cc})₃, -OP(R^{cc})₂, - OP(R^{cc})₃, -B(R^{aa})₂, -B(OR^{cc})₂, -BR^{aa}(OR^{cc}), alkyl, haloalkyl, alkenyl, alkynyl, cycloalkyl, heterocyclyl, aryl, and heteroaryl, wherein each of the alkyl, alkenyl, alkynyl, cycloalkyl, heterocyclyl, aryl, and heteroaryl is independently substituted with 0, 1, 2, 3, 4, or 5 R^{dd} groups;
or two geminal hydrogen atoms on a carbon atom are substituted with group =O, =S, =NN(R^{bb})₂, =NNR^{bb}C(=O)R^{aa}, =NNR^{bb}C(=O)OR^{aa}, =NNR^{bb}S(=O)₂R^{aa}, =NR^{bb}, or =NOR^{cc};
each R^{aa} is independently selected from alkyl, haloalkyl, alkenyl, alkynyl, cycloalkyl, heterocyclyl, aryl, and heteroaryl, or two R^{aa} groups are joined to form a heterocyclyl or heteroaryl ring, wherein each of the alkyl, alkenyl, alkynyl, cycloalkyl, heterocyclyl, aryl, and heteroaryl is independently substituted with 0, 1, 2, 3, 4, or 5 R^{dd} groups;
each R^{bb} is independently selected from: hydrogen, -OH, -OR^{aa}, -N(R^{cc})₂, -CN, -C(=O)R^{aa}, -C(=O)N(R^{cc})₂, - CO₂R^{aa}, -SO₂R^{aa}, -C(=NR^{cc})OR^{aa}, -C(=NR^{cc})N(R^{cc})₂, -SO₂N(R^{cc})₂, -SO₂R^{cc}, -SO₂OR^{cc}, -SOR^{aa}, - C(=S)N(R^{cc})₂, -C(=O)SR^{cc}, -C(=S)SR^{cc}, -P(=O)₂R^{aa}, -P(=O)(R^{aa})₂, -P(=O)₂N(R^{cc})₂, -P(=O)(NR^{cc})₂, alkyl, haloalkyl, alkenyl, alkynyl, cycloalkyl, heterocyclyl, aryl, and heteroaryl, or two R^{bb} groups are joined to form a heterocyclyl or heteroaryl ring, wherein each of the alkyl, alkenyl, alkynyl, cycloalkyl, heterocyclyl, aryl, and heteroaryl is independently substituted with 0, 1, 2, 3, 4, or 5 R^{dd} groups;
each R^{cc} is independently selected from hydrogen, alkyl, haloalkyl, alkenyl, alkynyl, cycloalkyl, heterocyclyl, aryl, and heteroaryl, or two R^{cc} groups are joined to form a heterocyclyl or heteroaryl ring, wherein each of the alkyl, alkenyl, alkynyl, cycloalkyl, heterocyclyl, aryl, and heteroaryl is independently substituted with 0, 1, 2, 3, 4, or 5 R^{dd} groups;
each R^{dd} is independently selected from: halogen, -CN, -NO₂, -N₃, -SO₂H, -SO₃H, -OH, -OR^{ee}, -ON(R^{ff})₂, - N(R^{ff})₂, -N(R^{ff})₃⁺X⁻, -N(OR^{ee})R^{ff}, -SH, -SR^{ee}, -SSR^{ee}, -C(=O)R^{ee}, -CO₂H, -CO₂R^{ee}, -OC(=O)R^{ee}, -OCO₂R^{ee}, - C(=O)N(R^{ff})₂, -OC(=O)N(R^{ff})₂, -NR^{ff}C(=O)R^{ee}, -NR^{ff}CO₂R^{ee}, -NR^{ff}C(=O)N(R^{ff})₂, -C(=NR^{ff})OR^{ee}, - OC(=NR^{ff})R^{ee}, -OC(=NR^{ff})OR^{ee}, -C(=NR^{ff})N(R^{ff})₂, -OC(=NR^{ff})N(R^{ff})₂, -NR^{ff}C(=NR^{ff})N(R^{ff})₂, -NR^{ff}SO₂R^{ee}, -SO₂N(R^{ff})₂, -SO₂R^{ee}, -SO₂OR^{ee}, -OSO₂R^{ee}, -S(=O)R^{ee}, -Si(R^{ee})₃, -OSi(R^{ee})₃, -C(=S)N(R^{ff})₂, -C(=O)SR^{ee}, - C(=S)SR^{ee}, -SC(=S)SR^{ee}, -P(=O)₂R^{ee}, -P(=O)(R^{ee})₂, -OP(=O)(R^{ee})₂, -OP(=O)(OR^{ee})₂, alkyl, haloalkyl, alkenyl, alkynyl, cycloalkyl, heterocyclyl, aryl, and heteroaryl, wherein each of the alkyl, alkenyl, alkynyl, cycloalkyl, heterocyclyl, aryl, and heteroaryl is independently substituted with 0, 1, 2, 3, 4, or 5 R^{gg} groups, or two geminal R^{dd} substituents may be joined to form =O or =S;
each R^{ee} is independently selected from alkyl, haloalkyl, alkenyl, alkynyl, cycloalkyl, aryl, heterocyclyl, and heteroaryl, wherein each of the alkyl, alkenyl, alkynyl, cycloalkyl, heterocyclyl, aryl, and heteroaryl is independently substituted with 0, 1, 2, 3, 4, or 5 R^{gg} groups;
each R^{ff} is independently selected from hydrogen, alkyl, haloalkyl, alkenyl, alkynyl, cycloalkyl, heterocyclyl, aryl, and heteroaryl, or two R^{ff} groups are joined to form a heterocyclyl or heteroaryl ring, wherein each of the alkyl, alkenyl, alkynyl, cycloalkyl, heterocyclyl, aryl, and heteroaryl is independently substituted with 0, 1, 2, 3, 4, or 5 R^{gg} groups;
each R^{gg} is independently: halogen, -CN, -NO₂, -N₃, -SO₂H, -SO₃H, -OH, -OC₁₋₆ alkyl, -ON(C₁₋₆ alkyl)₂, - N(C₁₋₆ alkyl)₂, -N(C₁₋₆ alkyl)₃⁺X⁻, -NH(C₁₋₆ alkyl)₂⁺X⁻, -NH₂(C₁₋₆ alkyl)⁺X⁻, -NH₃⁺X⁻, -N(OC₁₋₆ alkyl)(C₁₋₆ alkyl), -N(OH)(C₁₋₆ alkyl), -NH(OH), -SH, -SC₁₋₆ alkyl, -SS(C₁₋₆ alkyl), -C(=O)(C₁₋₆ alkyl), -CO₂H, -CO₂(C₁₋₆ alkyl), -OC(=O)(C₁₋₆ alkyl), -OCO₂(C₁₋₆ alkyl), -C(=O)NH₂, -C(=O)N(C₁₋₆ alkyl)₂, -OC(=O)NH(C₁₋₆ alkyl), -NHC(=O)(C₁₋₆ alkyl), -N(C₁₋₆ alkyl)C(=O)(C₁₋₆ alkyl), -NHCO₂(C₁₋₆ alkyl), -NHC(=O)N(C₁₋₆ alkyl)₂, - NHC(=O)NH(C₁₋₆ alkyl), -NHC(=O)NH₂, -C(=NH)O(C₁₋₆ alkyl), -OC(=NH)(C₁₋₆ alkyl), -OC(=NH)OC₁₋₆ alkyl, -C(=NH)N(C₁₋₆ alkyl)₂, -C(=NH)NH(C₁₋₆ alkyl), -C(=NH)NH₂, -OC(=NH)N(C₁₋₆ alkyl)₂, - OC(NH)NH(C₁₋₆ alkyl), -OC(NH)NH₂, -NHC(NH)N(C₁₋₆ alkyl)₂, -NHC(=NH)NH₂, -NHSO₂(C₁₋₆ alkyl), - SO₂N(C₁₋₆ alkyl)₂, -SO₂NH(C₁₋₆ alkyl), -SO₂NH₂, -SO₂C₁₋₆ alkyl, -SO₂OC₁₋₆ alkyl, -OSO₂C₁₋₆ alkyl, -SOC₁₋₆ alkyl, -Si(C₁₋₆ alkyl)₃, -OSi(C₁₋₆ alkyl)₃, -C(=S)N(C₁₋₆ alkyl)₂, C(=S)NH(C₁₋₆ alkyl), C(=S)NH₂, -C(=O)S(C₁₋₆ alkyl), -C(=S)SC₁₋₆ alkyl, -SC(=S)SC₁₋₆ alkyl, -P(=O)₂(C₁₋₆ alkyl), -P(=O)(C₁₋₆ alkyl)₂, -OP(=O)(C₁₋₆ alkyl)₂, -OP(=O)(OC₁₋₆ alkyl)₂, C₁₋₆ alkyl, C₁₋₆ haloalkyl, C₂-C₆ alkenyl, C₂-C₆ alkynyl, C₃-C₇ cycloalkyl, C₆-C₁₀ aryl, 3- to 7-membered heterocyclyl, or 5- to 10-membered heteroaryl; or two geminal R^{gg} substituents may be joined to form =O or =S; wherein X⁻ is a counter ion.

Examples of substituents on nitrogen atoms include, but are not limited to: hydrogen, -OH, -OR^{aa}, -N(R^{cc})₂, - CN, -C(=O)R^{aa}, -C(=O)N(R^{cc})₂, -CO₂R^{aa}, -SO₂R^{aa}, -C(=NR^{bb})R^{aa}, -C(=NR^{cc})OR^{aa}, -C(=NR^{cc})N(R^{cc})₂, - SO₂N(R^{cc})₂, -SO₂R^{cc}, -SO₂OR^{cc}, -SOR^{aa}, -C(=S)N(R^{cc})₂, -C(=O)SR^{cc}, -C(=S)SR^{cc}, -P(=O)₂R^{aa}, -P(=O)(R^{aa})₂, -P(=O)₂N(R^{cc})₂, -P(=O)(NR^{cc})₂, alkyl, haloalkyl, alkenyl, alkynyl, cycloalkyl, heterocyclyl, aryl, and heteroaryl, or two R^{cc} groups connected to a nitrogen atom are joined to form a heterocyclyl or heteroaryl ring, wherein each of the alkyl, alkenyl, alkynyl, cycloalkyl, heterocyclyl, aryl, and heteroaryl is independently substituted with 0, 1, 2, 3, 4, or 5 R^{dd} groups, and wherein R^{aa}, R^{bb}, R^{cc}, and R^{dd} are as described above.

The "nucleic acid" refers to a single- or double-stranded deoxyribonucleic acid (DNA) or ribonucleic acid (RNA) molecule, as well as heterozygous molecules thereof. Examples of nucleic acid molecules include, but are not limited to, messenger RNA (mRNA), micro RNA (miRNA), small interfering RNA (siRNA), self-amplifying RNA (saRNA), antisense oligonucleotide (ASO), and the like. The nucleic acid may be further chemically modified, the chemical modification being selected from pseudouridine, N1-methyl-pseudouridine, 5-methoxyuridine, 5-methylcytosine, and a combination thereof. The mRNA molecule comprises a protein-coding region and may further comprise an expression control sequence. Typical expression control sequences include, but are not limited to, a 5' cap, a 5' untranslated region (5' UTR), a 3' untranslated region (3' UTR), a polyadenylation sequence (PolyA), and a miRNA binding site.

The "cationic lipid" refers to a lipid molecule capable of being positively charged under physiological pH conditions. In some embodiments, the cationic lipid is an amino lipid.

The "neutral lipid" refers to a lipid molecule that is uncharged under specific pH conditions, e.g., physiological pH conditions. Examples of neutral lipids include, but are not limited to, 1,2-distearoyl-sn-glycero-3-phosphocholine (DSPC), 1,2-dimyristoyl-sn-glycero-3-phosphocholine (DMPC), 1,2-dioleoyl-sn-glycero-3-phosphocholine (DOPC), 1,2-dipalmitoyl-sn-glycero-3-phosphocholine (DPPC), 1-palmitoyl-2-oleoyl-sn-glycero-3-phosphocholine (POPC), 1,2-dioleoyl-sn-glycero-3-phosphoethanolamine (DOPE), 1,2-dimyristoyl-sn-glycero-3-phosphoethanolamine (DMPE), 1-palmitoyl-2-oleoyl-sn-glycero-3-phosphoethanolamine (POPE), and 1,2-dipalmitoyl-sn-glycero-3-phosphoethanolamine (DPPE).

The "structural lipid" refers to lipids that enhance nanoparticle stability by filling the gaps between the lipids and commonly used ones are, e.g., steroids. Steroid is a compound having a perhydrocyclopentanophenanthrene carbon framework. In one preferred embodiment, the steroid is selected from cholesterol, sitosterol, coprosterol, fucosterol, brassicasterol, ergosterol, tomatine, ursolic acid, α-tocopherol, stigmasterol, avenasterol, ergocalciferol, and campesterol.

The "polymeric lipid" refers to a molecule containing a polymeric moiety and a lipid moiety. In some embodiments, the polymeric lipid is a polyethylene glycol (PEG) lipid. Other lipids capable of reducing aggregation, such as products resulting from the conjugation of lipids with compounds that have uncharged, hydrophilic, steric hindrance moieties, can also be used.

The "lipid nanoparticle" refers to a particle containing a lipid ingredient and having a nanoscale size.

The "biodegradable group" refers to a functional group containing a biodegradable bond such as ester, disulfide bond, amide, and the like. Biodegradation can affect the process of removing compounds from the body. The direction of the biodegradable groups of the present disclosure is from head to tail in the ionizable lipid molecule.

### Other definitions

The term "treat" as used herein relates to reversing, alleviating, or inhibiting the progression of a disorder or condition to which the term applies, or one or more symptoms of such a disorder or condition, or preventing the disorder or condition. The noun form "treatment" as used herein relates to the action of the verb treat, as just defined above.

The term "pharmaceutically acceptable salt" as used herein refers to those carboxylates and amino acid addition salts of the compound of the present disclosure, which are, within the scope of sound medical judgment, suitable for use in contact with the tissues of patients without undue toxicity, irritation, allergic response, and the like, and are commensurate with a reasonable benefit/risk ratio and effective for their intended use, including the zwitterionic forms, where possible, of the compound of the present disclosure. Pharmaceutically acceptable base addition salts are formed with metals or amines, such as alkali metal and alkaline earth metal hydroxides or organic amines. Examples of metals used as cations are sodium, potassium, magnesium, calcium, and the like. Examples of suitable amines are N,N-dibenzylethylenediamine, chloroprocaine, choline, diethanolamine, ethylenediamine, N-methylglucamine, and procaine.

Base addition salts of acidic compounds may be prepared by contacting the free acid form with a sufficient amount of a desired base to form the salt in a conventional manner. The free acid may be regenerated by contacting the salt form with an acid and separating the free acid in a conventional manner. The free acid forms differ somewhat from their respective salt forms in certain physical properties, such as solubility in polar solvents, but the salts are equivalent to their respective free acids for the purposes of the present disclosure. The salt may be sulfate, pyrosulfate, bisulfate, sulfite, bisulfite, nitrate, phosphate, monohydrogenphosphate, dihydrogenphosphate, metaphosphate, pyrophosphate, chloride, bromide, and iodide prepared from inorganic acids such as hydrochloric acid, nitric acid, sulfuric acid, hydrobromic acid, hydroiodic acid, phosphoric acid, and the like. Representative salts include: hydrobromide, hydrochloride, sulfate, bisulfate, nitrate, acetate, oxalate, valerate, oleate, palmitate, stearate, laurate, borate, benzoate, lactate, phosphate, tosylate, citrate, maleate, fumarate, succinate, tartrate, naphthoate, mesylate, glucoheptonate, lactobionate, laurylsulfonate, isethionate, and the like. The salts may also be prepared from organic acids such as aliphatic mono- and dicarboxylic acids, phenyl-substituted alkanoic acids, hydroxyalkanoic acids, alkanedioic acids, aromatic acids, aliphatic and aromatic sulfonic acids, and the like. Representative salts include acetate, propionate, octanoate, isobutyrate, oxalate, malonate, succinate, suberate, sebacate, fumarate, maleate, mandelate, benzoate, chlorobenzoate, methylbenzoate, dinitrobenzoate, naphthoate, benzenesulfonate, tosylate, phenylacetate, citrate, lactate, maleate, tartrate, mesylate, and the like. The pharmaceutically acceptable salts may include cations based on the alkali metals and alkaline earth metals, such as sodium, lithium, potassium, calcium, magnesium, and the like, as well as non-toxic ammonium, quaternary ammonium, and amine cations including, but not limited to, ammonium, tetramethylammonium, tetraethylammonium, methylamine, dimethylamine, trimethylamine, triethylamine, ethylamine, and the like. Also encompassed are salts of amino acids such as arginate, gluconate, galacturonate, and the like (see, e.g., Berge S. M. et al., "Pharmaceutical Salts", J. Pharm. Sci., 1977; 66:1-19, which is incorporated herein by reference).

The "subject" to which the compound is administered includes, but is not limited to: a human (i.e., a male or female of any age group, e.g., a pediatric subject (e.g., an infant, a child, or an adolescent) or an adult subject (e.g., a young adult, a middle-aged adult, or an older adult)) and/or a non-human animal, e.g., a mammal, such as a primate (e.g., a cynomolgus monkey or a rhesus monkey), a cow, a pig, a horse, a sheep, a goat, a rodent, a cat, and/or a dog. In some embodiments, the subject is a human. In some embodiments, the subject is a non-human animal. The terms "human", "patient", and "subject" are used interchangeably herein.

The "disease", "disorder", and "condition" are used interchangeably herein.

Unless otherwise indicated, the term "treat" used herein includes effects that, when a subject suffers from a particular disease, disorder, or condition, reduce the severity of the disease, disorder, or condition, or delay or slow the progression of the disease, disorder, or condition ("therapeutic treatment"), and also includes effects that occur before the subject begins to suffer from a particular disease, disorder, or condition ("prophylactic treatment").

Generally, the "effective amount" of the pharmaceutical composition refers to an amount sufficient to elicit a biological response of interest. It will be appreciated by those of ordinary skill in the art that, the effective amount of the pharmaceutical composition of the present disclosure may vary depending on the following factors: for example, biological objectives, pharmacokinetics of the pharmaceutical composition, the disease to be treated, the mode of administration, and the age, health, and symptom of the subject. The effective amount includes a therapeutically effective amount and a prophylactically effective amount.

Unless otherwise indicated, the "therapeutically effective amount" of a pharmaceutical composition used herein is an amount sufficient to provide a therapeutic benefit in the treatment of a disease, disorder, or condition, or an amount to delay or minimize one or more symptoms associated with a disease, disorder, or condition. The therapeutically effective amount of a pharmaceutical composition refers to an amount of a therapeutic agent that, alone or in combination with other therapies, provides a therapeutic benefit in the treatment of a disease, disorder, or condition. The term "therapeutically effective amount" may include an amount that improves the overall treatment, reduces or avoids symptoms or causes of a disease or condition, or enhances the therapeutic effect of other therapeutic agents.

Unless otherwise indicated, the "prophylactically effective amount" of a pharmaceutical composition used herein is an amount sufficient to prevent a disease, disorder, or condition, an amount sufficient to prevent one or more symptoms associated with a disease, disorder, or condition, or an amount to prevent the recurrence of a disease, disorder, or condition. The prophylactically effective amount of a pharmaceutical composition refers to an amount of a therapeutic agent that, alone or in combination with other agents, provides a prophylactic benefit in the prevention of a disease, disorder, or condition. The term "prophylactically effective amount" may include an amount that improves the overall prophylaxis, or an amount to enhance the prophylactic effect of other prophylactic agents.

The "combination" and related terms refer to the simultaneous or sequential administration of the pharmaceutical composition of the present disclosure and an additional therapeutic agent. For example, the pharmaceutical composition of the present disclosure may be administered simultaneously or sequentially with the additional therapeutic agent in separate unit dosage forms, or simultaneously with the additional therapeutic agent in a single unit dosage form.

### DETAILED DESCRIPTION

Herein, the "compound of the present disclosure" refers to the following compound, a pharmaceutically acceptable salt, isotopic variant, tautomer, or stereoisomer thereof.

As used herein, compounds are named using standard nomenclature. For compounds with asymmetric centers, it will be appreciated that all optical isomers and mixtures thereof are included, unless otherwise indicated. Furthermore, all isomeric compounds and carbon-carbon double bonds included in the present disclosure may be present in a Z or E form unless otherwise specified. A compound present in different tautomeric forms is not limited to any particular tautomer, but is intended to encompass all tautomeric forms.

In one embodiment, the present disclosure relates to a compound of formula (I), or an isotopic variant, tautomer, or stereoisomer thereof, or a pharmaceutically acceptable salt thereof: 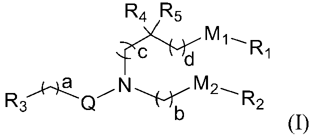 wherein,
a = 1, 2, 3, 4, 5, or 6;
b = 4, 5, 6, 7, 8, 9, or 10;
c = 1, 2, 3, 4, 5, or 6;
d = 0, 1, 2, 3, 4, or 5;
c + d = 3, 4, 5, 6, 7, 8, or 9;
M₁ and M₂ are independently selected from -C(O)O-, -OC(O)-, -O-, -SC(O)O-, -OC(O)NR-, -NRC(O)NR-, - OC(O)S-, -OC(O)O-, -NRC(O)O-, -SC(O)-, -C(O)S-, -NR-, -C(O)NR-, -NRC(O)-, -NRC(O)S-, -SC(O)NR-, -C(O)-, -OC(S)-, -C(S)O-, -OC(S)NR-, -NRC(S)O-, -S-S-, and -S(O)₀₋₂-;
Q is selected from a chemical bond, -C(O)O-, -O-, -SC(O)O-, -OC(O)NRₐ-, -NRₐC(O)NR'ₐ-, -OC(O)S-, - OC(O)O-, -NRₐC(O)O-, -OC(O)-, -SC(O)-, -C(O)S-, -NRₐ-, -C(O)NRₐ-, -NRₐC(O)-, -NRₐC(O)S-, - SC(O)NRₐ-, -C(O)-, -OC(S)-, -C(S)O-, -OC(S)NRₐ-, -NRₐC(S)O-, -S-S-, and -S(O)₀₋₂-;
Rₐ and R'ₐ are independently selected from H, C₁₋₁₀ alkyl, C₃₋₁₄ cycloalkyl, and 3- to 14-membered heterocyclyl;
R₁ and R₂ are independently selected from C₄₋₂₀ alkyl, C₄₋₂₀ alkenyl, and C₄₋₂₀ alkynyl, which are optionally substituted with one or more Rₛ and in which one or more methylene units are optionally and independently replaced by -NR'-;
the substitution site of Rₛ on R₁ or R₂ is separated from M₁ or M₂ by 0-12 carbon atoms;
R and R' are each independently selected from H and C₁₋₂₀ alkyl;
Rₛ is independently selected from H, C₁₋₂₀ alkyl, -L_{b}-OR_{b}, -L_{b}-SR_{b}, and -L_{b}-NR_{b}R'_{b};
L_{b} is independently selected from a chemical bond and C₁₋₂₀ alkylene;
R_{b} and R'_{b} are independently selected from H, C₁₋₂₀ alkyl, C₃₋₁₄ cycloalkyl, and 3- to 14-membered heterocyclyl; R₃ is selected from CN, -OR_{c}, -C(O)R_{c}, -OC(O)R_{c}, -NR"C(O)R_{c}, -NR_{c}R'_{c}, -NR"C(O)NR_{c}R'_{c}, -NR"C(O)R_{c}, - NR"S(O)₂R_{c}, -OC(O)NR_{c}R'_{c}, -NR"C(O)OR_{c}, -N(OR_{c})C(O)R_{c}, -N(OR_{c})S(O)₂R_{c}, -N(OR_{c})C(O)OR_{c}, - N(OR_{c})C(O)R_{c}R'_{c}, 3- to 14-membered heterocyclyl, and 5- to 14-membered heteroaryl;
R_{c} and R'_{c} are independently selected from H, C₁₋₁₀ alkyl, C₃₋₁₀ cycloalkyl, and 3- to 10-membered heterocyclyl;
R" is independently selected from H and C₁₋₆ alkyl;
R₄ and R₅ are independently C₁₋₈ alkyl, which is optionally substituted with one or more R*;
or R₄ and R₅, together with the carbon atom to which they are connected, form C₃₋₁₄ cycloalkylene or 3- to 14-membered heterocyclylene, which is optionally substituted with one or more R*;
R* is independently selected from H, halogen, cyano, C₁₋₄ alkyl, C₁₋₈ haloalkyl, -L_{d}-OR_{d}, -L_{d}-SR_{d}, and -L_{d}-NR_{d}R'_{d};
L_{d} is independently selected from a chemical bond and C₁₋₈ alkylene;
R_{d} and R'_{d} are independently selected from H, C₁₋₈ alkyl, C₃₋₁₄ cycloalkyl, and 3- to 14-membered heterocyclyl.
a, b, c, and d

In one embodiment, a is 1; in another embodiment, a is 2; in another embodiment, a is 3; in another embodiment, a is 4; in another embodiment, a is 5; in another embodiment, a is 6.

In a more specific embodiment, a = 2, 3, 4, 5, or 6; in another more specific embodiment, a = 2, 3, 4, or 5; in another more specific embodiment, a = 2, 3, or 4; in another more specific embodiment, a = 2 or 3; in another more specific embodiment, a = 3 or 4.

In one embodiment, b is 4; in another embodiment, b is 5; in another embodiment, b is 6; in another embodiment, b is 7; in another embodiment, b is 8; in another embodiment, b is 9; in another embodiment, b is 10.

In a more specific embodiment, b = 4, 5, 6, 7, 8, or 9; in another more specific embodiment, b = 5, 6, 7, or 8; in another more specific embodiment, b = 5, 6, or 7; in another more specific embodiment, b = 6, 7, or 8; in another more specific embodiment, b = 5 or 7; in another more specific embodiment, b = 6 or 7; in another more specific embodiment, b = 6 or 8; in another more specific embodiment, b = 7 or 8.

In one embodiment, c is 1; in another embodiment, c is 2; in another embodiment, c is 3; in another embodiment, c is 4; in another embodiment, c is 5; in another embodiment, c is 6.

In a more specific embodiment, c = 2, 3, 4, 5, or 6; in another more specific embodiment, c = 2, 3, 5, or 6; in another more specific embodiment, c = 3, 4, 5, or 6; in another more specific embodiment, c = 2, 3, 4, or 5; in another more specific embodiment, c = 3, 5, or 6; in another more specific embodiment, c = 2, 4, or 5; in another more specific embodiment, c = 2 or 4; in another more specific embodiment, c = 2 or 6; in another more specific embodiment, c = 5 or 6.

In one embodiment, d is 0; in another embodiment, d is 1; in another embodiment, d is 2; in another embodiment, d is 3; in another embodiment, d is 4; in another embodiment, d is 5.

In a more specific embodiment, d = 1, 2, 3, 4, or 5; in another more specific embodiment, d = 0, 1, 2, 3, or 4; in another more specific embodiment, d = 1, 2, 3, or 4; in another more specific embodiment, d = 0, 1, 2, or 3; in another more specific embodiment, d = 1, 2, or 4; in another more specific embodiment, d = 0, 1, or 2; in another more specific embodiment, d = 0 or 1; in another more specific embodiment, d = 2 or 4; in another more specific embodiment, d = 0 or 3.

In one embodiment, c + d = 3; in another embodiment, c + d = 4; in another embodiment, c + d = 5; in another embodiment, c + d = 6; in another embodiment, c + d = 7; in another embodiment, c + d = 8; in another embodiment, c + d = 9.

In a more specific embodiment, c + d = 4, 5, or 6; in another more specific embodiment, c + d = 5, 6, or 7; in another more specific embodiment, c + d = 6 or 7; in another more specific embodiment, c + d = 5 or 6.

### M₁ and M₂

In one embodiment, M₁ is -C(O)O-; in another embodiment, M₁ is -OC(O)-; in another embodiment, M₁ is - O-; in another embodiment, M₁ is -SC(O)O-; in another embodiment, M₁ is -OC(O)NR-; in another embodiment, M₁ is -NRC(O)NR-; in another embodiment, M₁ is -OC(O)S-; in another embodiment, M₁ is - OC(O)O-; in another embodiment, M₁ is -NRC(O)O-; in another embodiment, M₁ is -SC(O)-; in another embodiment, M₁ is -C(O)S-; in another embodiment, M₁ is -NR-; in another embodiment, M₁ is -C(O)NR-, e.g., -C(O)NH-; in another embodiment, M₁ is -NRC(O)-, e.g., -NHC(O)-; in another embodiment, M₁ is - NRC(O)S-; in another embodiment, M₁ is -SC(O)NR-; in another embodiment, M₁ is -C(O)-; in another embodiment, M₁ is -OC(S)-; in another embodiment, M₁ is -C(S)O-; in another embodiment, M₁ is -OC(S)NR-; in another embodiment, M₁ is -NRC(S)O-; in another embodiment, M₁ is -S-S-; in another embodiment, M₁ is -S(O)₀₋₂-, e.g., -S-, e.g., -S(O)-, e.g., -S(O)₂-.

In one embodiment, M₂ is -C(O)O-; in another embodiment, M₂ is -OC(O)-; in another embodiment, M₂ is - O-; in another embodiment, M₂ is -SC(O)O-; in another embodiment, M₂ is -OC(O)NR-; in another embodiment, M₂ is -NRC(O)NR-; in another embodiment, M₂ is -OC(O)S-; in another embodiment, M₂ is - OC(O)O-; in another embodiment, M₂ is -NRC(O)O-; in another embodiment, M₂ is -SC(O)-; in another embodiment, M₂ is -C(O)S-; in another embodiment, M₂ is -NR-; in another embodiment, M₂ is -C(O)NR-; in another embodiment, M₂ is -NRC(O)-; in another embodiment, M₂ is -NRC(O)S-; in another embodiment, M₂ is -SC(O)NR-; in another embodiment, M₂ is -C(O)-; in another embodiment, M₂ is -OC(S)-; in another embodiment, M₂ is -C(S)O-; in another embodiment, M₂ is -OC(S)NR-; in another embodiment, M₂ is - NRC(S)O-; in another embodiment, M₂ is -S-S-; in another embodiment, M₂ is -S(O)₀₋₂-, e.g., -S-, e.g., -S(O)-, e.g., -S(O)₂-.

In a more specific embodiment, M₁ and M₂ are independently selected from -C(O)O-, -C(O)S-, -OC(O)-, - SC(O)-, -OC(O)O-, -C(O)NR-, and -NRC(O)-; in another more specific embodiment, M₁ and M₂ are independently selected from -C(O)O-, -C(O)S-, -OC(O)-, -SC(O)-, and -OC(O)O-; in another more specific embodiment, M₁ and M₂ are independently selected from -C(O)O-, -C(O)S-, -OC(O)-, and -SC(O)-. Preferably, M₁ and M₂ are independently selected from -C(O)O-, -OC(O)-, and -OC(O)O-; in another more specific embodiment, M₁ and M₂ are not both -OC(O)O-; in another more specific embodiment, M₁ and M₂ are not both -OC(O)-; in another more specific embodiment, M₁ and M₂ are independently selected from - C(O)O- and -OC(O)-.

In a more specific embodiment, M₁ is -C(O)O- or -OC(O)-, preferably -C(O)O-; M₂ is -C(O)O-.

In a more specific embodiment, M₁ is -OC(O)- or -SC(O)-; M₂ is -C(O)O- or -C(O)S-.

In a more specific embodiment, M₁ is -OC(O)-; M₂ is -C(O)O-.

In a more specific embodiment, M₁ and M₂ are independently selected from -C(O)O- and -C(O)S-, preferably -C(O)O-;

In a more specific embodiment, one of M₁ and M₂ is -OC(O)O-, and the other is selected from -C(O)O-, - C(O)S-, -OC(O)-, and -SC(O)-, preferably -C(O)O- and -OC(O)-.

In a more specific embodiment, M₁ is -OC(O)O-, and M₂ is selected from -C(O)O-, -C(O)S-, -OC(O)-, and - SC(O)-, preferably -C(O)O- and -OC(O)-.

In a more specific embodiment, M₂ is -OC(O)O-, and M₁ is selected from -C(O)O-, -C(O)S-, -OC(O)-, and - SC(O)-, preferably -C(O)O- and -OC(O)-.

### Q

In one embodiment, Q is a chemical bond; in another embodiment, Q is -C(O)O-; in another embodiment, Q is -O-; in another embodiment, Q is -SC(O)O-; in another embodiment, Q is -OC(O)NRₐ-; in another embodiment, Q is -NRₐC(O)NR'ₐ-; in another embodiment, Q is -OC(O)S-; in another embodiment, Q is - OC(O)O-; in another embodiment, Q is -NRₐC(O)O-; in another embodiment, Q is -OC(O)-; in another embodiment, Q is -SC(O)-; in another embodiment, Q is -C(O)S-; in another embodiment, Q is -NRₐ-; in another embodiment, Q is -C(O)NRₐ-; in another embodiment, Q is -NRₐC(O)-; in another embodiment, Q is -NRₐC(O)S-; in another embodiment, Q is -SC(O)NRₐ-; in another embodiment, Q is -C(O)-; in another embodiment, Q is -OC(S)-; in another embodiment, Q is -C(S)O-; in another embodiment, Q is -OC(S)NRₐ-; in another embodiment, Q is -NRₐC(S)O-; in another embodiment, Q is -S-S-; in another embodiment, Q is - S(O)₀₋₂-, e.g., -S-, e.g., -S(O)-, e.g., -S(O)₂-.

In a more specific embodiment, Q is selected from a chemical bond, -OC(O)-, and -SC(O)-; in another more specific embodiment, Q is a chemical bond or -SC(O)-; in another more specific embodiment, Q is -SC(O)- or -OC(O)-.

### Rₐ and R'ₐ

In one embodiment, Rₐ is H; in another embodiment, Rₐ is C₁₋₁₀ alkyl, preferably C₁₋₆ alkyl, and preferably C₁₋₄ alkyl; in another embodiment, Rₐ is C₃₋₁₄ cycloalkyl, preferably C₃₋₁₀ cycloalkyl; in another embodiment, Rₐ is 3- to 14-membered heterocyclyl, preferably 3- to 10-membered heterocyclyl.

In one embodiment, R'ₐ is H; in another embodiment, R'ₐ is C₁₋₁₀ alkyl, preferably C₁₋₆ alkyl, and preferably C₁₋₄ alkyl; in another embodiment, R'ₐ is C₃₋₁₄ cycloalkyl, preferably C₃₋₁₀ cycloalkyl; in another embodiment, R'ₐ is 3- to 14-membered heterocyclyl, preferably 3- to 10-membered heterocyclyl.

In a more specific embodiment, Rₐ and R'ₐ are independently selected from H, C₁₋₆ alkyl, C₃₋₁₀ cycloalkyl, and 3- to 10-membered heterocyclyl; in another more specific embodiment, Rₐ and R'ₐ are independently selected from H and C₁₋₆ alkyl; in another more specific embodiment, Rₐ and R'ₐ are independently selected from H and C₁₋₄ alkyl.

### R₁ and R₂

In one embodiment, R₁ is C₄₋₂₀ alkyl, preferably C₆₋₁₄ alkyl, preferably C₇₋₁₂ alkyl, preferably C₈₋₁₂ alkyl, preferably C₈₋₁₁ alkyl, preferably C₉₋₁₁ alkyl, preferably C₈₋₁₀ alkyl, preferably C₉₋₁₀ alkyl, preferably C₁₀ alkyl, preferably C₈₋₉ alkyl, preferably C₉ alkyl, preferably C₈ alkyl, preferably C₈₋₁₂ linear alkyl, preferably C₇₋₁₁ linear alkyl, preferably C₈₋₁₁ linear alkyl, preferably C₉₋₁₁ linear alkyl, preferably C₁₀₋₁₁ linear alkyl, preferably C₈₋₁₀ linear alkyl, preferably C₉₋₁₀ linear alkyl, preferably C₈₋₉ linear alkyl, preferably C₁₁ linear alkyl, preferably C₁₀ linear alkyl, and preferably C₉ linear alkyl; in another embodiment, R₁ is C₄₋₂₀ alkenyl, preferably C₆₋₁₄ alkenyl, preferably C₇₋₁₂ alkenyl, and preferably C₈₋₁₂ alkenyl; in another embodiment, R₁ is C₄₋₂₀ alkynyl, preferably C₆₋₁₄ alkynyl, preferably C₇₋₁₂ alkynyl, and preferably C₈₋₁₂ alkynyl; in another embodiment, R₁ is optionally substituted with one or more Rₛ; in another embodiment, R₁ is optionally substituted with 1, 2, 3, or 4 Rₛ, and preferably, optionally substituted with 1 Rₛ; in another embodiment, R₁ is unsubstituted; in another embodiment, one or more methylene units in R₁ are optionally and independently replaced by -NR'-; preferably, one methylene unit in R₁ is optionally replaced by -NR'-.

In one embodiment, R₁ is -(CH₂)₇CH₃; in another embodiment, R₁ is -(CH₂)₈CH₃; in another embodiment, R₁ is -(CH₂)₉CH₃; in another embodiment, R₁ is -(CH₂)₁₀CH₃; in another embodiment, R₁ is 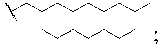 in another embodiment, R₁ is 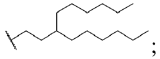 in another embodiment, R₁ is 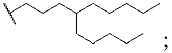 in another embodiment, R₁ is 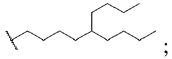 in another embodiment, R₁ is 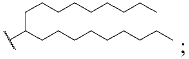 in another embodiment, R₁ is 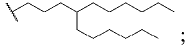 in another embodiment, R₁ is 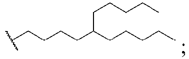 in another embodiment, R₁ is 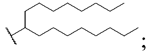 in another embodiment, R₁ is 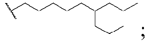 in another embodiment, R₁ is 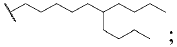 in another embodiment, R₁ is 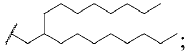 in another embodiment, R₁ is 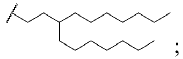 in another embodiment, R₁ is 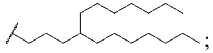 in another embodiment, R₁ is 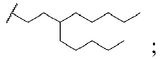 in another embodiment, R₁ is 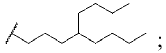 in another embodiment, R₁ is

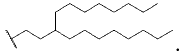

In one embodiment, R₂ is C₄₋₂₀ alkyl, preferably C₆₋₁₄ alkyl, preferably C₇₋₁₂ alkyl, preferably C₈₋₁₂ alkyl, preferably C₈₋₁₁ alkyl, preferably C₉₋₁₁ alkyl, preferably C₈₋₁₀ alkyl, preferably C₉₋₁₀ alkyl, preferably C₁₀ alkyl, preferably C₈₋₉ alkyl, preferably C₉ alkyl, preferably C₈ alkyl, preferably C₈₋₁₂ linear alkyl, preferably C₇₋₁₁ linear alkyl, preferably C₈₋₁₁ linear alkyl, preferably C₉₋₁₁ linear alkyl, preferably C₁₀₋₁₁ linear alkyl, preferably C₈₋₁₀ linear alkyl, preferably C₉₋₁₀ linear alkyl, preferably C₈₋₉ linear alkyl, preferably C₁₁ linear alkyl, preferably C₁₀ linear alkyl, and preferably C₉ linear alkyl; in another embodiment, R₂ is C₄₋₂₀ alkenyl, preferably C₆₋₁₄ alkenyl, preferably C₇₋₁₂ alkenyl, and preferably C₈₋₁₂ alkenyl; in another embodiment, R₂ is C₄₋₂₀ alkynyl, preferably C₆₋₁₄ alkynyl, preferably C₇₋₁₂ alkynyl, and preferably C₈₋₁₂ alkynyl; in another embodiment, R₂ is optionally substituted with one or more Rₛ; in another embodiment, R₂ is optionally substituted with 1, 2, 3, or 4 Rₛ, and preferably, optionally substituted with 1 Rₛ; in another embodiment, R₂ is unsubstituted; in another embodiment, one or more methylene units in R₂ are optionally and independently replaced by -NR'-; preferably, one methylene unit in R₂ is optionally replaced by -NR'-.

In one embodiment, R₂ is -(CH₂)₇CH₃; in another embodiment, R₂ is -(CH₂)₈CH₃; in another embodiment, R₂ is -(CH₂)₉CH₃; in another embodiment, R₂ is -(CH₂)₁₀CH₃; in another embodiment, R₂ is 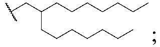 in another embodiment, R₂ is 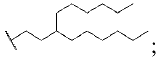 in another embodiment, R₂ is 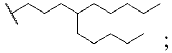 in another embodiment, R₂ is 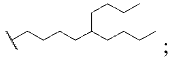 in another embodiment, R₂ is 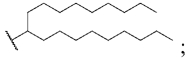 in another embodiment, R₂ is 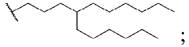 in another embodiment, R₂ is 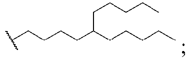 in another embodiment, R₂ is 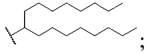 in another embodiment, R₂ is 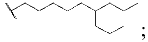 in another embodiment, R₂ is 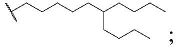 in another embodiment, R₂ is 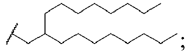 in another embodiment, R₂ is 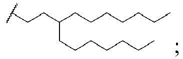 in another embodiment, R₂ is 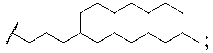 in another embodiment, R₂ is 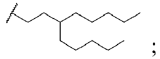 in another embodiment, R₂ is 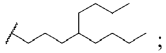 in another embodiment, R₂ is

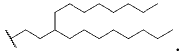

In a more specific embodiment, R₁ and R₂ are independently selected from C₈₋₁₂ alkyl, C₈₋₁₂ alkenyl, and C₈₋₁₂ alkynyl; in another more specific embodiment, R₁ and R₂ are independently selected from C₈₋₁₀ alkyl, C₈₋₁₀ alkenyl, and C₈₋₁₀ alkynyl.

In a more specific embodiment, R₁ and R₂ are independently C₈₋₁₂ alkyl, preferably C₈₋₁₁ alkyl, preferably C₉₋₁₁ alkyl, preferably C₈₋₁₀ alkyl, preferably C₉₋₁₀ alkyl, and preferably C₉ alkyl.

In a more specific embodiment, R₁ and R₂ are independently selected from the following groups: -(CH₂)₇CH₃, -(CH₂)₈CH₃, -(CH₂)₉CH₃, -(CH₂)₁₀CH₃, 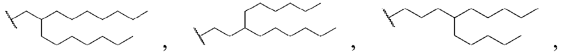 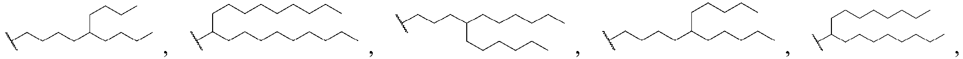 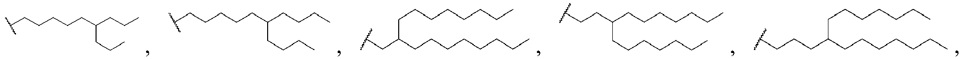 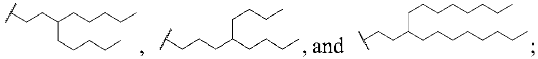 in another more specific embodiment, R₁ and R₂ are independently selected from -(CH₂)₇CH₃, -(CH₂)₈CH₃, -(CH₂)₉CH₃, -(CH₂)₁₀CH₃,

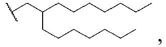

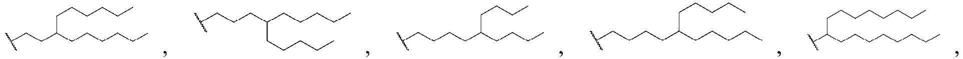

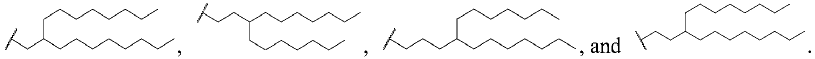

In one embodiment, R₁ and R₂ are not both substituted; in another embodiment, one of R₁ and R₂ is substituted, and the other is unsubstituted; in another embodiment, R₁ is unsubstituted, and R₂ is substituted; in another embodiment, R₁ is substituted, and R₂ is unsubstituted; in another embodiment, one of R₁ and R₂ is substituted with 1, 2, 3, or 4 (preferably 1) Rₛ, and the other is unsubstituted; in another embodiment, R₁ is substituted with 1, 2, 3, or 4 (preferably 1) Rₛ, and R₂ is unsubstituted; in another embodiment, R₁ is unsubstituted, and R₂ is substituted with 1, 2, 3, or 4 (preferably 1) Rₛ.

In a more specific embodiment, one of R₁ and R₂ is selected from -(CH₂)₇CH₃, -(CH₂)₈CH₃, -(CH₂)₉CH₃, and -(CH₂)₁₀CH₃, preferably -(CH₂)₈CH₃, -(CH₂)₉CH₃, and -(CH₂)₁₀CH₃; the other is selected from: 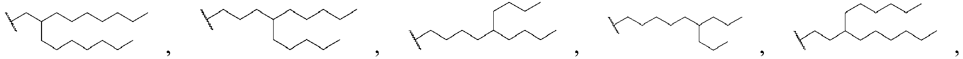 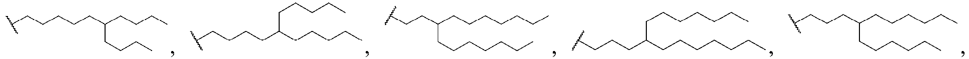 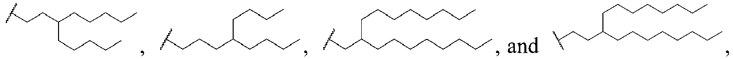 preferably selected from:

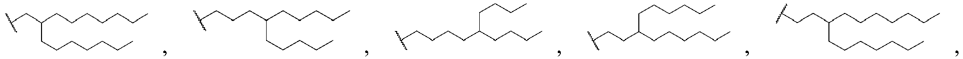

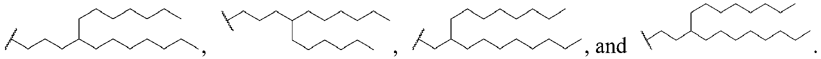

In a more specific embodiment, R₁ and R₂ are independently selected from: -(CH₂)₇CH₃, -(CH₂)₈CH₃, - (CH₂)₉CH₃, 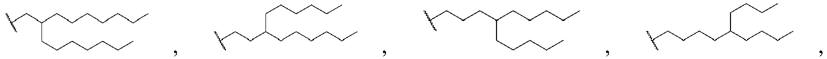 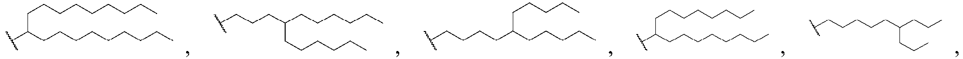 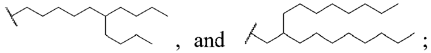 in another more specific embodiment, R₁ and R₂ are independently selected from: -(CH₂)₇CH₃, -(CH₂)₈CH₃, -(CH₂)₉CH₃,

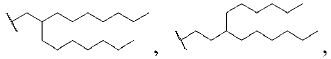

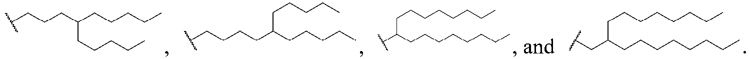

In a more specific embodiment, R₁ and R₂ are independently selected from: -(CH₂)₇CH₃, -(CH₂)₈CH₃, - (CH₂)₉CH₃, -(CH₂)₁₀CH₃, 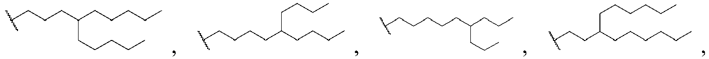 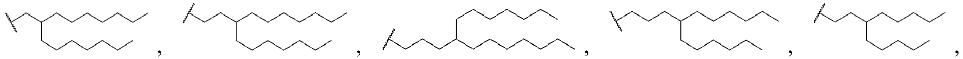 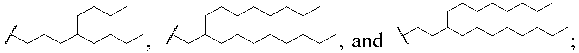 in another more specific embodiment, R₁ and R₂ are independently selected from: -(CH₂)₈CH₃, -(CH₂)₉CH₃, -(CH₂)₁₀CH₃,

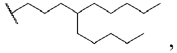

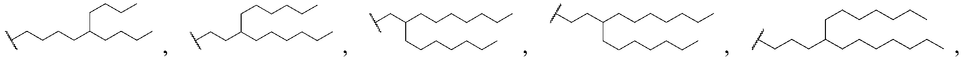

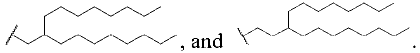

In a more specific embodiment, one of R₁ and R₂ is selected from C₉₋₁₀ linear alkyl, preferably C₉ linear alkyl, and the other is selected from: 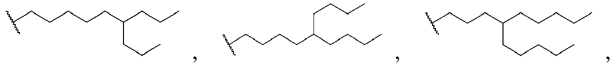 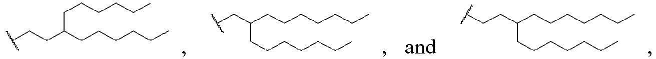 preferably

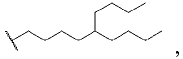

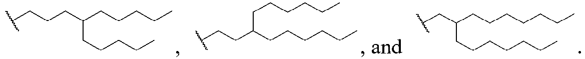

In a more specific embodiment, one of R₁ and R₂ is C₉ linear alkyl, and the other is selected from: 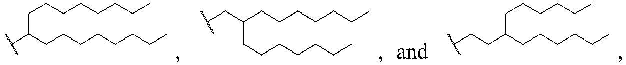 preferably 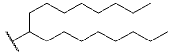 and

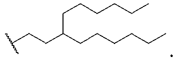

In a more specific embodiment, R₂ is selected from: 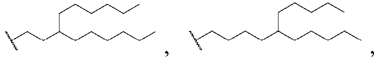 and 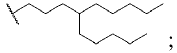 in another more specific embodiment, R₂ is selected from: 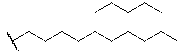 and 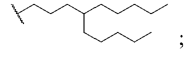 in another more specific embodiment, R₂ is selected from: 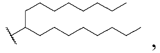 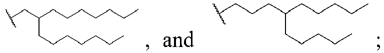 in another more specific embodiment, R₂ is selected from: 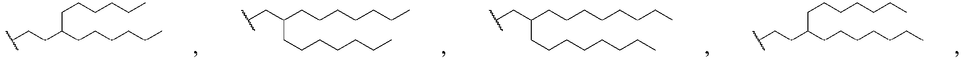 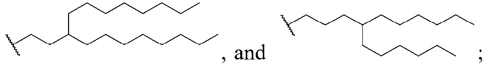 in another more specific embodiment, R₂ is selected from: 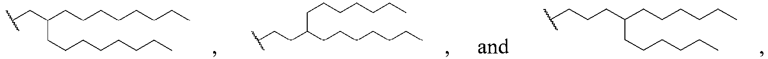 preferably 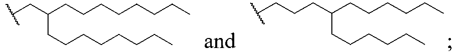 in another more specific embodiment, R₂ is selected from:

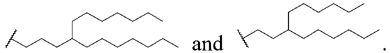

### R and R'

In one embodiment, R is H; in another embodiment, R is C₁₋₂₀ alkyl, preferably C₁₋₁₄ alkyl, preferably C₁₋₁₀ alkyl, and preferably C₁₋₆ alkyl.

In one embodiment, R' is H; in another embodiment, R' is C₁₋₂₀ alkyl, preferably C₁₋₁₄ alkyl, preferably C₁₋₁₀ alkyl, and preferably C₁₋₆ alkyl.

In a more specific embodiment, R and R' are each independently selected from H and C₁₋₂₀ alkyl; in another more specific embodiment, R and R' are each independently selected from H and C₁₋₁₄ alkyl; in another more specific embodiment, R and R' are each independently selected from H and C₁₋₁₀ alkyl; in another more specific embodiment, R and R' are each independently selected from H and C₁₋₆ alkyl; in another more specific embodiment, R is H.

### Rₛ

In one embodiment, Rₛ is H; in another embodiment, Rₛ is H; in another embodiment, Rₛ is C₁₋₂₀ alkyl; in another embodiment, Rₛ is -L_{b}-OR_{b}; in another embodiment, Rₛ is -L_{b}-SR_{b}; in another embodiment, Rₛ is -L_{b}-NR_{b}R'_{b}.

In a more specific embodiment, Rₛ is independently selected from H, C₁₋₁₄ alkyl, -L_{b}-OR_{b}, and -L_{b}-NR_{b}R'_{b}; in another more specific embodiment, Rₛ is independently selected from H and C₁₋₁₄ alkyl; in another more specific embodiment, Rₛ is independently selected from H and C₁₋₁₂ alkyl; in another more specific embodiment, Rₛ is independently selected from H and C₁₋₁₀ alkyl; in another more specific embodiment, Rₛ is independently selected from H and C₁₋₉ alkyl; in another more specific embodiment, Rₛ is independently selected from H and C₃₋₉ alkyl; in another more specific embodiment, Rₛ is independently selected from H and C₁₋₈ alkyl; in another more specific embodiment, Rₛ is independently selected from H and C₃₋₈ alkyl; in another more specific embodiment, Rₛ is independently selected from H and C₄₋₈ alkyl; in another more specific embodiment, Rₛ is independently selected from H and C₅₋₈ alkyl; in another more specific embodiment, Rₛ is independently selected from H and C₆₋₈ alkyl; in another more specific embodiment, Rₛ is independently selected from H and C₁₋₇ alkyl; in another more specific embodiment, Rₛ is independently selected from H and C₃₋₇ alkyl; in another more specific embodiment, Rₛ is independently selected from H and C₄₋₇ alkyl; in another more specific embodiment, Rₛ is independently selected from H and C₅₋₇ alkyl; in another more specific embodiment, Rₛ is independently selected from H and C₁₋₆ alkyl; in another more specific embodiment, Rₛ is independently selected from H and C₅₋₆ alkyl; in another more specific embodiment, Rₛ is independently selected from H and C₁₋₅ alkyl; in another more specific embodiment, Rₛ is independently selected from H and C₄₋₅ alkyl; in another more specific embodiment, Rₛ is independently selected from H and C₅ alkyl.

In a more specific embodiment, Rₛ is independently selected from C₁₋₁₄ alkyl, -L_{b}-OR_{b}, and -L_{b}-NR_{b}R'_{b}; in another more specific embodiment, Rₛ is independently C₁₋₁₄ alkyl; in another more specific embodiment, Rₛ is independently C₁₋₁₂ alkyl; in another more specific embodiment, Rₛ is independently C₁₋₁₀ alkyl; in another more specific embodiment, Rₛ is independently C₁₋₉ alkyl; in another more specific embodiment, Rₛ is independently C₃₋₉ alkyl; in another more specific embodiment, Rₛ is independently C₁₋₈ alkyl; in another more specific embodiment, Rₛ is independently C₃₋₈ alkyl; in another more specific embodiment, Rₛ is independently C₄₋₈ alkyl; in another more specific embodiment, Rₛ is independently C₅₋₈ alkyl; in another more specific embodiment, Rₛ is independently C₆₋₈ alkyl; in another more specific embodiment, Rₛ is independently C₁₋₇ alkyl; in another more specific embodiment, Rₛ is independently C₃₋₇ alkyl; in another more specific embodiment, Rₛ is independently C₄₋₇ alkyl; in another more specific embodiment, Rₛ is independently C₅₋₇ alkyl; in another more specific embodiment, Rₛ is independently C₁₋₆ alkyl; in another more specific embodiment, Rₛ is independently C₅₋₆ alkyl; in another more specific embodiment, Rₛ is independently C₁₋₅ alkyl; in another more specific embodiment, Rₛ is independently C₄₋₅ alkyl; in another more specific embodiment, Rₛ is independently C₉ alkyl; in another more specific embodiment, Rₛ is independently C₈ alkyl; in another more specific embodiment, Rₛ is independently C₇ alkyl; in another more specific embodiment, Rₛ is independently C₆ alkyl; in another more specific embodiment, Rₛ is independently C₅ alkyl.

In one embodiment, the substitution site of Rₛ on R₁ is separated from M₁ by 0 carbon atoms; in another embodiment, the substitution site of Rₛ on R₁ is separated from M₁ by 1 carbon atom; in another embodiment, the substitution site of Rₛ on R₁ is separated from M₁ by 2 carbon atoms; in another embodiment, the substitution site of Rₛ on R₁ is separated from M₁ by 3 carbon atoms; in another embodiment, the substitution site of Rₛ on R₁ is separated from M₁ by 4 carbon atoms; in another embodiment, the substitution site of Rₛ on R₁ is separated from M₁ by 5 carbon atoms; in another embodiment, the substitution site of Rₛ on R₁ is separated from M₁ by 6 carbon atoms; in another embodiment, the substitution site of Rₛ on R₁ is separated from M₁ by 7 carbon atoms; in another embodiment, the substitution site of Rₛ on R₁ is separated from M₁ by 8 carbon atoms; in another embodiment, the substitution site of Rₛ on R₁ is separated from M₁ by 9 carbon atoms; in another embodiment, the substitution site of Rₛ on R₁ is separated from M₁ by 10 carbon atoms; in another embodiment, the substitution site of Rₛ on R₁ is separated from M₁ by 11 carbon atoms; in another embodiment, the substitution site of Rₛ on R₁ is separated from M₁ by 12 carbon atoms.

In one embodiment, the substitution site of Rₛ on R₂ is separated from M₂ by 0 carbon atoms; in another embodiment, the substitution site of Rₛ on R₂ is separated from M₂ by 1 carbon atom; in another embodiment, the substitution site of Rₛ on R₂ is separated from M₂ by 2 carbon atoms; in another embodiment, the substitution site of Rₛ on R₂ is separated from M₂ by 3 carbon atoms; in another embodiment, the substitution site of Rₛ on R₂ is separated from M₂ by 4 carbon atoms; in another embodiment, the substitution site of Rₛ on R₂ is separated from M₂ by 5 carbon atoms; in another embodiment, the substitution site of Rₛ on R₂ is separated from M₂ by 6 carbon atoms; in another embodiment, the substitution site of Rₛ on R₂ is separated from M₂ by 7 carbon atoms; in another embodiment, the substitution site of Rₛ on R₂ is separated from M₂ by 8 carbon atoms; in another embodiment, the substitution site of Rₛ on R₂ is separated from M₂ by 9 carbon atoms; in another embodiment, the substitution site of Rₛ on R₂ is separated from M₂ by 10 carbon atoms; in another embodiment, the substitution site of Rₛ on R₂ is separated from M₂ by 11 carbon atoms; in another embodiment, the substitution site of Rₛ on R₂ is separated from M₂ by 12 carbon atoms.

In a more specific embodiment, the substitution site of Rₛ on R₁ is separated from M₁ by 0-12 carbon atoms; in another more specific embodiment, the substitution site of Rₛ on R₁ is separated from M₁ by 0-10 carbon atoms; in another more specific embodiment, the substitution site of Rₛ on R₁ is separated from M₁ by 0-6 carbon atoms; in another more specific embodiment, the substitution site of Rₛ on R₁ is separated from M₁ by 0-5 carbon atoms; in another more specific embodiment, the substitution site of Rₛ on R₁ is separated from M₁ by 0-4 carbon atoms; in another more specific embodiment, the substitution site of Rₛ on R₁ is separated from M₁ by 0-3 carbon atoms; in another more specific embodiment, the substitution site of Rₛ on R₁ is separated from M₁ by 0-2 carbon atoms; the substitution site of Rₛ on R₁ is separated from M₁ by at least 2 carbon atoms; in another more specific embodiment, the substitution site of Rₛ on R₁ is separated from M₁ by 2-4 carbon atoms; in another more specific embodiment, the substitution site of Rₛ on R₁ is separated from M₁ by 2-3 carbon atoms; in another more specific embodiment, the substitution site of Rₛ on R₁ is separated from M₁ by 3-4 carbon atoms; the substitution site of Rₛ on R₁ is separated from M₁ by at least 1 carbon atom; in another more specific embodiment, the substitution site of Rₛ on R₁ is separated from M₁ by 1-5 carbon atoms; in another more specific embodiment, the substitution site of Rₛ on R₁ is separated from M₁ by 1-4 carbon atoms; in another more specific embodiment, the substitution site of Rₛ on R₁ is separated from M₁ by 1-3 carbon atoms; in another more specific embodiment, the substitution site of Rₛ on R₁ is separated from M₁ by 3 carbon atoms.

In a more specific embodiment, the substitution site of Rₛ on R₂ is separated from M₂ by 0-12 carbon atoms; in another more specific embodiment, the substitution site of Rₛ on R₂ is separated from M₂ by 0-10 carbon atoms; in another more specific embodiment, the substitution site of Rₛ on R₂ is separated from M₂ by 0-6 carbon atoms; in another more specific embodiment, the substitution site of Rₛ on R₂ is separated from M₂ by 0-5 carbon atoms; in another more specific embodiment, the substitution site of Rₛ on R₂ is separated from M₂ by 0-4 carbon atoms; in another more specific embodiment, the substitution site of Rₛ on R₂ is separated from M₂ by 0-3 carbon atoms; in another more specific embodiment, the substitution site of Rₛ on R₂ is separated from M₂ by 0-2 carbon atoms; the substitution site of Rₛ on R₂ is separated from M₂ by at least 2 carbon atoms; in another more specific embodiment, the substitution site of Rₛ on R₂ is separated from M₂ by 2-4 carbon atoms; in another more specific embodiment, the substitution site of Rₛ on R₂ is separated from M₁ by 2-3 carbon atoms; in another more specific embodiment, the substitution site of Rₛ on R₂ is separated from M₂ by 3-4 carbon atoms; the substitution site of Rₛ on R₂ is separated from M₂ by at least 1 carbon atom; in another more specific embodiment, the substitution site of Rₛ on R₂ is separated from M₂ by 1-5 carbon atoms; in another more specific embodiment, the substitution site of Rₛ on R₂ is separated from M₂ by 1-4 carbon atoms; in another more specific embodiment, the substitution site of Rₛ on R₂ is separated from M₂ by 1-3 carbon atoms; in another more specific embodiment, the substitution site of Rₛ on R₂ is separated from M₂ by 3 carbon atoms.

### L_{b}, R_{b}, and R'_{b}

In one embodiment, L_{b} is a chemical bond; in another embodiment, L_{b} is C₁₋₂₀ alkylene, preferably C₁₋₁₄ alkylene, preferably C₁₋₁₀ alkylene, and preferably C₁₋₆ alkylene.

In a more specific embodiment, L_{b} is independently selected from a chemical bond and C₁₋₁₄ alkylene; in another more specific embodiment, L_{b} is independently selected from a chemical bond and C₁₋₁₀ alkylene; in another more specific embodiment, L_{b} is independently selected from a chemical bond and C₁₋₆ alkylene.

In one embodiment, R_{b} is H; in another embodiment, R_{b} is C₁₋₂₀ alkyl, preferably C₁₋₁₄ alkyl, preferably C₁₋₁₀ alkyl, and preferably C₁₋₆ alkyl; in another embodiment, R_{b} is C₃₋₁₄ cycloalkyl, preferably C₃₋₁₀ cycloalkyl; in another embodiment, R_{b} is 3- to 14-membered heterocyclyl, preferably 3- to 10-membered heterocyclyl.

In one embodiment, R'_{b} is H; in another embodiment, R'_{b} is C₁₋₂₀ alkyl, preferably C₁₋₁₄ alkyl, preferably C₁₋₁₀ alkyl, and preferably C₁₋₆ alkyl; in another embodiment, R'_{b} is C₃₋₁₄ cycloalkyl, preferably C₃₋₁₀ cycloalkyl; in another embodiment, R'_{b} is 3- to 14-membered heterocyclyl, preferably 3- to 10-membered heterocyclyl.

In a more specific embodiment, R_{b} and R'_{b} are independently selected from H and C₁₋₁₄ alkyl; in another more specific embodiment, R_{b} and R'_{b} are independently selected from H and C₁₋₁₀ alkyl; in another more specific embodiment, R_{b} and R'_{b} are independently selected from H and C₁₋₆ alkyl.

### R₃

In one embodiment, R₃ is CN; in another embodiment, R₃ is -OR_{c}, e.g., -OH; in another embodiment, R₃ is - C(O)R_{c}; in another embodiment, R₃ is -OC(O)R_{c}; in another embodiment, R₃ is -NR"C(O)R_{c}; in another embodiment, R₃ is -NR_{c}R'_{c}, e.g., -N(CH₃)₂; in another embodiment, R₃ is -NR"C(O)NRₑR'_{c}; in another embodiment, R₃ is -NR"C(O)R_{c}; in another embodiment, R₃ is -NR"S(O)₂R_{c}; in another embodiment, R₃ is - OC(O)NR_{c}R'_{c}; in another embodiment, R₃ is -NR"C(O)OR_{c}; in another embodiment, R₃ is -N(OR_{c})C(O)R_{c}; in another embodiment, R₃ is -N(OR_{c})S(O)₂R_{c}; in another embodiment, R₃ is -N(OR_{c})C(O)OR_{c}; in another embodiment, R₃ is -N(OR_{c})C(O)R_{c}R'_{c}; in another embodiment, R₃ is 3- to 14-membered heterocyclyl; in another embodiment, R₃ is 5- to 14-membered heteroaryl.

In a more specific embodiment, R₃ is selected from CN, -OR_{c}, and -NR_{c}R'_{c}; in another more specific embodiment, R₃ is selected from -OR_{c} and -NR_{c}R'_{c}; in another more specific embodiment, R₃ is selected from -OH and -N(CH₃)₂.

### R_{c} and R'_{c}

In one embodiment, R_{c} is H; in another embodiment, R_{c} is C₁₋₁₀ alkyl, preferably C₁₋₆ alkyl, and preferably C₁₋₄ alkyl, e.g., Me; in another embodiment, R_{c} is C₃₋₁₀ cycloalkyl, preferably C₃₋₇ cycloalkyl; in another embodiment, R_{c} is 3- to 10-membered heterocyclyl, preferably 3- to 7-membered heterocyclyl.

In one embodiment, R'_{c} is H; in another embodiment, R'_{c} is C₁₋₁₀ alkyl, preferably C₁₋₆ alkyl, and preferably C₁₋₄ alkyl, e.g., Me; in another embodiment, R'_{c} is C₃₋₁₀ cycloalkyl, preferably C₃₋₇ cycloalkyl; in another embodiment, R'_{c} is 3- to 10-membered heterocyclyl, preferably 3- to 7-membered heterocyclyl.

In a more specific embodiment, R_{c} and R'_{c} are independently selected from H, C₁₋₆ alkyl, C₃₋₇ cycloalkyl, and 3- to 7-membered heterocyclyl; in another more specific embodiment, R_{c} and R'_{c} are independently selected from H and C₁₋₆ alkyl; in another more specific embodiment, R_{c} and R'_{c} are independently selected from H and C₁₋₄ alkyl; in another more specific embodiment, R_{c} and R'_{c} are independently selected from H and Me.

### R"

In one embodiment, R" is H; in another embodiment, R" is C₁₋₆ alkyl, preferably C₁₋₄ alkyl.

### R₄ and R₅

In one embodiment, R₄ is C₁₋₈ alkyl, preferably C₁₋₆ alkyl, preferably C₁₋₃ alkyl, preferably C₁₋₂ alkyl, and preferably methyl; in another embodiment, R₄ is optionally substituted with one or more R*; in another embodiment, R₄ is optionally substituted with 1, 2, or 3 R*; in another embodiment, R₄ is unsubstituted.

In one embodiment, R₅is C₁₋₈ alkyl, preferably C₁₋₆ alkyl, preferably C₁₋₃ alkyl, preferably C₁₋₂ alkyl, and preferably methyl; in another embodiment, R₅ is optionally substituted with one or more R*; in another embodiment, R₅ is optionally substituted with 1, 2, or 3 R*; in another embodiment, R₅ is unsubstituted.

In one embodiment, R₄ and R₅, together with the carbon atom to which they are connected, form C₃₋₁₄ cycloalkylene, preferably form C₃₋₁₀ cycloalkylene, preferably form C₃₋₆ cycloalkylene (e.g., cyclopropylene, e.g., cyclobutylene, e.g., cyclopentylene, e.g., cyclohexylene), preferably form C₃₋₅ cycloalkylene, preferably form C₃₋₄ cycloalkylene, preferably form cyclopropylene, and preferably form cyclopentylene; in another embodiment, R₄ and R₅, together with the carbon atom to which they are connected, form 3- to 14-membered heterocyclylene, preferably form 3- to 10-membered heterocyclylene, and preferably form 3- to 6-membered heterocyclylene; in another embodiment, the ring formed by R₄ and R₅ together with the carbon atom to which they are connected is optionally substituted with one or more R*; in another embodiment, the ring formed by R₄ and R₅ together with the carbon atom to which they are connected is optionally substituted with 1, 2, or 3 R*; in another embodiment, the ring formed by R₄ and R₅ together with the carbon atom to which they are connected is unsubstituted.

In a more specific embodiment, R₄ and R₅, together with the carbon atom to which they are connected, form C₃₋₁₀ cycloalkylene or 3- to 10-membered heterocyclylene; in another more specific embodiment, R₄ and R₅, together with the carbon atom to which they are connected, form C₃₋₆ cycloalkylene or 3- to 6-membered heterocyclylene; in another more specific embodiment, R₄ and R₅, together with the carbon atom to which they are connected, form C₃₋₆ cycloalkylene (e.g., cyclopropylene, cyclobutylene, cyclopentylene, or cyclohexylene); in another more specific embodiment, R₄ and R₅, together with the carbon atom to which they are connected, form C₃₋₄ cycloalkylene; in another more specific embodiment, R₄ and R₅, together with the carbon atom to which they are connected, form cyclopropylene; in another more specific embodiment, R₄ and R₅, together with the carbon atom to which they are connected, do not form a ring.

### R*

In one embodiment, R* is H; in another embodiment, R* is halogen; in another embodiment, R* is cyano; in another embodiment, R* is C₁₋₈ alkyl, preferably C₁₋₆ alkyl, and preferably C₁₋₃ alkyl; in another embodiment, R* is C₁₋₄ haloalkyl, preferably C₁₋₆ haloalkyl, and preferably C₁₋₃ haloalkyl; in another embodiment, R* is - L_{d}-OR_{d}; in another embodiment, R* is -L_{d}-SR_{d}; in another embodiment, R* is -L_{d}-NR_{d}R'_{d}.

In a more specific embodiment, R* is independently selected from H, halogen, cyano, C₁₋₆ alkyl, C₁₋₆ haloalkyl, -L_{d}-OR_{d}, -L_{d}-SR_{d}, and -L_{d}-NR_{d}R'_{d}; in another more specific embodiment, R* is independently selected from H, halogen, cyano, C₁₋₆ alkyl, and C₁₋₆ haloalkyl; in another more specific embodiment, R* is independently selected from H, C₁₋₃ alkyl, and C₁₋₃ haloalkyl.

### L_{d}, R_{d}, and R'_{d}

In one embodiment, L_{d} is a chemical bond; in another embodiment, L_{d} is C₁₋₈ alkylene, preferably C₁₋₆ alkylene, and preferably C₁₋₃ alkylene.

In a more specific embodiment, L_{d} is independently selected from a chemical bond and C₁₋₆ alkylene; in another more specific embodiment, L_{d} is independently selected from a chemical bond and C₁₋₃ alkylene.

In one embodiment, R_{d} is H; in another embodiment, R_{d} is C₁₋₄ alkyl, preferably C₁₋₆ alkyl; in another embodiment, R_{d} is C₃₋₁₄ cycloalkyl, preferably C₃₋₁₀ cycloalkyl; in another embodiment, R_{d} is 3- to 14-membered heterocyclyl, preferably 3- to 10-membered heterocyclyl.

In one embodiment, R'_{d} is H; in another embodiment, R'_{d} is C₁₋₄ alkyl, preferably C₁₋₆ alkyl; in another embodiment, R'_{d} is C₃₋₁₄ cycloalkyl, preferably C₃₋₁₀ cycloalkyl; in another embodiment, R'_{d} is 3- to 14-membered heterocyclyl, preferably 3- to 10-membered heterocyclyl.

In a more specific embodiment, R_{d} and R'_{d} are independently selected from H, C₁₋₆ alkyl, C₃₋₁₀ cycloalkyl, and 3- to 10-membered heterocyclyl; in another more specific embodiment, R_{d} and R'_{d} are independently selected from H and C₁₋₆ alkyl.

Any one of the technical solutions, or any combination thereof, in any one of the specific embodiments can be combined with any one of the technical solutions, or any combination thereof, in other specific embodiments. For example, any one of the technical solutions, or any combination thereof, of G₁ can be combined with any one of the technical solutions, or any combination thereof, of M₁, M₂, Q, Rₐ, R'ₐ, R₁, R₂, R, R', Rₛ, L_{b}, R_{b}, R'_{b}, R₃, R_{c}, R'_{c}, R", R₄, R₅, R*, L_{d}, R_{d}, R'_{d}, a, b, c, d, and the like. The present disclosure is intended to include all combinations of these technical solutions, not all of which are described herein due to limited space.

In a more specific embodiment, the present disclosure provides a compound of formula (I), or an isotopic variant, tautomer, or stereoisomer thereof, or a pharmaceutically acceptable salt thereof: 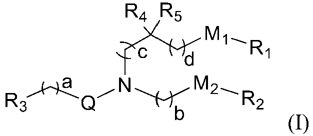 wherein,
a = 1, 2, 3, 4, 5, or 6;
b= 4, 5, 6, 7, 8, 9, or 10;
c = 1, 2, 3, 4, 5, or 6;
d = 0, 1, 2, 3, 4, or 5;
c + d = 3, 4, 5, 6, 7, 8, or 9;
M₁ and M₂ are independently selected from -C(O)O-, -OC(O)-, -O-, -SC(O)O-, -OC(O)NR-, -NRC(O)NR-, - OC(O)S-, -OC(O)O-, -NRC(O)O-, -SC(O)-, -C(O)S-, -NR-, -C(O)NR-, -NRC(O)-, -NRC(O)S-, -SC(O)NR-, -C(O)-, -OC(S)-, -C(S)O-, -OC(S)NR-, -NRC(S)O-, -S-S-, and -S(O)₀₋₂-;
Q is selected from a chemical bond, -C(O)O-, -O-, -SC(O)O-, -OC(O)NRₐ-, -NRₐC(O)NR'ₐ-, -OC(O)S-, - OC(O)O-, -NRₐC(O)O-, -OC(O)-, -SC(O)-, -C(O)S-, -NRₐ-, -C(O)NRₐ-, -NRₐC(O)-, -NRₐC(O)S-, - SC(O)NRₐ-, -C(O)-, -OC(S)-, -C(S)O-, -OC(S)NRₐ-, -NRₐC(S)O-, -S-S-, and -S(O)₀₋₂-;
Rₐ and R'ₐ are independently selected from H, C₁₋₁₀ alkyl, C₃₋₁₄ cycloalkyl, and 3- to 14-membered heterocyclyl; R₁ and R₂ are independently selected from C₄₋₂₀ alkyl, C₄₋₂₀ alkenyl, and C₄₋₂₀ alkynyl, which are optionally substituted with one or more Rₛ and in which one or more methylene units are optionally and independently replaced by -NR'-;
the substitution site of Rₛ on R₁ or R₂ is separated from M₁ or M₂ by 0-12 carbon atoms;
R and R' are each independently selected from H and C₁₋₂₀ alkyl;
Rₛ is independently selected from H, C₁₋₂₀ alkyl, -L_{b}-OR_{b}, -L_{b}-SR_{b}, and -L_{b}-NR_{b}R'_{b};
L_{b} is independently selected from a chemical bond and C₁₋₂₀ alkylene;
R_{b} and R'_{b} are independently selected from H, C₁₋₂₀ alkyl, C₃₋₁₄ cycloalkyl, and 3- to 14-membered heterocyclyl;
R₃ is selected from CN, -OR_{c}, -C(O)R_{c}, -OC(O)R_{c}, -NR"C(O)R_{c}, -NR_{c}R'_{c}, -NR"C(O)NR_{c}R'_{c}, -NR"C(O)R_{c}, - NR"S(O)₂R_{c}, -OC(O)NR_{c}R'_{c}, -NR"C(O)OR_{c}, -N(OR_{c})C(O)R_{c}, -N(OR_{c})S(O)₂R_{c}, -N(OR_{c})C(O)OR_{c}, - N(OR_{c})C(O)R_{c}R'_{c}, 3- to 14-membered heterocyclyl, and 5- to 14-membered heteroaryl;
R_{c} and R'_{c} are independently selected from H, C₁₋₁₀ alkyl, C₃₋₁₀ cycloalkyl, and 3- to 10-membered heterocyclyl;
R" is independently selected from H and C₁₋₆ alkyl;
R₄ and R₅ are independently C₁₋₈ alkyl, which is optionally substituted with one or more R*;
or R₄ and R₅, together with the carbon atom to which they are connected, form C₃₋₁₄ cycloalkylene or 3- to 14-membered heterocyclylene, which is optionally substituted with one or more R*;
R* is independently selected from H, halogen, cyano, C₁₋₄ alkyl, C₁₋₈ haloalkyl, -L_{d}-OR_{d}, -L_{d}-SR_{d}, and -L_{d}-NR_{d}R'_{d};
L_{d} is independently selected from a chemical bond and C₁₋₈ alkylene;
R_{d} and R'_{d} are independently selected from H, C₁₋₈ alkyl, C₃₋₁₄ cycloalkyl, and 3- to 14-membered heterocyclyl.

In a more specific embodiment, the present disclosure provides the compound of formula (I) described above, or the isotopic variant, tautomer, or stereoisomer thereof, or the pharmaceutically acceptable salt thereof, wherein a = 2, 3, 4, 5, or 6; preferably, a = 2, 3, 4, or 5; preferably, a = 2, 3, or 4; preferably, a = 2 or 3; preferably, a = 3 or 4; preferably, a = 2; preferably, a = 3; preferably, a = 4.

In a more specific embodiment, the present disclosure provides the compound of formula (I) described above, or the isotopic variant, tautomer, or stereoisomer thereof, or the pharmaceutically acceptable salt thereof, wherein b = 4, 5, 6, 7, 8, or 9; preferably, b = 5, 6, 7, or 8; preferably, b = 6, 7, or 8; preferably, b = 6 or 7; preferably, b = 6 or 8; preferably, b = 7 or 8; preferably, b = 7.

In a more specific embodiment, the present disclosure provides the compound of formula (I) described above, or the isotopic variant, tautomer, or stereoisomer thereof, or the pharmaceutically acceptable salt thereof, wherein c = 2, 3, 4, 5, or 6; preferably, c = 2, 3, 5, or 6; preferably, c = 3, 4, 5, or 6; preferably, c = 2, 3, 4, or 5; preferably, c = 3, 5, or 6; preferably, c = 2, 4, or 5; preferably, c = 2 or 4; preferably, c = 2 or 6; preferably, c = 5 or 6; preferably, c = 2; preferably, c = 3; preferably, c = 5; preferably, c = 6.

In a more specific embodiment, the present disclosure provides the compound of formula (I) described above, or the isotopic variant, tautomer, or stereoisomer thereof, or the pharmaceutically acceptable salt thereof, wherein d = 1, 2, 3, 4, or 5; preferably, d = 0, 1, 2, 3, or 4; preferably, d = 1, 2, 3, or 4; preferably, d = 0, 1, 2, or 3; preferably, d = 1, 2, or 4; preferably, d = 0, 1, or 2; preferably, d = 0 or 1; preferably, d = 2 or 4; preferably, d = 0 or 3; preferably, d = 0; preferably, d = 1; preferably, d = 3; preferably, d = 4.

In a more specific embodiment, the present disclosure provides the compound of formula (I) described above, or the isotopic variant, tautomer, or stereoisomer thereof, or the pharmaceutically acceptable salt thereof, wherein c + d = 5, 6, or 7; preferably, c + d = 6 or 7; preferably, c + d = 5 or 6; preferably, c + d = 6.

In a more specific embodiment, the present disclosure provides the compound of formula (I) described above, or the isotopic variant, tautomer, or stereoisomer thereof, or the pharmaceutically acceptable salt thereof, wherein M₁ and M₂ are independently selected from -C(O)O-, -C(O)S-, -OC(O)-, -SC(O)-, -OC(O)O-, - C(O)NR-, and -NRC(O)-, preferably selected from -C(O)O-, -C(O)S-, -OC(O)-, -SC(O)-, and -OC(O)O-, and preferably selected from -C(O)O-, -C(O)S-, -OC(O)-, and -SC(O)-.

In a more specific embodiment, M₁ and M₂ are independently selected from -C(O)O-, -OC(O)-, and -OC(O)O-; preferably, they are not both -OC(O)O-; preferably, they are not both -OC(O)-; preferably, they are selected from -C(O)O- and -OC(O)-.

In a more specific embodiment, M₁ is -C(O)O- or -OC(O)-, preferably -C(O)O-; M₂ is -C(O)O-.

In a more specific embodiment, M₁ is -OC(O)- or -SC(O)-; M₂ is -C(O)O- or -C(O)S-.

In a more specific embodiment, M₁ is -OC(O)-; M₂ is -C(O)O-.

In a more specific embodiment, M₁ and M₂ are independently selected from -C(O)O- and -C(O)S-, preferably -C(O)O-.

In a more specific embodiment, one of M₁ and M₂ is -OC(O)O-, and the other is selected from -C(O)O-, - C(O)S-, -OC(O)-, and -SC(O)-, preferably -C(O)O- and -OC(O)-; preferably, M₁ is selected from -C(O)O- and OC(O)-, preferably -C(O)O-, and M₂ is -OC(O)O-.

In a more specific embodiment, the present disclosure provides the compound of formula (I) described above, or the isotopic variant, tautomer, or stereoisomer thereof, or the pharmaceutically acceptable salt thereof, wherein Q is selected from a chemical bond, -OC(O)-, and -SC(O)-, preferably a chemical bond or -SC(O)-, and preferably -SC(O)- or -OC(O)-.

In a more specific embodiment, the present disclosure provides the compound of formula (I) described above, or the isotopic variant, tautomer, or stereoisomer thereof, or the pharmaceutically acceptable salt thereof, wherein Rₐ and R'ₐ are independently selected from H, C₁₋₆ alkyl, C₃₋₁₀ cycloalkyl, and 3- to 10-membered heterocyclyl, preferably selected from H and C₁₋₆ alkyl, and preferably selected from H and C₁₋₄ alkyl.

In a more specific embodiment, the present disclosure provides the compound of formula (I) described above, or the isotopic variant, tautomer, or stereoisomer thereof, or the pharmaceutically acceptable salt thereof, wherein R₁ and R₂ are independently selected from C₆₋₁₄ alkyl, C₆₋₁₄ alkenyl, and C₆₋₁₄ alkynyl, preferably selected from C₆₋₁₄ alkyl.

In a more specific embodiment, R₁ and R₂ are independently selected from C₈₋₁₂ alkyl, C₈₋₁₂ alkenyl, and C₈₋₁₂ alkynyl, preferably C₈₋₁₀ alkyl, C₈₋₁₀ alkenyl, and C₈₋₁₀ alkynyl.

In a more specific embodiment, R₁ and R₂ are independently C₈₋₁₂ alkyl, preferably C₈₋₁₁ alkyl, preferably C₉₋₁₁ alkyl, preferably C₈₋₁₀ alkyl, preferably C₉₋₁₀ alkyl, and preferably C₉ alkyl.

In a more specific embodiment, R₁ and R₂ are optionally substituted with 1, 2, 3, or 4 Rₛ, and preferably, optionally substituted with 1 Rₛ.

In a more specific embodiment, R₁ and R₂ are not both substituted; preferably, one of R₁ and R₂ is substituted, and the other is unsubstituted; preferably, R₁ is unsubstituted, and R₂ is substituted; preferably, R₁ is substituted, and R₂ is unsubstituted.

In a more specific embodiment, R₁ and R₂ are independently selected from the following groups: -(CH₂)₇CH₃, -(CH₂)₈CH₃, -(CH₂)₉CH₃, -(CH₂)₁₀CH₃, 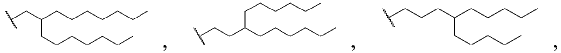 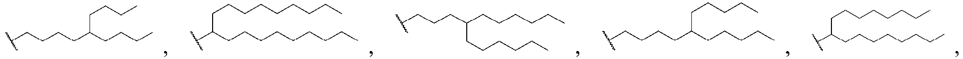 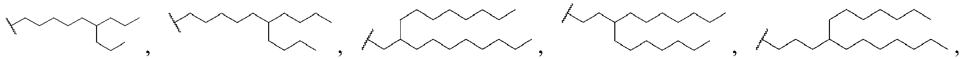 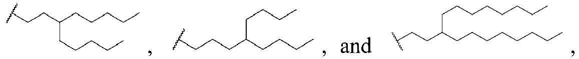 preferably selected from -(CH₂)₇CH₃, - (CH₂)₈CH₃, -(CH₂)₉CH₃, -(CH₂)₁₀CH₃,

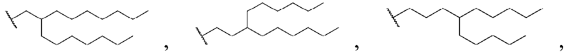

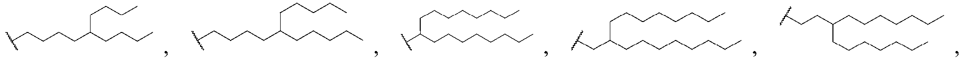

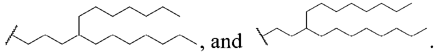

In a more specific embodiment, the present disclosure provides the compound of formula (I) described above, or the isotopic variant, tautomer, or stereoisomer thereof, or the pharmaceutically acceptable salt thereof, wherein R and R' are each independently selected from H and C₁₋₁₄ alkyl, preferably selected from H and C₁₋₁₀ alkyl, and preferably selected from H and C₁₋₆ alkyl; preferably, R is H.

In a more specific embodiment, the present disclosure provides the compound of formula (I) described above, or the isotopic variant, tautomer, or stereoisomer thereof, or the pharmaceutically acceptable salt thereof, wherein Rₛ is independently selected from H, C₁₋₁₄ alkyl, -L_{b}-OR_{b}, and -L_{b}-NR_{b}R'_{b}, preferably H and C₁₋₁₄ alkyl, preferably H and C₁₋₁₂ alkyl, preferably H and C₁₋₁₀ alkyl, preferably H and C₁₋₉ alkyl, preferably H and C₃₋₉ alkyl, preferably H and C₁₋₈ alkyl, preferably H and C₃₋₈ alkyl, preferably H and C₄₋₈ alkyl, preferably H and C₅₋₈ alkyl, preferably H and C₆₋₈ alkyl, preferably H and C₁₋₇ alkyl, preferably H and C₃₋₇ alkyl, preferably H and C₄₋₇ alkyl, preferably H and C₅₋₇ alkyl, preferably H and C₁₋₆ alkyl, preferably H and C₅₋₆ alkyl, preferably H and C₁₋₅ alkyl, preferably H and C₄₋₅ alkyl, and preferably H and C₅ alkyl.

In a more specific embodiment, Rₛ is independently preferably selected from C₁₋₁₄ alkyl, -L_{b}-OR_{b}, and -L_{b}-NR_{b}R'_{b}, preferably C₁₋₁₄ alkyl, preferably C₁₋₁₂ alkyl, preferably C₁₋₁₀ alkyl, preferably C₁₋₉ alkyl, preferably C₃₋₉ alkyl, preferably C₁₋₅ alkyl, preferably C₃₋₈ alkyl, preferably C₄₋₈ alkyl, preferably C₅₋₈ alkyl, preferably C₆₋₈ alkyl, preferably C₁₋₇ alkyl, preferably C₃₋₇ alkyl, preferably C₄₋₇ alkyl, preferably C₅₋₇ alkyl, preferably C₁₋₆ alkyl, preferably C₅₋₆ alkyl, preferably C₁₋₅ alkyl, preferably C₄₋₅ alkyl, and preferably C₅ alkyl.

In a more specific embodiment, the present disclosure provides the compound of formula (I) described above, or the isotopic variant, tautomer, or stereoisomer thereof, or the pharmaceutically acceptable salt thereof, wherein L_{b} is independently selected from a chemical bond and C₁₋₁₄ alkylene, preferably selected from C₁₋₁₀ alkylene, and preferably selected from C₁₋₆ alkylene.

In a more specific embodiment, R_{b} and R'_{b} are independently selected from H and C₁₋₁₄ alkyl, preferably selected from H and C₁₋₁₀ alkyl, and preferably selected from H and C₁₋₆ alkyl.

In a more specific embodiment, the present disclosure provides the compound of formula (I) described above, or the isotopic variant, tautomer, or stereoisomer thereof, or the pharmaceutically acceptable salt thereof, wherein R₃ is selected from CN, -OR_{c}, and -NR_{c}R'_{c}, preferably selected from -OR_{c} and -NR_{c}R'ₑ, preferably - NR_{c}R'_{c}, preferably -N(CH₃)₂, preferably -OR_{c}, and preferably OH.

In a more specific embodiment, the present disclosure provides the compound of formula (I) described above, or the isotopic variant, tautomer, or stereoisomer thereof, or the pharmaceutically acceptable salt thereof, wherein R_{c} and R'_{c} are independently selected from H, C₁₋₁₀ alkyl, C₃₋₇ cycloalkyl, and 3- to 7-membered heterocyclyl, preferably selected from H and C₁₋₁₀ alkyl, preferably selected from H and C₁₋₆ alkyl, and preferably selected from H and C₁₋₄ alkyl (e.g., methyl).

In a more specific embodiment, the present disclosure provides the compound of formula (I) described above, or the isotopic variant, tautomer, or stereoisomer thereof, or the pharmaceutically acceptable salt thereof, wherein R₄ and R₅are independently C₁₋₆ alkyl, preferably C₁₋₃ alkyl, preferably C₁₋₂ alkyl, and preferably methyl.

In a more specific embodiment, R₄ and R₅ are optionally substituted with 1, 2, or 3 R*.

In a more specific embodiment, R₄ and R₅, together with the carbon atom to which they are connected, form C₃₋₁₀ cycloalkylene or 3- to 10-membered heterocyclylene, preferably form C₃₋₆ cycloalkylene or 3- to 6-membered heterocyclylene, preferably form C₃₋₆ cycloalkylene (e.g., cyclopropylene, cyclobutylene, cyclopentylene, or cyclohexylene), preferably form C₃₋₄ cycloalkylene, and preferably form cyclopropylene. In a more specific embodiment, the ring formed by R₄, R₅, and the carbon atom to which they are connected is optionally substituted with 1, 2, or 3 R*.

In a more specific embodiment, the present disclosure provides the compound of formula (I) described above, or the isotopic variant, tautomer, or stereoisomer thereof, or the pharmaceutically acceptable salt thereof, wherein R* is independently selected from H, halogen, cyano, C₁₋₆ alkyl, C₁₋₆ haloalkyl, -L_{d}-OR_{d}, -L_{d}-SR_{d}, and -L_{d}-NR_{d}R'_{d}, preferably selected from H, halogen, cyano, C₁₋₆ alkyl, and C₁₋₆ haloalkyl, and preferably selected from H, C₁₋₃ alkyl, and C₁₋₃ haloalkyl.

In a more specific embodiment, the present disclosure provides the compound of formula (I) described above, or the isotopic variant, tautomer, or stereoisomer thereof, or the pharmaceutically acceptable salt thereof, wherein L_{d} is independently selected from a chemical bond and C₁₋₆ alkylene, preferably selected from a chemical bond and C₁₋₃ alkylene.

In a more specific embodiment, R_{d} and R'_{d} are independently selected from H, C₁₋₆ alkyl, C₃₋₁₀ cycloalkyl, and 3- to 10-membered heterocyclyl, preferably selected from H and C₁₋₆ alkyl.

In a more specific embodiment, the present disclosure provides the compound of formula (I) described above, or the isotopic variant, tautomer, or stereoisomer thereof, or the pharmaceutically acceptable salt thereof, wherein the substitution site of Rₛ on R₁ or R₂ is separated from M₁ or M₂ by 0-10 carbon atoms, preferably 0-6 carbon atoms, preferably 0-5 carbon atoms, preferably 0-4 carbon atoms, preferably 0-3 carbon atoms, and preferably 0-2 carbon atoms.

In a more specific embodiment, the substitution site of Rₛ on R₁ or R₂ is separated from M₁ or M₂ by at least 2 carbon atoms, preferably 2-4 carbon atoms, preferably 2-3 carbon atoms, and preferably 3-4 carbon atoms. In a more specific embodiment, the substitution site of Rₛ on R₁ or R₂ is separated from M₁ or M₂ by at least 1 carbon atom, preferably 1-5 carbon atoms, preferably 1-4 carbon atoms, preferably 1-3 carbon atoms, and preferably 3 carbon atoms.

In a more specific embodiment, the present disclosure provides the compound of formula (I) described above, or the isotopic variant, tautomer, or stereoisomer thereof, or the pharmaceutically acceptable salt thereof: 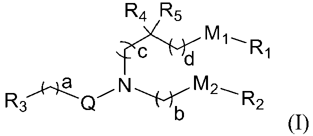 wherein,
a = 1, 2, 3, 4, 5, or 6;
b = 4, 5, 6, 7, 8, 9, or 10;
c = 1, 2, 3, 4, 5, or 6;
d = 1, 2, 3, 4, or 5;
c + d = 3, 4, 5, 6, 7, 8, or 9;
M₁ is -OC(O)- or -SC(O)-;
M₂ is -C(O)O- or -C(O)S-;
Q is selected from a chemical bond, -OC(O)-, and -SC(O)-;
R₁ and R₂ are independently selected from C₆₋₁₄ alkyl, C₆₋₁₄ alkenyl, and C₆₋₁₄ alkynyl;
one of R₁ and R₂ is substituted with 1, 2, 3, or 4 Rₛ, and the other is unsubstituted;
the substitution site of Rₛ on R₁ or R₂ is separated from M₁ or M₂ by at least 1 carbon atom;
Rₛ is independently selected from C₁₋₁₄ alkyl, -L_{b}-OR_{b}, and -L_{b}-NR_{b}R'_{b}, preferably C₁₋₁₄ alkyl;
L_{b} is independently selected from a chemical bond and C₁₋₁₄ alkylene;
R_{b} and R'_{b} are independently selected from H and C₁₋₁₄ alkyl;
R₃ is selected from -OR_{c} and -NR_{c}R'_{c};
R_{c} and R'_{c} are independently selected from H or C₁₋₅ alkyl;
R₄ and R₅ are independently C₁₋₅ alkyl;
or R₄ and R₅, together with the carbon atom to which they are connected, form C₃₋₆ cycloalkylene or 3- to 6-membered heterocyclylene.

In a more specific embodiment, the present disclosure provides the compound of formula (I) described above, or the isotopic variant, tautomer, or stereoisomer thereof, or the pharmaceutically acceptable salt thereof, wherein
a = 2, 3, 4, or 5;
b = 5, 6, 7, or 8;
c = 1, 2, 3, 4, 5, or 6;
d = 1, 2, 3, 4, or 5;
c + d = 5, 6, or 7;
M₁ is -OC(O)- or -SC(O)-;
M₂ is -C(O)O- or -C(O)S-;
Q is selected from a chemical bond, -OC(O)-, and -SC(O)-;
R₁ and R₂ are independently selected from C₈₋₁₂ alkyl, C₈₋₁₂ alkenyl, C₈₋₁₂ alkynyl, and preferably C₈₋₁₂ alkyl;
one of R₁ and R₂ is substituted with 1 R₅, and the other is unsubstituted;
the substitution site of Rₛ on R₁ or R₂ is separated from M₁ or M₂ by at least 1 carbon atom;
Rₛ is C₁₋₁₂ alkyl;
R₃ is selected from -OR_{c} and -NR_{c}R'_{c};
R_{c} and R'_{c} are independently H and C₁₋₆ alkyl;
R₄ and R₅are independently C₁₋₃ alkyl;
or R₄ and R₅, together with the carbon atom to which they are connected, form C₃₋₆ cycloalkylene.

In a more specific embodiment, the present disclosure provides the compound of formula (I) described above, or the isotopic variant, tautomer, or stereoisomer thereof, or the pharmaceutically acceptable salt thereof, wherein
a = 2, 3, or 4;
b = 6, 7, or 8;
c = 2, 3, 4, 5, or 6;
d = 1, 2, 3, 4, or 5;
c + d = 6 or 7;
M₁ is -OC(O)-; M₂ is -C(O)O-;
Q is a chemical bond or -SC(O)-;
R₁ and R₂ are independently C₈₋₁₂ alkyl, preferably C₈₋₁₁ alkyl, and preferably C₉₋₁₁ alkyl;
one of R₁ and R₂ is substituted with 1 Rₛ, and the other is unsubstituted;
the substitution site of Rₛ on R₁ or R₂ is separated from M₁ or M₂ by at least 1 carbon atom, preferably 1-4 carbon atoms;
Rₛ is C₁₋₁₀ alkyl, preferably C₁₋₅ alkyl, preferably C₃₋₈ alkyl, and preferably C₄₋₈ alkyl;
R₃ is selected from -OR_{c} and -NR_{c}R'_{c}, preferably -OH and -N(CH₃)₂;
R_{c} and R'_{c} are independently H and C₁₋₄ alkyl, preferably H and methyl;
R₄ and R₅are independently C₁₋₃ alkyl, preferably methyl;
or R₄ and R₅, together with the carbon atom to which they are connected, form C₃₋₄ cycloalkylene, preferably cyclopropylene.

In a more specific embodiment, one of R₁ and R₂ is selected from -(CH₂)₇CH₃, -(CH₂)₈CH₃, -(CH₂)₉CH₃, and -(CH₂)₁₀CH₃, preferably -(CH₂)₈CH₃, -(CH₂)₉CH₃, and -(CH₂)₁₀CH₃; the other is selected from: 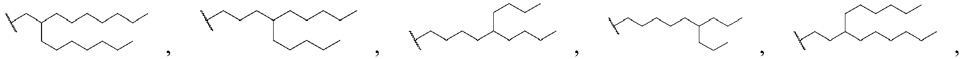 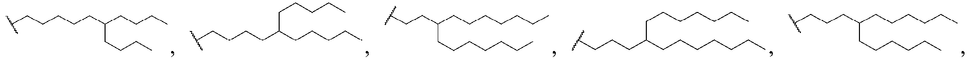 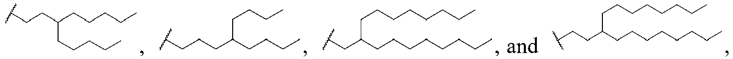 preferably selected from:

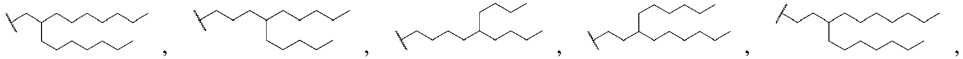

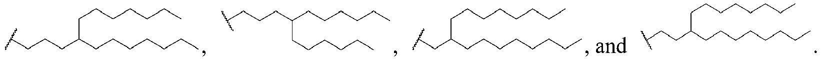

In a more specific embodiment, the present disclosure provides the compound of formula (I) described above, or the isotopic variant, tautomer, or stereoisomer thereof, or the pharmaceutically acceptable salt thereof, which has a structure of formula (II) or formula (III): 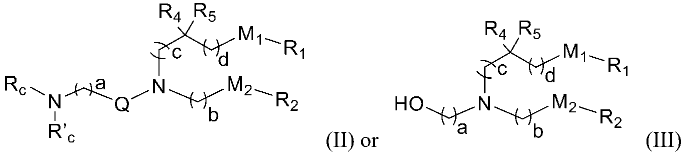 wherein the variables are as defined in the present disclosure.

In a more specific embodiment, the present disclosure provides the compound of formula (II) described above, or the isotopic variant, tautomer, or stereoisomer thereof, or the pharmaceutically acceptable salt thereof, 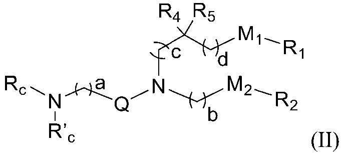 wherein,
a = 2, 3, 4, 5, or 6; preferably, a = 2, 3, 4, or 5;
b = 4, 5, 6, 7, 8, or 9; preferably, b = 5, 6, 7, or 8;
c = 1, 2, 3, 4, 5, or 6;
d = 1, 2, 3, 4, or 5;
c + d = 5, 6, or 7;
M₁ and M₂ are independently selected from -C(O)O-, -C(O)S-, -OC(O)-, -SC(O)-, and -OC(O)O-, preferably selected from -C(O)O-, -C(O)S-, -OC(O)-, and -SC(O)-;
Q is -SC(O)- or -OC(O)-;
R₁ and R₂ are independently C₆₋₁₄ alkyl, which is optionally substituted with 1, 2, 3, or 4 Rₛ;
the substitution site of Rₛ on R₁ or R₂ is separated from M₁ or M₂ by 0-5 carbon atoms;
Rₛ is independently selected from C₁₋₁₄ alkyl, -L_{b}-OR_{b}, and -L_{b}-NR_{b}R'_{b}, preferably C₁₋₁₄ alkyl;
L_{b} is independently selected from a chemical bond and C₁₋₁₄ alkylene;
R_{b} and R'_{b} are independently selected from H and C₁₋₁₄ alkyl;
R_{c} and R'_{c} are independently C₁₋₅ alkyl;
R₄ and R₅ are independently C₁₋₅ alkyl;
or R₄ and R₅, together with the carbon atom to which they are connected, form C₃₋₆ cycloalkylene or 3- to 6-membered heterocyclylene.

In a more specific embodiment, the present disclosure provides the compound of formula (II) described above, or the isotopic variant, tautomer, or stereoisomer thereof, or the pharmaceutically acceptable salt thereof, wherein
a = 3 or 4;
b = 6 or 7;
c = 2, 3, 4, or 5; preferably, c = 2, 4, or 5;
d = 1, 2, 3, or 4; preferably, d = 1, 2, or 4;
c + d = 6;
M₁ and M₂ are independently selected from -C(O)O-, -OC(O)-, and -OC(O)O-; preferably, they are not both - OC(O)O-; preferably, they are not both -OC(O)-; preferably, they are selected from -C(O)O- and -OC(O)-; Q is -SC(O)-;
R₁ and R₂ are independently C₈₋₁₂ alkyl, preferably C₈₋₁₀ alkyl;
one of R₁ and R₂ is substituted with 1 Rₛ, and the other is unsubstituted;
the substitution site of Rₛ on R₁ or R₂ is separated from M₁ or M₂ by 0-4 carbon atoms;
Rₛ is C₁₋₁₂ alkyl, preferably C₁₋₉ alkyl, preferably C₃₋₉ alkyl, preferably C₁₋₅ alkyl, and preferably C₅₋₈ alkyl; R_{c} and R'_{c} are independently C₁₋₄ alkyl, preferably methyl;
R₄ and R₅ are independently C₁₋₃ alkyl, preferably methyl;
or R₄ and R₅, together with the carbon atom to which they are connected, form C₃₋₆ cycloalkylene, preferably C₃₋₄ cycloalkylene, e.g., cyclopropylene.

In a more specific embodiment, R₁ and R₂ are independently selected from: -(CH₂)₇CH₃, -(CH₂)₈CH₃, - (CH₂)₉CH₃, 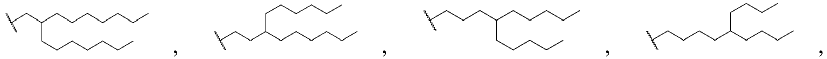 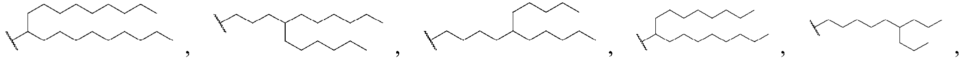 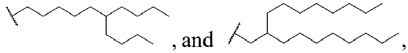 preferably selected from -(CH₂)₇CH₃, -(CH₂)₈CH₃, -(CH₂)₉CH₃, 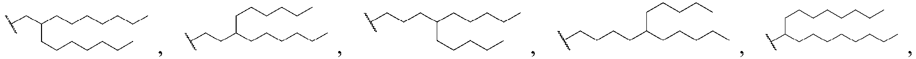 and

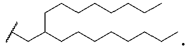

In a more specific embodiment, R₁ is unsubstituted, and R₂ is substituted.

In a more specific embodiment, the present disclosure provides the compound of formula (II) described above, or the isotopic variant, tautomer, or stereoisomer thereof, or the pharmaceutically acceptable salt thereof, wherein
a = 3 or 4; preferably, a = 4;
b = 6 or 7; preferably, b = 7;
c = 2 or 4, preferably, c = 2;
d = 2 or 4; preferably, d = 4;
c + d =6;
M₁ is -C(O)O- or -OC(O)-, preferably -C(O)O-; M₂ is -C(O)O-;
Q is -SC(O)-;
R₁ is selected from C₈₋₁₂ linear alkyl, preferably C₉₋₁₀ linear alkyl;
R₂ is selected from C₈₋₁₂ alkyl, preferably C₉₋₁₀ alkyl, which is substituted with 1 Rₛ;
the substitution site of Rₛ on R₂ is separated from M₂ by at least 2 carbon atoms;
Rₛ is C₁₋₆ alkyl, preferably C₅₋₆ alkyl, preferably C₁₋₅ alkyl, and preferably C₅ alkyl;
R_{c} and R'_{c} are independently C₁₋₄ alkyl, preferably methyl;
R₄ and R₅ are independently C₁₋₃ alkyl, preferably methyl;
preferably, the substitution site of Rₛ on R₂ is separated from M₂ by 2-4 carbon atoms, preferably 3-4 carbon atoms.

In a more specific embodiment, R₂ is selected from: 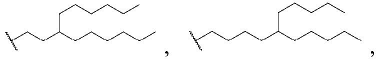 and 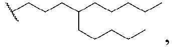 preferably

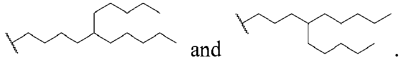

In a more specific embodiment, the present disclosure provides the compound of formula (II) described above, or the isotopic variant, tautomer, or stereoisomer thereof, or the pharmaceutically acceptable salt thereof, wherein
a = 3 or 4; preferably, a = 4;
b = 7;
c = 5;
d = 1;
M₁ is -OC(O)-; M₂ is -C(O)O-;
Q is -SC(O)-;
R₁ is selected from C₈₋₁₂ linear alkyl, preferably C₉₋₁₀ linear alkyl, and preferably C₉ linear alkyl;
R₂ is selected from C₈₋₁₂ alkyl, preferably C₉₋₁₀ alkyl, and preferably C₉ alkyl, which is substituted with 1 Rₛ;
the substitution site of Rₛ on R₂ is separated from M₂ by 0-3 carbon atoms;
Rₛ is C₁₋₈ alkyl, preferably C₅₋₈ alkyl;
R_{c} and R'_{c} are independently C₁₋₄ alkyl, preferably methyl;
R₄ and R₅ are independently C₁₋₃ alkyl, preferably methyl;
or R₄ and R₅, together with the carbon atom to which they are connected, form C₃₋₄ cycloalkylene, e.g., cyclopropylene.

In a more specific embodiment, R₂ is selected from: 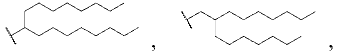 and

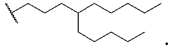

In a more specific embodiment, the present disclosure provides the compound of formula (III) described above, or the isotopic variant, tautomer, or stereoisomer thereof, or the pharmaceutically acceptable salt thereof, 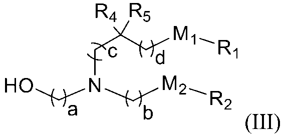 wherein,
a = 2, 3, 4, 5, or 6; preferably, a = 2, 3, 4, or 5;
b = 4, 5, 6, 7, 8, or 9; preferably, b = 5, 6, 7, or 8;
c = 1, 2, 3, 4, 5, or 6;
d = 1, 2, 3, 4, or 5;
c + d = 5, 6, or 7;
M₁ is -OC(O)- or -SC(O)-; M₂ is -C(O)O- or -C(O)S-;
R₁ and R₂ are independently C₆₋₁₄ alkyl, which is optionally substituted with 1, 2, 3, or 4 Rₛ;
the substitution site of Rₛ on R₁ or R₂ is separated from M₁ or M₂ by at least 1 carbon atom, preferably 1-5 carbon atoms;
Rₛ is independently selected from H, C₁₋₁₄ alkyl, -L_{b}-OR_{b}, and -L_{b}-NR_{b}R'_{b}, preferably selected from H and C₁₋₁₄ alkyl;
L_{b} is independently selected from a chemical bond and C₁₋₁₄ alkylene;
R_{b} and R'_{b} are independently H and C₁₋₁₄ alkyl;
R₄ and R₅ are independently C₁₋₆ alkyl;
or R₄ and R₅, together with the carbon atom to which they are connected, form C₃₋₆ cycloalkylene or 3- to 6-membered heterocyclylene.

In a more specific embodiment, the present disclosure provides the compound of formula (III) described above, or the isotopic variant, tautomer, or stereoisomer thereof, or the pharmaceutically acceptable salt thereof, wherein
a = 2, 3, or 4;
b = 6, 7, or 8;
c = 2, 3, 4, 5, or 6; preferably, c = 2, 3, 5, or 6;
d = 1, 2, 3, or 4;
c + d = 6 or 7;
M₁ is -OC(O)-; M₂ is -C(O)O-;
R₁ and R₂ are independently C₈₋₁₂ alkyl, preferably C₈₋₁₁ alkyl, and preferably C₉₋₁₁ alkyl;
one of R₁ and R₂ is substituted with 1 Rₛ, and the other is unsubstituted;
the substitution site of Rₛ on R₁ or R₂ is separated from M₁ or M₂ by 1-5 carbon atoms, preferably 1-4 carbon atoms;
Rₛ is C₁₋₁₂ alkyl, preferably C₁₋₁₀ alkyl, preferably C₁₋₈ alkyl, preferably C₃₋₈ alkyl, and preferably C₄₋₈ alkyl; R₄ and R₅ are independently C₁₋₃ alkyl, preferably methyl;
or R₄ and R₅, together with the carbon atom to which they are connected, form C₃₋₆ cycloalkylene, preferably C₃₋₄ cycloalkylene, e.g., cyclopropylene.

In a more specific embodiment, R₁ and R₂ are independently selected from: -(CH₂)₇CH₃, -(CH₂)₈CH₃, - (CH₂)₉CH₃, -(CH₂)₁₀CH₃, 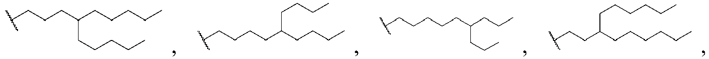 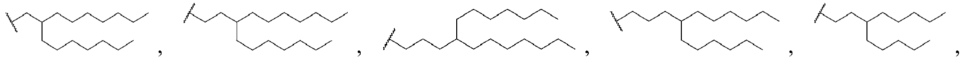 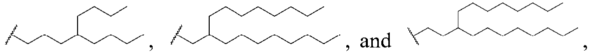 preferably selected from -(CH₂)₈CH₃, - (CH₂)₉CH₃, -(CH₂)₁₀CH₃,

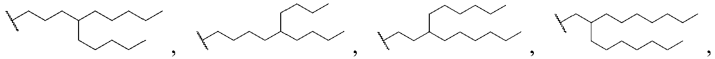

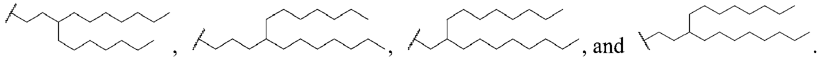

In a more specific embodiment, R₁ is unsubstituted, and R₂ is substituted.

In a more specific embodiment, the present disclosure provides the compound of formula (III) described above, or the isotopic variant, tautomer, or stereoisomer thereof, or the pharmaceutically acceptable salt thereof, wherein
a = 2, 3, or 4; preferably, a = 2 or 3;
b = 6 or 7; preferably, b = 7;
c = 5;
d = 1;
M₁ is -OC(O)-; M₂ is -C(O)O-;
R₁ and R₂ are independently C₈₋₁₂ alkyl, preferably C₈₋₁₀ alkyl, preferably C₉₋₁₀ alkyl, and preferably C₉ alkyl; one of R₁ and R₂ is substituted with 1 Rₛ, and the other is unsubstituted;
the substitution site of Rₛ on R₁ or R₂ is separated from M₁ or M₂ by at least 1 carbon atom;
Rₛ is C₁₋₁₂ alkyl, preferably C₁₋₁₀ alkyl, preferably C₁₋₇ alkyl, preferably C₃₋₇ alkyl, preferably C₄₋₇ alkyl, and preferably C₅₋₇ alkyl;
R₄ and R₅ are independently C₁₋₃ alkyl, preferably methyl;
or R₄ and R₅, together with the carbon atom to which they are connected, form C₃₋₆ cycloalkylene, preferably C₃₋₄ cycloalkylene, e.g., cyclopropylene; preferably, R₄ and R₅, together with the carbon atom to which they are connected, do not form a ring.
In a more specific embodiment, the substitution site of Rₛ on R₁ or R₂ is separated from M₁ or M₂ by 1-4 carbon atoms, preferably 1-3 carbon atoms.

In a more specific embodiment, one of R₁ and R₂ is selected from C₉₋₁₀ linear alkyl, preferably C₉ linear alkyl, and the other is selected from: 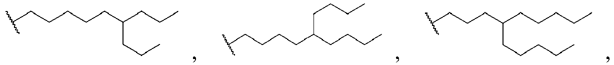 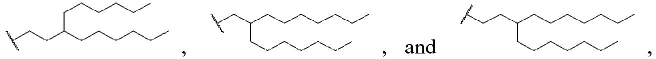 preferably

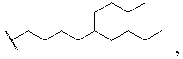

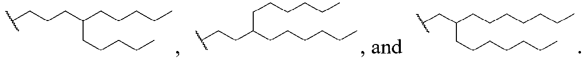

In a more specific embodiment, R₁ is unsubstituted, and R₂ is substituted;
R₁ is selected from C₈₋₁₀ linear alkyl, preferably C₉₋₁₀ linear alkyl, and preferably C₉ linear alkyl;
R₂ is selected from C₈₋₁₀ alkyl, preferably C₉₋₁₀ alkyl, and preferably C₉ alkyl, which is substituted with 1 Rₛ.

In a more specific embodiment, R₁ is substituted, and R₂ is unsubstituted;
R₁ is selected from C₈₋₁₀ alkyl, preferably C₉₋₁₀ alkyl, and preferably C₉ alkyl, which is substituted with 1 Rₛ;
R₂ is selected from C₈₋₁₀ linear alkyl, preferably C₉₋₁₀ linear alkyl, and preferably C₉ linear alkyl;
Rₛ is C₁₋₁₀ alkyl, preferably C₁₋₅ alkyl, preferably C₄₋₅ alkyl, and preferably C₅ alkyl.

In a more specific embodiment, the present disclosure provides the compound of formula (III) described above, or the isotopic variant, tautomer, or stereoisomer thereof, or the pharmaceutically acceptable salt thereof, wherein
a = 2, 3, or 4; preferably, a = 3 or 4;
b = 6, 7, or 8; preferably, b = 6 or 8;
c = 5 or 6;
d = 1 or 2;
c + d = 7;
M₁ is -OC(O)-; M₂ is -C(O)O-;
R₁ is selected from C₈₋₁₂ linear alkyl, preferably C₈₋₁₀ linear alkyl, preferably C₈₋₉ linear alkyl, and preferably C₉ linear alkyl;
R₂ is selected from C₈₋₁₂ alkyl, preferably C₉₋₁₁ alkyl, which is substituted with 1 Rₛ;
the substitution site of Rₛ on R₁ or R₂ is separated from M₁ or M₂ by at least 2 carbon atoms, preferably 2-3 carbon atoms;
Rₛ is C₁₋₁₂ alkyl, preferably C₁₋₁₀ alkyl, preferably C₁₋₇ alkyl, and preferably C₆₋₇ alkyl;
R₄ and R₅ are independently C₁₋₃ alkyl, preferably methyl;
or R₄ and R₅, together with the carbon atom to which they are connected, form C₃₋₆ cycloalkylene, preferably C₃₋₄ cycloalkylene, e.g., cyclopropylene; preferably, R₄ and R₅, together with the carbon atom to which they are connected, do not form a ring;
preferably, R₂ is selected from:

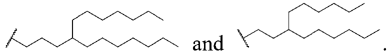

In a more specific embodiment, the present disclosure provides the compound of formula (III) described above, or the isotopic variant, tautomer, or stereoisomer thereof, or the pharmaceutically acceptable salt thereof, wherein
a = 2, 3, or 4; preferably, a = 3;
b = 7 or 8; preferably, b = 7;
c = 3;
d = 3;
M₁ is -OC(O)-; M₂ is -C(O)O-;
R₁ is selected from C₈₋₁₂ linear alkyl, preferably C₉₋₁₁ linear alkyl;
R₂ is selected from C₈₋₁₂ alkyl, preferably C₉₋₁₁ alkyl, which is substituted with 1 Rₛ;
the substitution site of Rₛ on R₂ is separated from M₂ by at least 1 carbon atoms;
Rₛ is C₁₋₁₂ alkyl, preferably C₁₋₁₀ alkyl, preferably C₁₋₈ alkyl, and preferably C₆₋₈ alkyl;
R₄ and R₅ are independently C₁₋₃ alkyl, preferably methyl;
or R₄ and R₅, together with the carbon atom to which they are connected, form C₃₋₆ cycloalkylene, preferably C₃₋₄ cycloalkylene, e.g., cyclopropylene.

In a more specific embodiment, the substitution site of Rₛ on R₂ is separated from M₂ by 1-3 carbon atoms.

In a more specific embodiment, R₂ is selected from:

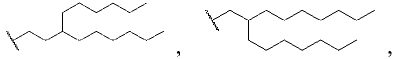

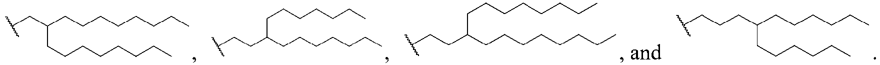

In a more specific embodiment, the present disclosure provides the compound of formula (III) described above, or the isotopic variant, tautomer, or stereoisomer thereof, or the pharmaceutically acceptable salt thereof, wherein
a = 2, 3, or 4; preferably, a = 3;
b = 7 or 8; preferably, b = 7;
c=2;
d=4;
M₁ is -OC(O)-; M₂ is -C(O)O-;
R₁ is selected from C₈₋₁₂ linear alkyl, preferably C₉₋₁₁ linear alkyl, and preferably C₁₀ linear alkyl;
R₂ is selected from C₈₋₁₂ alkyl, preferably C₉₋₁₁ alkyl, and preferably C₁₀ alkyl, which is substituted with 1 Rₛ; the substitution site of Rₛ on R₂ is separated from M₂ by at least 1 carbon atoms;
Rₛ is C₁₋₁₂ alkyl, preferably C₁₋₁₀ alkyl, preferably C₁₋₈ alkyl, and preferably C₆₋₈ alkyl;
R₄ and R₅ are independently C₁₋₃ alkyl, preferably methyl;
or R₄ and R₅, together with the carbon atom to which they are connected, form C₃₋₆ cycloalkylene, preferably C₃₋₄ cycloalkylene, e.g., cyclopropylene; preferably, R₄ and R₅, together with the carbon atom to which they are connected, do not form a ring.

In a more specific embodiment, the substitution site of Rₛ on R₂ is separated from M₂ by 1-3 carbon atoms.

In a more specific embodiment, R₂ is selected from: 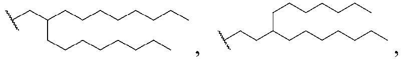 and 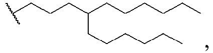 preferably

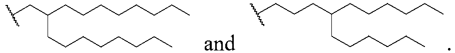

In a more specific embodiment, the present disclosure provides the compound of formula (III) described above, or the isotopic variant, tautomer, or stereoisomer thereof, or the pharmaceutically acceptable salt thereof, 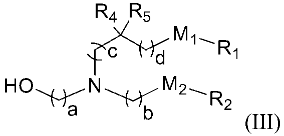 wherein,
a = 2, 3, 4, 5, or 6; preferably, a = 2, 3, or 4;
b = 4, 5, 6, 7, 8, or 9; preferably, b = 5, 6, 7, or 8;
c = 1, 2, 3, 4, 5, or 6;
d = 0, 1, 2, 3, 4, or 5;
c + d = 5, 6, or 7;
M₁ and M₂ are independently selected from -C(O)O- and -C(O)S-, preferably -C(O)O-; or one of M₁ and M₂ is -OC(O)O-, and the other is selected from -C(O)O-, -C(O)S-, -OC(O)-, and -SC(O)-, preferably -C(O)O- and -OC(O)-;
R₁ and R₂ are independently C₆₋₁₄ alkyl, which is optionally substituted with 1, 2, 3, or 4 Rₛ;
the substitution site of Rₛ on R₁ or R₂ is separated from M₁ or M₂ by 0-4 carbon atoms;
Rₛ is independently selected from H, C₁₋₁₄ alkyl, -L_{b}-OR_{b}, and -L_{b}-NR_{b}R'_{b}, preferably selected from H and C₁₋₁₄ alkyl;
L_{b} is independently selected from a chemical bond and C₁₋₁₄ alkylene;
R_{b} and R'_{b} are independently selected from H and C₁₋₁₄ alkyl;
R₄ and R₅ are independently C₁₋₆ alkyl;
or R₄ and R₅, together with the carbon atom to which they are connected, form C₃₋₆ cycloalkylene or 3- to 6-membered heterocyclylene.

In a more specific embodiment, the present disclosure provides the compound of formula (III) described above, or the isotopic variant, tautomer, or stereoisomer thereof, or the pharmaceutically acceptable salt thereof, wherein
a = 2, 3, or 4; preferably, a = 2;
b = 5, 6, or 7; preferably, b = 5 or 7;
c = 2, 3, 4, 5, or 6; preferably, c = 2 or 6; preferably, c = 6;
d = 0, 1, 2, or 3; preferably, d = 0 or 3; preferably, d = 0;
c + d = 5 or 6; preferably, c + d = 6;
M₁ and M₂ are -C(O)O-;
R₁ is selected from C₈₋₁₂ alkyl, preferably C₈₋₁₀ alkyl, preferably C₉₋₁₀ alkyl, and preferably C₉ alkyl, which is substituted with 1 Rₛ;
the substitution site of Rₛ on R₁ is separated from M₁ by at least 1 carbon atoms;
R₂ is selected from C₈₋₁₂ linear alkyl, preferably C₉₋₁₁ linear alkyl;
Rₛ is C₁₋₁₂ alkyl, preferably C₁₋₁₀ alkyl, preferably C₁₋₅ alkyl, and preferably C₅ alkyl;
R₄ and R₅ are independently C₁₋₃ alkyl, preferably methyl;
or R₄ and R₅, together with the carbon atom to which they are connected, form C₃₋₆ cycloalkylene, preferably C₃₋₄ cycloalkylene, e.g., cyclopropylene; preferably, R₄ and R₅, together with the carbon atom to which they are connected, do not form a ring.

In a more specific embodiment, the substitution site of Rₛ on R₁ is separated from M₁ by 1-3 carbon atoms, preferably 3 carbon atoms.

In a more specific embodiment, R₁ is

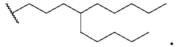

In a more specific embodiment, the present disclosure provides the compound of formula (III) described above, or the isotopic variant, tautomer, or stereoisomer thereof, or the pharmaceutically acceptable salt thereof, wherein
a = 2, 3, or 4; preferably, a = 3 or 4;
b = 5, 6, or 7; preferably, b = 6 or 7;
c = 3, 4, 5, or 6; preferably, c = 3, 5, or 6; preferably, c = 5 or 6; preferably, c = 6;
d = 0, 1, or 2; preferably, d = 0 or 1; preferably, d = 0;
c + d = 5 or 6; preferably, c + d = 6;
one of M₁ and M₂ is -OC(O)O-, and the other is selected from -C(O)O- and -OC(O)-; preferably, M₁ is selected from -C(O)O- and OC(O)-, preferably -C(O)O-, and M₂ is -OC(O)O-;
R₁ and R₂ are independently C₈₋₁₂ alkyl, preferably C₈₋₁₀ alkyl, preferably C₉₋₁₀ alkyl, and preferably C₉ alkyl; one of R₁ and R₂ is substituted with 1 Rₛ, and the other is unsubstituted;
the substitution site of Rₛ on R₁ or R₂ is separated from M₁ or M₂ by 0-4 carbon atoms;
Rₛ is C₁₋₁₂ alkyl, preferably C₁₋₁₀ alkyl, preferably C₁₋₈ alkyl, and preferably C₆₋₈ alkyl;
R₄ and R₅ are independently C₁₋₃ alkyl, preferably methyl;
or R₄ and R₅, together with the carbon atom to which they are connected, form C₃₋₆ cycloalkylene, preferably C₃₋₄ cycloalkylene, e.g., cyclopropylene; preferably, R₄ and R₅, together with the carbon atom to which they are connected, do not form a ring.

In a more specific embodiment, the substitution site of Rₛ on R₁ or R₂ is separated from M₁ or M₂ by 0-3 carbon atoms, preferably 0-2 carbon atoms.

In a more specific embodiment, one of R₁ and R₂ is C₉ linear alkyl, and the other is selected from: 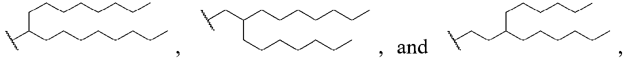 preferably 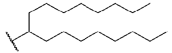 and

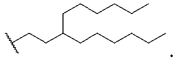

In a more specific embodiment, the present disclosure provides the compound of formula (I) described above, or the isotopic variant, tautomer, or stereoisomer thereof, or the pharmaceutically acceptable salt thereof, wherein the compound is selected from compounds in Table 1.

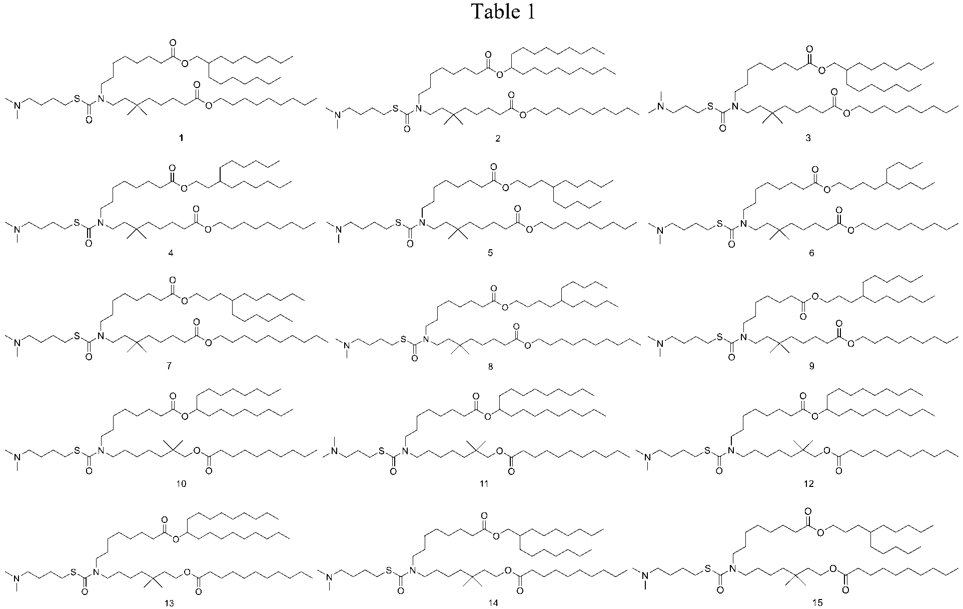

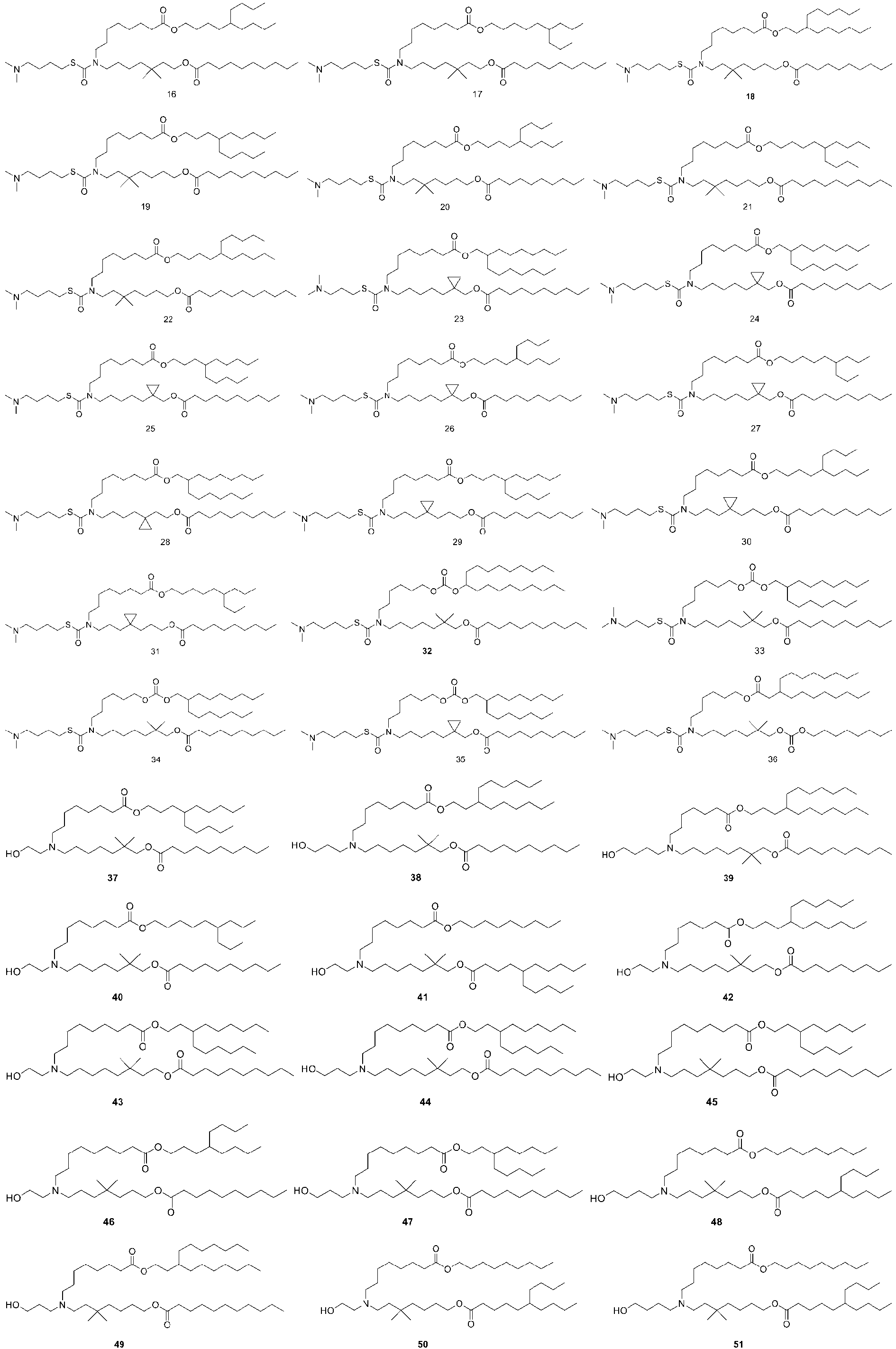

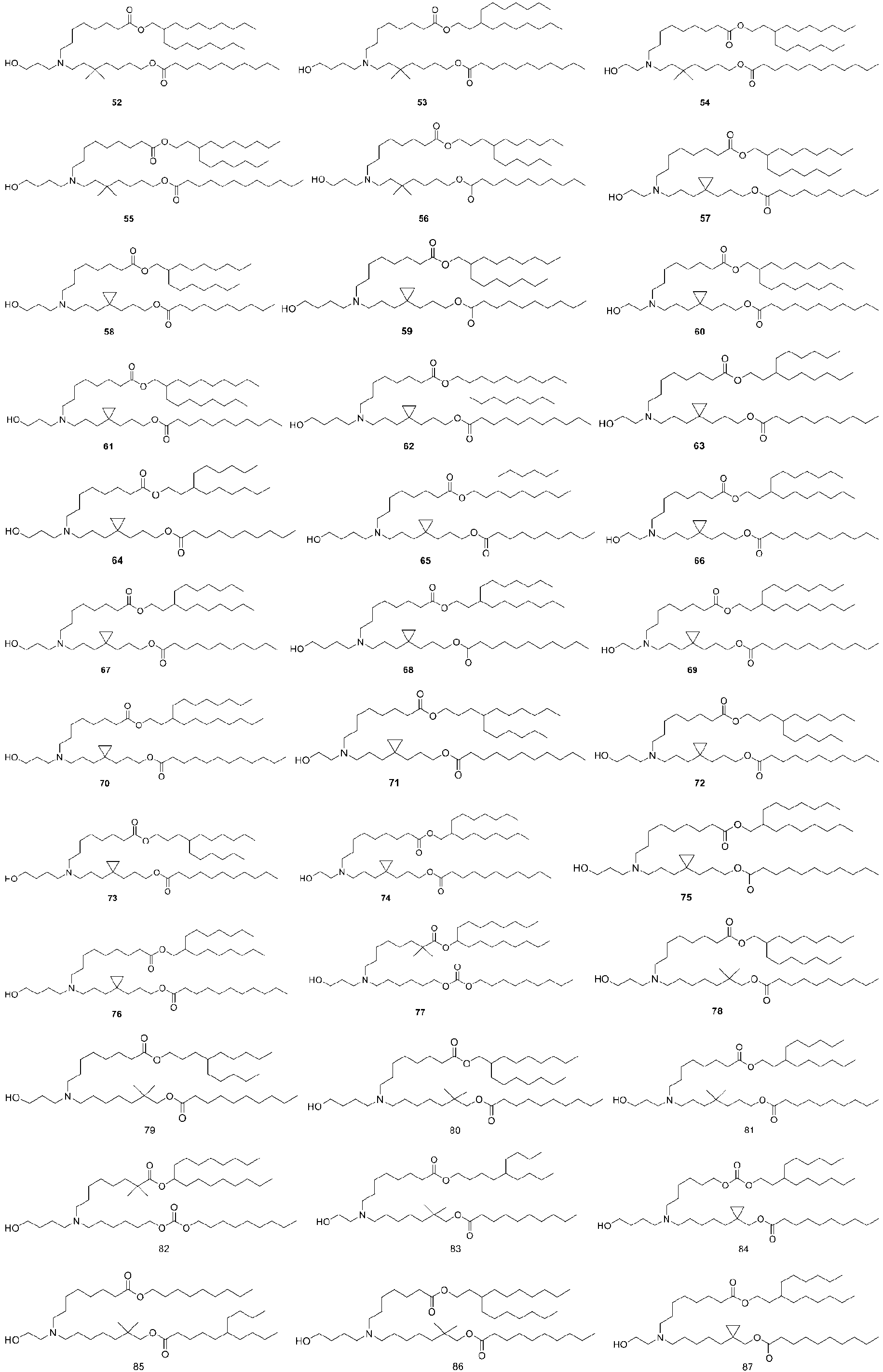

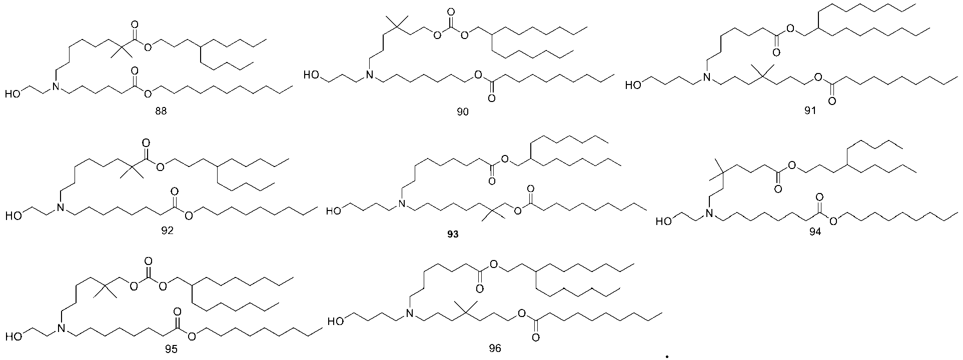

In a more specific embodiment, the present disclosure further provides a pharmaceutical composition comprising the compound of the present disclosure, or the isotopic variant, tautomer, or stereoisomer thereof, or the pharmaceutically acceptable salt thereof, and a pharmaceutically acceptable auxiliary material.

In a more specific embodiment, the present disclosure further provides a nanoparticle composition comprising a lipid component, and optionally a load, wherein the lipid component comprises the compound of the present disclosure, or the isotopic variant, tautomer, or stereoisomer thereof, or the pharmaceutically acceptable salt thereof, and the load is selected from one or more of a therapeutic agent, a prophylactic agent, and a diagnostic agent.

In a more specific embodiment, the present disclosure provides a nanoparticle composition comprising a lipid component, and optionally a load, wherein the lipid component comprises the compound of the present disclosure.

Optionally, the lipid component comprises the following components in molar percentage:
20 mol%-85 mol% of any of the compounds of the present disclosure described above;
10 mol%-75 mol% of a structural lipid;
1.0 mol%-30 mol% of a neutral lipid;
0.25 mol%-10 mol% of a polymeric lipid;
optionally, the lipid component comprises the following components in molar percentage:
50 mol% of any of the compounds of the present disclosure described above;
10 mol% of a neutral lipid;
38.5 mol% of a structural lipid;
1.5 mol% of a polymeric lipid.

In a more specific embodiment, the present disclosure provides the nanoparticle composition described above, wherein the neutral lipid is selected from one or more of DSPC, DMPC, DOPC, DPPC, POPC, DOPE, DMPE, POPE, and DPPE, preferably DSPC and/or DOPE.

In a more specific embodiment, the present disclosure provides the nanoparticle composition described above, wherein the structural lipid is selected from one or more of cholesterol, sitosterol, coprosterol, fucosterol, brassicasterol, ergosterol, tomatine, ursolic acid, α-tocopherol, stigmasterol, avenasterol, ergocalciferol, and campesterol, preferably cholesterol and/or β-sitosterol, and more preferably cholesterol.

In a more specific embodiment, the present disclosure provides the nanoparticle composition described above, wherein the polymeric lipid is a PEGylated lipid.

Optionally, the PEGylated lipid is selected from one or more of PEG-modified phosphatidylethanolamine, PEG-modified phosphatidic acid, PEG-modified ceramide, PEG-modified dialkylamine, PEG-modified diacylglycerol, and PEG-modified dialkylglycerol.

Optionally, the PEGylated lipid comprises a PEG moiety of about 1000 Da to about 20 kDa, preferably a PEG moiety of about 1000 Da to about 5000 Da.

Optionally, the PEGylated lipid is selected from one or more of DMPE-PEG1000, DPPE-PEG1000, DSPE-PEG1000, DOPE-PEG1000, DMG-PEG2000, Ceramide-PEG2000, DMPE-PEG2000, DPPE-PEG2000, DSPE-PEG2000, Azido-PEG2000, DSPE-PEG2000-Mannose, Ceramide-PEG5000, and DSPE-PEG5000, preferably DMG-PEG2000.

In a more specific embodiment, the present disclosure further provides use of the compound of the present disclosure, or the isotopic variant, tautomer, or stereoisomer thereof, or the pharmaceutically acceptable salt thereof, or the pharmaceutical composition of the present disclosure, or the nanoparticle composition of the present disclosure in the manufacture of a medicament for treating, diagnosing, or preventing a disease, wherein preferably, the medicament for treating, diagnosing, or preventing a disease is a therapeutic or prophylactic mRNA vaccine.

In a more specific embodiment, the present disclosure further provides use of the compound of the present disclosure, or the isotopic variant, tautomer, or the stereoisomer thereof, or the pharmaceutically acceptable salt thereof, or the pharmaceutical composition of the present disclosure, or the nanoparticle composition of the present disclosure in the manufacture of a medicament for delivering a load, wherein the load is selected from one or more of a therapeutic agent, a prophylactic agent, and a diagnostic agent.

In a more specific embodiment, the present disclosure further provides a method for treating, diagnosing, or preventing a disease in a subject, comprising administering to the subject the pharmaceutical composition of the present disclosure or the nanoparticle composition of the present disclosure.

In a more specific embodiment, the present disclosure further provides the compound, or the isotopic variant, tautomer, or stereoisomer thereof, or the pharmaceutically acceptable salt thereof of the present disclosure, or the pharmaceutical composition of the present disclosure, or the nanoparticle composition of the present disclosure for use in treating, diagnosing, or preventing a disease.

In a more specific embodiment, the present disclosure further provides a method for delivering a load into a subject's body, comprising administering to the subject the pharmaceutical composition of the present disclosure or the nanoparticle composition of the present disclosure,
wherein the load is selected from one or more of a therapeutic agent, a prophylactic agent, and a diagnostic agent.

In a more specific embodiment, the present disclosure further provides the compound, or the isotopic variant, tautomer, or stereoisomer thereof, or the pharmaceutically acceptable salt thereof of the present disclosure, or the pharmaceutical composition of the present disclosure, or the nanoparticle composition of the present disclosure for use in delivering a load,
wherein the load is selected from one or more of a therapeutic agent, a prophylactic agent, and a diagnostic agent.

In a more specific embodiment, provided are the nanoparticle composition of the present disclosure, the use of the present disclosure, or the method of the present disclosure, wherein the therapeutic agent, the prophylactic agent, or the diagnostic agent is a nucleic acid;
preferably, the nucleic acid is selected from one or more of ASO, RNA, and DNA;
preferably, the RNA is selected from one or more of a small interfering RNA (siRNA), a short hairpin RNA (shRNA), an antisense RNA (aRNA), a messenger RNA (mRNA), a long non-coding RNA (lncRNA), a micro RNA (miRNA), a small activating RNA (saRNA), a multimeric coding nucleic acid (MCNA), a polymeric coding nucleic acid (PCNA), a guide RNA (gRNA), a CRISPR RNA (crRNA), and a ribozyme, preferably an mRNA, and more preferably a modified mRNA.

In a more specific embodiment, the present disclosure further provides a method for preparing a compound of formula (II), which comprises:
reacting a compound of formula (IIA) with a compound of formula (IIB) to give the compound of formula (II), 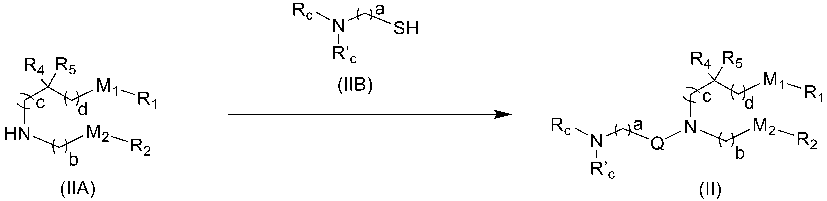
wherein the variables are as defined in the present disclosure.

In a more specific embodiment, the present disclosure further provides a method for preparing a compound of formula (III), which comprises:
reacting a compound of formula (IIIA) with a compound of formula (IIIB) to give the compound of formula (III); 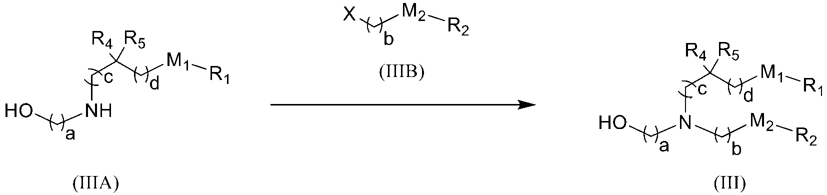
or reacting a compound of formula (IIIC) with a compound of formula (IIID) to give the compound of formula (II), 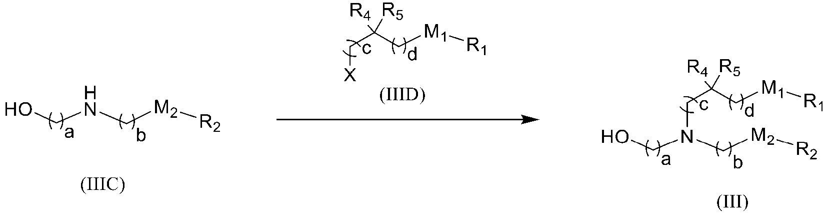
wherein X is halogen, and the other variables are as defined in the present disclosure.

The compounds of the present disclosure may comprise one or more asymmetric centers and may therefore exist in various stereoisomeric forms, e.g., enantiomeric and/or diastereomeric forms. For example, the compounds of the present disclosure may be individual enantiomers, diastereomers, or geometric isomers (e.g., cis and trans isomers), or may be in the form of a mixture of stereoisomers, including racemic mixtures and mixtures enriched in one or more stereoisomers. Isomers may be separated from the mixture by methods known to those skilled in the art, including chiral high performance liquid chromatography (HPLC) and the formation and crystallization of chiral salts; or preferred isomers may be prepared by asymmetric synthesis. The compounds of the present disclosure may exist in the form of tautomers. The tautomer is a functional group isomer resulting from the rapid movement of an atom in a molecule between two positions. The tautomer is a special functional group isomer. A pair of tautomers can be interconverted, but usually one isomer that is relatively stable is the main existing form thereof. The most prominent examples are enol and keto tautomers. The present disclosure further comprises isotopically labeled compounds (isotopic variants), which are equivalent to those described in formula (IV), except that one or more atoms are replaced by an atom having an atomic mass or mass number different from the atomic mass or mass number usually found in nature. Examples of isotopes that can be incorporated into the compounds of the present disclosure include isotopes of hydrogen, carbon, nitrogen, oxygen, phosphorus, sulfur, fluorine, and chlorine, such as ²H, ³H, ¹³C, ¹¹C, ¹⁴C, ¹⁵N, ¹⁸O, ¹⁷O, ³¹P, ³²P, ³⁵S, ¹⁸F, and ³⁶Cl, respectively. The compounds of the present disclosure containing the above isotopes and/or other isotopes of other atoms, prodrugs thereof, and pharmaceutically acceptable salts of the compounds or prodrugs are all within the scope of the present disclosure. Certain isotopically labeled compounds of the present disclosure, such as those into which radioisotopes (e.g., ³H and ¹⁴C) are incorporated, are useful in drug and/or substrate tissue distribution assays. Tritium (i.e., ³H) and carbon-14 (i.e., ¹⁴C) isotopes are particularly preferred for their ease of preparation and detectability. Furthermore, substitution with heavier isotopes such as deuterium (i.e., ²H) may afford therapeutic benefits (e.g., increased *in vivo* half-life or reduced dose) resulting from greater metabolic stability and hence may be preferred in some circumstances. Isotopically labeled compounds of formula (IV) of the present disclosure and prodrugs thereof can generally be prepared by using readily available isotopically labeled reagents to replace non-isotopically labeled reagents in the following procedures and/or the processes disclosed in the examples and preparation examples.

The present disclosure further provides a pharmaceutical formulation comprising a therapeutically effective amount of the compound of formula (IV) or a therapeutically acceptable salt thereof and a pharmaceutically acceptable carrier, diluent, or excipient. All of these forms are within the present disclosure.

### Pharmaceutical composition and kit

In another aspect, the present disclosure provides a pharmaceutical composition comprising the nanoparticle composition of the present disclosure and a pharmaceutically acceptable excipient, wherein the nanoparticle composition comprises the compound of the present disclosure.

A pharmaceutically acceptable excipient for use in the present disclosure refers to a non-toxic carrier, adjuvant, or vehicle that does not destroy the pharmacological activity of the compound being formulated together. Pharmaceutically acceptable carriers, adjuvants, or vehicles that may be used in the composition of the present disclosure include, but are not limited to, ion exchangers, aluminium oxide, aluminium stearate, lecithin, serum proteins (e.g., human serum albumin), buffer substances (e.g., phosphates), glycine, sorbic acid, potassium sorbate, partial glyceride mixtures of saturated vegetable fatty acids, water, salts or electrolytes (e.g., protamine sulfate), disodium hydrogen phosphate, potassium hydrogen phosphate, sodium chloride, zinc salts, silica gel, magnesium trisilicate, polyvinylpyrrolidone, cellulose-based substances, polyethylene glycol, sodium carboxymethylcellulose, polyacrylates, waxes, polyethylene-polyoxypropylene-block polymers, polyethylene glycol, and lanolin.

The present disclosure further comprises a kit (e.g., a pharmaceutical package). The provided kit may comprise the nanoparticle composition of the present disclosure and an additional therapeutic, diagnostic, or prophylactic agent, and first and second containers (e.g., vials, ampoules, bottles, syringes, and/or dispersible packages or other suitable containers) containing the nanoparticle composition of the present disclosure and the additional therapeutic, diagnostic, or prophylactic agent. In some embodiments, the provided kit may further optionally comprise a third container containing a pharmaceutical excipient for diluting or suspending the nanoparticle composition of the present disclosure and/or the additional therapeutic, diagnostic, or prophylactic agent. In some embodiments, the nanoparticle composition of the present disclosure and the additional therapeutic, diagnostic, or prophylactic agent provided in the first container and the second container are combined to form a unit dosage form.

### Administration

The pharmaceutical composition provided by the present disclosure may be given via many routes, including but not limited to: oral administration, parenteral administration, inhalation administration, topical administration, rectal administration, nasal administration, buccal administration, vaginal administration, administration via an implant, or other routes of administration. For example, the parenteral administration as used herein includes subcutaneous administration, intradermal administration, intravenous administration, intramuscular administration, intra-articular administration, intra-arterial administration, intrasynovial administration, intrasternal administration, intracerebrospinal administration, intralesional administration, and intracranial injection or infusion techniques.

Generally, an effective amount of the pharmaceutical composition of the present disclosure is given. The amount of the pharmaceutical composition actually given can be determined by a physician in the light of the relevant circumstances, including the condition to be treated or prevented, the selected route of administration, the pharmaceutical composition actually given, the age, weight, and response of an individual patient, the severity of symptoms of a patient, and the like.

When used to prevent the condition of the present disclosure, the pharmaceutical composition provided herein is given to a subject at risk of developing the condition, typically on the basis of the advice and under the supervision of a physician at dose levels as described above. Subjects at risk of developing a particular condition generally include subjects with a family history of the condition, or those determined to be particularly susceptible to developing the condition by genetic testing or screening.

The pharmaceutical composition provided herein may also be given for a long time ("long-term administration"). The long-term administration refers to administration of a compound or a pharmaceutical composition thereof over a long period of time, e.g., 3 months, 6 months, 1 year, 2 years, 3 years, 5 years, etc., or means that the administration may be continued indefinitely, e.g., for the remainder of the subject's life. In some embodiments, the long-term administration is intended to provide a constant level of the compound in the blood over a long period of time, e.g., within a therapeutic window.

The pharmaceutical composition of the present disclosure may be further delivered using a variety of administration methods. For example, in some embodiments, the pharmaceutical composition may be given by bolus injection, e.g., to increase the concentration of the compound in the blood to an effective level. Bolus doses depend on a target systemic level of an active component throughout the body, for example, intramuscular or subcutaneous bolus doses allow a slow release of the active component, while a bolus delivered directly to the vein (e.g., by intravenous (IV) drip) can be delivered more rapidly, allowing the concentration of the active component in the blood to increase rapidly to an effective level. In other embodiments, the pharmaceutical composition may be given in the form of continuous infusion, e.g., by intravenous (IV) drip, so as to provide a steady-state concentration of the active component in the body of the subject. In addition, in other embodiments, a bolus dose of the pharmaceutical composition may be given first, followed by continuous infusion.

In order to provide blood levels similar to or lower than those using injection doses, transdermal doses are generally selected in amounts of from about 0.01 to about 20% by weight, preferably from about 0.1 to about 20% by weight, preferably from about 0.1 to about 10% by weight, and more preferably from about 0.5 to about 15% by weight.

From about 1 to about 120 hours, in particular 24 to 96 hours, injection dose levels range from about 0.1 mg/kg/h to at least 10 mg/kg/h. In order to achieve an adequate steady-state level, a preloaded bolus at about 0.1 mg/kg to about 10 mg/kg or more may also be given. For a human patient of 40 to 80 kg, the maximum total dose cannot exceed about 2 g/day.

Injectable compositions are typically based on injectable sterile saline or phosphate buffered saline, or other injectable excipients known in the art. As mentioned before, in such compositions, the active compound is typically a minor component, with often about 0.05 to 10% by weight, the remainder being injectable excipients and the like.

### Examples

In order to make the technical solutions of the present disclosure more apparent and explicit, the present disclosure is further elaborated through the following examples. The following examples are only intended to illustrate specific embodiments of the present disclosure for a better understanding by those skilled in the art, rather than limit the scope of protection of the present disclosure. In specific embodiments of the present disclosure, technical means or methods that are not specified are those conventional in the art. Materials, reagents and the like used in the examples are commercially available unless otherwise indicated.

**Table 2**

| **English or abbreviation** | **Full name** |
|---|---|
| THF | Tetrahydrofuran |
| DCM | Dichloromethane |
| MeOH | Methanol |
| EtOH | Ethanol |
| IPA | Isopropanol |
| DMF | *N,N*-Dimethylformamide |
| DMSO | Dimethyl sulfoxide |
| ACN | Acetonitrile |
| CPME | Cyclopentyl methyl ether |
| CDCl₃ | Deuterated chloroform |
| CD₃OD | Deuterated methanol |
| DMSO-*d₆* | Deuterated dimethyl sulfoxide |
| DMAP | 4-Dimethylaminopyridine |
| BTC | Triphosgene |
| NaOH | Sodium hydroxide |
| NaH | Sodium hydride |
| BH₃-THF | Borane-tetrahydrofuran |
| NaBH₄ | Sodium borohydride |
| KI | Potassium iodide |
| NaI | Sodium iodide |
| K₂CO₃ | Potassium carbonate |
| NH₄HCO₃ | Ammonium bicarbonate |
| EDCI | 1-(3-Dimethylaminopropyl)-3-ethylcarbodiimide |
| thiourea | Thiourea |
| DIPEA | *N,N-Diisopropylethylamine* |
| Et₃N | Triethylamine |
| CBr₄ | Tetrabromomethane |
| PPh₃ | Triphenylphosphine |

### Example 1: Synthesis of Compound 1

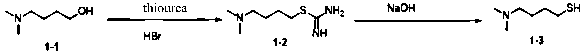

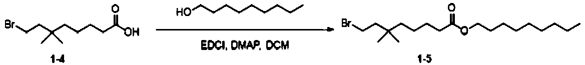

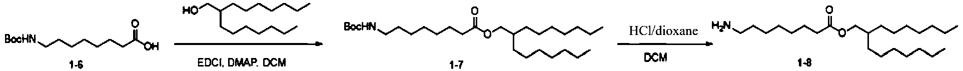

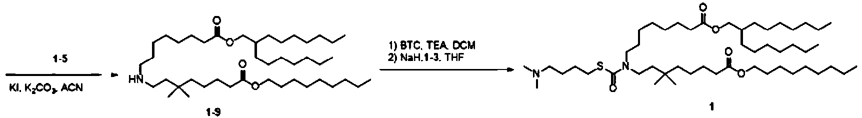

In a 100 mL sealed tube, 4-dimethylamino-1-butanol (3.0 g, 25.5 mmol, 1.0 eq.) and thiourea (8.4 g, 110.0 mmol, 4.3 eq.) were added, and then an aqueous HBr solution (48%, 60 mL) was added. The mixture was heated to 120 °C and stirred for reaction overnight, which was directly used in the next step without purification. The system described above was cooled to 0 °C, and NaOH (10.2 g, 255.0 mmol, 10.0 eq.) was added in batches. The mixture was heated to 120 °C and reacted for 2 h. The mixture was cooled to room temperature and extracted with dichloromethane. The organic phases were combined, dried over anhydrous sodium sulfate, and filtered to remove the drying agent, and the solvent was removed to give compound 1-3 (2.45 g) as a colorless oil.

In a 100 mL three-necked flask, compound 1-4 (1.26 g, 5.0 mmol, 1.0 eq.), 1-nonanol (0.72 g, 5.0 mmol, 1.0 eq.), EDCI (1.44 g, 7.5 mmol, 1.5 eq.), and DMAP (0.31 g, 2.5 mmol, 0.5 eq.) were dissolved in dichloromethane (20 mL). The mixture was stirred at room temperature overnight. The mixture was extracted with dichloromethane and washed with water (3 × 20 mL). The organic phases were combined, concentrated by rotary evaporation under reduced pressure, directly mixed with silica gel, and purified by silica gel column chromatography to give compound 1-5 (1.6 g).

In a 100 mL three-necked flask, compound 1-6 (1.5 g, 5.8 mmol, 1.0 eq.), 2-heptyl-1-nonanol (1.40 g, 5.8 mmol, 1.0 eq.), EDCI (1.66 g, 8.7 mmol, 1.5 eq.), and DMAP (0.35 g, 2.9 mmol, 0.5 eq.) were dissolved in dichloromethane (20 mL). The mixture was stirred at room temperature overnight. The mixture was washed with water (3 × 15 mL). The organic phase was concentrated by rotary evaporation under reduced pressure, directly mixed with silica gel, and purified by silica gel column chromatography to give compound 1-7 (3.3 g).

In a 100 mL three-necked flask, compound 1-7 (2.8 g, 5.8 mmol, 1.0 eq.) was dissolved in dichloromethane (20 mL), and HCl/dioxane (4 M, 14 mL) was added. The mixture was stirred at room temperature overnight. The organic solvent was removed by rotary evaporation, the reaction liquid was adjusted to neutrality with a saturated sodium bicarbonate solution, and the mixture was extracted with DCM (3 × 20 mL). The organic phase was collected, dried over anhydrous Na₂SO₄, and purified by silica gel column chromatography to give compound 1-8 (1.95 g).

In a 40 mL sealed tube, compound 1-8 (680 mg, 1.8 mmol, 1.0 eq.), KI (365 mg, 2.2 mmol, 1.2 eq.), K₂CO₃ (745 mg, 5.4 mmol, 3.0 eq.), and compound 1-5 (668.9 mg, 1.8 mmol, 1.0 eq.) were dissolved in acetonitrile (10 mL). The mixture was heated to 80 °C and stirred for reaction overnight. After the reaction was completed, the mixture was cooled to room temperature and filtered, and the filter cake was washed with acetonitrile (2 × 5 mL). The filtrate was collected and concentrated by rotary evaporation to dryness. The residue was purified by column chromatography to give compound 1-9 (580 mg) as a yellow oil.

In a 20 mL sealed tube, compound 1-9 (200 mg, 0.29 mmol, 1.0 eq.) was dissolved in DCM (5 mL). After the reaction system was cooled to 5 °C, TEA (59.5 mg, 0.59 mmol, 2.0 eq.) and BTC(87.3 mg, 0.29 mmol, 1.0 eq.) were added dropwise. The temperature was kept at 5 °C, the mixture was stirred for reaction for 1 h and concentrated to remove DCM, and tetrahydrofuran (5 mL) was added. In another 20 mL sealed tube, compound 1-3 (101.9 mg, 0.76 mmol, 2.6 eq.) was dissolved in tetrahydrofuran (5 mL). After the mixture was cooled to 0 °C, NaH (60%, 58.0 mg, 1.45 mmol, 5.0 eq.) was added. After stirring at 0 °C for 1 h, the above prepared THF mixed solution was added dropwise, and the mixture was reacted for another 1 h. The reaction liquid was poured into ice water (10 mL), and the mixture was extracted with ethyl acetate. The organic phases were combined, dried over anhydrous sodium sulfate, and filtered to remove the drying agent, and the solvent was removed to give a crude product. The crude product was purified by Prep-HPLC (Column: XSelect C18 (30 × 150 mm, 5 µm); Eluent A: H₂O/ACN 60/40, 10 mM NH₄HCO₃ + 1% NH₃•H₂O; Eluent B: IPA/ACN 90/10; Flow rate: 60 mL/min; Gradient program: 65%-85% B in 0-12 min) to give compound 1 (126.9 mg) as a light yellow oil.

**¹H NMR** (300 MHz, CDCl₃) δ: 4.06 (t, *J* = 6.6 Hz, 2H), 3.96 (d, *J* = 5.7 Hz, 2H), 3.36-3.15 (m, 4H), 2.90 (t, *J* = 6.9 Hz, 2H), 2.41 (s, 2H), 2.31-2.24 (m, 10H), 1.64-1.16 (m, 61H), 0.93-0.77 (m, 15H); **ESI-MS** m/z: 839.65 [M+H]⁺.

### Example 2: Synthesis of Compound 2

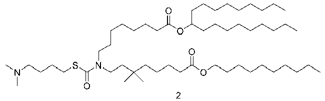

Compound 2 was prepared by referring to the method of Example 1 to give an oily product (157.3 mg).

**¹H NMR** (300 MHz, CDCl₃) δ: 4.86 (p, *J* = 6.3 Hz, 1H), 4.06 (t, *J* = 6.9 Hz, 2H), 3.37-3.17 (m, 4H), 2.90 (t, *J* = 6.9 Hz, 2H), 2.39-2.25 (m, 12H), 1.79-1.18 (m, 70H), 0.93-0.83 (m, 15H); **ESI-MS** m/z: 895.80 [M+H]⁺.

### Example 3: Synthesis of Compound 3

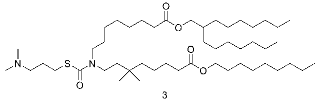

Compound 3 was prepared by referring to the method of Example 1 to give an oily product (184.0 mg).

**¹H NMR** (300 MHz, CDCl₃) δ: 4.06 (t, *J* = 6.9 Hz, 2H), 3.96 (d, *J* = 5.7 Hz, 2H), 3.35-3.18 (m, 4H), 2.91 (t, *J =* 7.2 Hz, 2H), 2.43-2.27 (m, 12H), 1.87-1.77 (m, 2H), 1.61-1.18 (m, 57H), 0.93-0.84 (m, 15H); **ESI-MS** m/z: 825.55 [M+H]⁺.

### Example 4: Synthesis of Compound 4

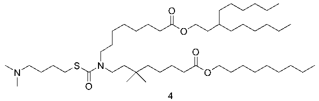

Compound 4 was prepared by referring to the method of Example 1 to give an oily product (103.2 mg).

**¹H NMR** (300 MHz, CDCl₃) δ: 4.07 (q, *J* = 6.9 Hz, 4H), 3.26 (m, 4H), 2.90 (t, *J* = 6.9 Hz, 2H), 2.54-2.42 (m, 5H), 2.30 (q, *J =* 7.8 Hz, 5H), 1.74-1.52 (m, 16H), 1.42-1.22 (m, 45H), 0.93-0.83 (m, 15H); **ESI-MS** m/z: 825.65 [M+H]⁺.

### Example 5: Synthesis of Compound 5

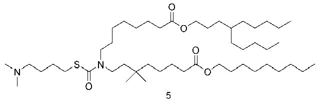

Compound 5 was prepared by referring to the method of Example 1 to give an oily product (82.3 mg).

**¹H NMR** (300 MHz, CDCl₃) δ: 4.05 (td, *J* = 6.9, 5.4 Hz, 4H), 3.26 (m, 4H), 2.97 -2.85 (m, 2H), 2.40-2.30 (m, 10H), 1.60 (m, 14H), 1.34-1.21 (m, 45H), 0.94-0.83 (m, 15H); **ESI-MS** m/z: 811.70 [M+H]⁺.

### Example 6: Synthesis of Compound 6

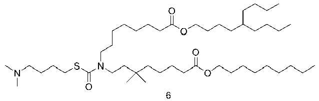

Compound 6 was prepared by referring to the method of Example 1 to give an oily product (42.7 mg).

**¹H NMR** (300 MHz, CDCl₃) δ: 4.06 (t, *J =* 6.9 Hz, 4H), 3.26 (m, 4H), 2.90 (t, *J =* 7.2 Hz, 2H), 2.62 (m, 6H), 2.30 (q, *J* = 7.0 Hz, 4H), 1.59 (m, 14H), 1.42-1.15 (m, 43H), 0.94-0.84 (m, 15H); **ESI-MS** m/z: 797.65 [M+H]⁺.

### Example 7: Synthesis of Compound 7

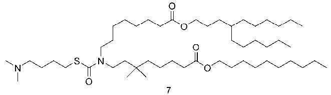

Compound 7 was prepared by referring to the method of Example 1 to give an oily product (145.3 mg).

**¹H NMR** (300 MHz, CDCl₃) δ: 4.05 (q, *J* = 6.6 Hz, 4H), 3.26 (m, 4H), 2.95-2.85 (m, 2H), 2.42-2.26 (m, 10H), 1.64-1.59 (m, 14H), 1.33-1.10 (m, 51H), 0.93-0.83 (m, 15H); **ESI-MS** m/z: 853.70 [M+H]⁺.

### Example 8: Synthesis of Compound 8

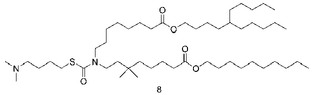

Compound 8 was prepared by referring to the method of Example 1 to give an oily product (125.4 mg).

**¹H NMR** (300 MHz, CDCl₃) δ: 4.06 (t, *J* = 6.6 Hz, 4H), 3.26 (m, 4H), 2.90 (t, *J* = 6.6 Hz, 2H), 2.47-2.27 (m, 10H), 1.68-1.60 (m, 16H), 1.39-1.27 (m, 47H), 0.94-0.83 (m, 15H); **ESI-MS** m/z: 840.10 [M+H]⁺.

### Example 9: Synthesis of Compound 9

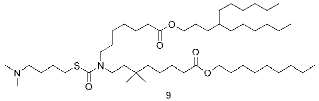

Compound 9 was prepared by referring to the method of Example 1 to give an oily product (120.4 mg).

**¹H NMR** (300 MHz, CD₃OD) δ: 4.06 (td, *J =* 6.6, 3.6 Hz, 4H), 3.31 (p, *J =* 1.5 Hz, 2H), 2.96-2.85 (m, 2H), 2.45-2.18 (m, 12H), 1.65-1.58 (m, 14H), 1.34-1.17 (m, 47H), 0.95-0.85 (m, 15H); **ESI-MS** m/z: 825.70 [M+H]⁺.

### Example 10: Synthesis of Compound 10

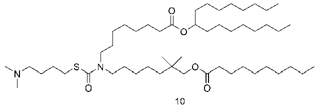

Compound 10 was prepared by referring to the method of Example 1 to give an oily product (228 mg).

**¹H NMR** (300 MHz, CDCl₃) δ: 4.86 (p, *J =* 6.3 Hz, 1H), 3.78 (s, 2H), 3.33-3.18 (m, 4H), 2.90 (t, *J=* 6.6 Hz, 2H), 2.38-2.25 (m, 12H), 1.69-1.44 (m, 16H), 1.34-1.17 (m, 48H), 0.90-0.85 (m, 15H); **ESI-MS** m/z: 853.65 [M+H]⁺.

### Example 11: Synthesis of Compound 11

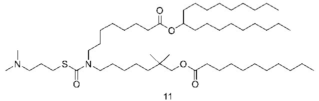

Compound 11 was prepared by referring to the method of Example 1 to give an oily product (117.9 mg).

**¹H NMR** (300 MHz, CDCl₃) δ: 4.86 (p, *J =* 6.3 Hz, 1H), 3.78 (s, 2H), 3.36-3.17 (m, 4H), 2.92 (t, *J* = 7.2 Hz, 2H), 2.46 (t, *J* = 7.2 Hz, 2H), 2.34-2.22 (m, 10H), 1.90-1.80 (m, 3H), 1.62-1.49 (m, 14H), 1.34-1.17 (m, 51H), 0.93-0.86 (m, 15H); **ESI-MS** m/z: 881.65 [M+H]⁺.

### Example 12: Synthesis of Compound 12

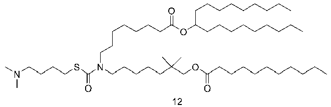

Compound 12 was prepared by referring to the method of Example 1 to give an oily product (75.3 mg).

**¹H NMR** (300 MHz, CDCl₃) δ: 4.86 (p, *J =* 6.3 Hz, 1H), 3.78 (s, 2H), 3.36-3.17 (m, 4H), 2.91 (t, *J* = 6.6 Hz, 2H), 2.48-2.28 (m, 12H), 1.89-1.60 (m, 10H), 1.51-1.16 (m, 60H), 0.93-0.87 (m, 15H); **ESI-MS** m/z: 895.70 [M+H]⁺.

### Example 13: Synthesis of Compound 13

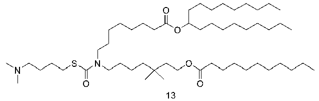

Compound 13 was prepared by referring to the method of Example 1 to give an oily product (95.1 mg).

**¹H NMR** (400 MHz, CDCl₃) δ: 4.86 (p, *J* = 6.4 Hz, 1H), 4.09 (t, *J =* 7.6 Hz, 2H), 3.31-3.24 (m, 4H), 2.90 (t, *J* = 7.6 Hz, 2H), 2.40-2.26 (m, 12H), 1.63-1.50 (m, 21H), 1.32-1.16 (m, 49H), 0.90-0.86 (m, 15H); **ESI-MS** m/z: 895.80 [M+H]⁺.

### Example 14: Synthesis of Compound 14

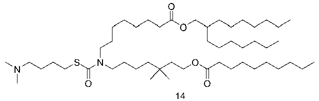

Compound 14 was prepared by referring to the method of Example 1 to give an oily product (63.6 mg).

**¹H NMR** (400 MHz, CDCl₃) δ: 4.09 (p, *J =* 7.6 Hz, 2H), 3.96 (d, *J* = 6.0 Hz, 2H), 3.31-3.24 (m, 4H), 2.90 (t, *J* = 7.2 Hz, 2H), 2.57-2.26 (m, 12H), 1.67-1.43 (m, 20H), 1.32-1.16 (m, 41H), 0.90-0.86 (m, 15H); **ESI-MS** m/z: 839.70 [M+H]⁺.

### Example 15: Synthesis of Compound 15

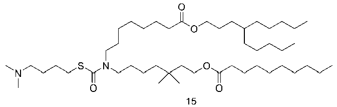

Compound 15 was prepared by referring to the method of Example 1 to give an oily product (70.5 mg).

**¹H NMR** (300 MHz, CD₃OD) δ: 4.12 (t, *J* = 7.2 Hz, 2H), 4.05 (t, *J* = 6.6 Hz, 2H), 3.35 (s, 2H), 2.90 (t, *J* = 6.6 Hz, 2H), 2.40-2.26 (m, 12H), 1.62-1.56 (m, 16H), 1.39-1.21 (m, 43H), 0.98-0.84 (m, 15H); **ESI-MS** m/z: 811.80 [M+H]⁺.

### Example 16: Synthesis of Compound 16

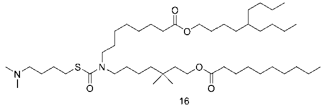

Compound 16 was prepared by referring to the method of Example 1 to give an oily product (46.4 mg).

**¹H NMR** (400 MHz, CD₃OD) δ: 4.12 (t, *J* = 7.2 Hz, 2H), 4.07 (t, *J* = 6.4 Hz, 2H), 3.35 (m, 2H), 2.92-2.83 (m, 2H), 2.39-2.26 (m, 12H), 1.64-1.51 (m, 16H), 1.44-1.21 (m, 41H), 0.95-0.86 (m, 15H); **ESI-MS** m/z: 797.65 [M+H]⁺.

### Example 17: Synthesis of Compound 17

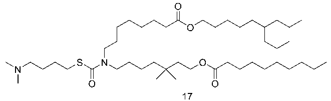

Compound 17 was prepared by referring to the method of Example 1 to give an oily product (34.8 mg).

**¹H NMR** (300 MHz, CDCl₃) δ: 4.12-4.03 (m, 4H), 3.31 (m, 4H), 3.06-2.78 (m, 10H), 2.32-2.25 (m, 4H), 2.00-1.95 (m, 2H), 1.62-1.56 (m, 14H), 1.44-1.21 (m, 37H), 0.94-0.85 (m, 15H); **ESI-MS** m/z: 783.60 [M+H]⁺.

### Example 18: Synthesis of Compound 18

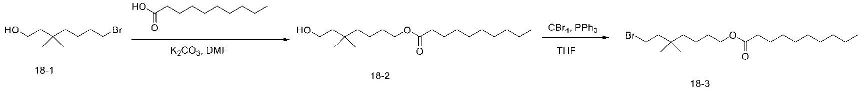

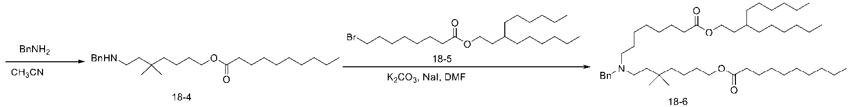

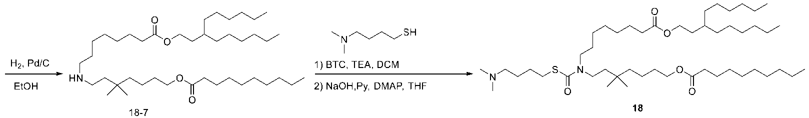

18-1 (5.0 g, 22.40 mmol, 1.0 eq.) and decanoic acid (5.0 g, 29.12 mmol, 1.3 eq.) were added to a DMF solution (50 mL), and potassium carbonate (9.3 g, 67.21 mmol, 3.0 eq.) was added under nitrogen atmosphere. The mixture was stirred at 70 °C for 2 h. After the reaction was completed, the reaction liquid was cooled to room temperature, diluted with water (100 mL), and then extracted with dichloromethane (3 × 300 mL). The organic phases were combined, washed with a saturated aqueous sodium chloride solution (3 × 300 mL), and dried over anhydrous sodium sulfate. The organic phase was collected by filtration, and the organic solvent was removed by using a rotary evaporator to give a crude product. The crude product was separated and purified by silica gel column chromatography to give compound 18-2 (7.7 g) as a yellow oil.

18-2 (7.7 g, 24.48 mmol, 1.0 eq.) was dissolved in tetrahydrofuran (80 mL), and triphenylphosphine (25.6 g, 97.93 mmol, 4.0 eq.) and tetrabromomethane (32.4 g, 97.93 mmol, 4.0 eq.) were added under nitrogen atmosphere. The mixture was stirred at room temperature for 5 h. The mixture was filtered, and the filtrate was diluted with water (100 mL) and then extracted with ethyl acetate (3 × 300 mL). The organic phases were combined, washed with a saturated aqueous sodium chloride solution (3 × 300 mL), and dried over anhydrous sodium sulfate. The organic phase was collected by filtration, and the organic solvent was removed by using a rotary evaporator to give a crude product. The crude product was purified by silica gel column chromatography to give compound 18-3 (8.9 g) as a light yellow oil.

18-3 (4.0 g, 10.59 mmol, 1.0 eq.) was dissolved in acetonitrile (40 mL), and benzylamine (2.2 g, 21.19 mmol, 2.0 eq.) was added in an ice bath. The mixture was reacted at 50 °C for 16 h under nitrogen atmosphere. After the reaction was completed, the reaction liquid was cooled to room temperature, diluted with water (50 mL), and then extracted with dichloromethane (3 × 100 mL). The organic phases were combined, washed with a saturated aqueous sodium chloride solution (3 × 100 mL), and dried over anhydrous sodium sulfate. The organic phase was collected by filtration, and the organic solvent was removed by using a rotary evaporator to give a crude product. The crude product was purified by silica gel column chromatography to give compound 18-4 (3.1 g) as a colorless oil.

18-4 (1.0 g, 2.47 mmol, 1.0 eq.) was dissolved in DMF (10.0 mL), and 18-5 (1.2 g, 2.97 mmol, 1.2 eq.), potassium carbonate (1.0 g, 7.43 mmol, 3.0 eq.), and sodium iodide (0.9 g, 6.19 mmol, 2.5 eq.) were added sequentially under nitrogen atmosphere. The mixture was stirred at 70 °C for 5 h. After the reaction was completed, the reaction liquid was cooled to room temperature, diluted with water (10 mL), and then extracted with dichloromethane (3 × 50 mL). The organic phases were combined, washed with a saturated aqueous sodium chloride solution (3 × 50 mL), and dried over anhydrous sodium sulfate. The organic phase was collected by filtration, and the organic solvent was removed by using a rotary evaporator to give a crude product. The crude product was purified by silica gel column chromatography to give compound 18-6 (1.5 g) as a yellow oil.

18-6 (1.5 g, 2.06 mmol, 1.0 eq.) was dissolved in ethanol (15 mL), and palladium on carbon (700.0 mg) was added at room temperature under hydrogen atmosphere. The mixture was reacted at room temperature overnight under hydrogen atmosphere and filtered under vacuum to remove palladium on carbon. The filter cake was washed with dichloromethane, and the filtrate was concentrated under reduced pressure to give a crude product, which was directly used in the next step without purification to give compound 18-7 (1.1 g) as a colorless oil.

18-7 (260 mg, 0.39 mmol, 1.0 eq.) was dissolved in dichloromethane (2 mL), and triphosgene (81.07 mg, 0.27 mmol, 0.7 eq.) and triethylamine (118.49 mg, 1.17 mmol, 3.0 eq.) were added in an ice bath. The mixture was stirred at room temperature for 1 h and concentrated under reduced pressure to remove the solvent. The above concentrated solution, 4-dimethylamino-1-butanethiol (259.4 mg, 1.93 mmol, 5.0 eq.), was dissolved in tetrahydrofuran (5 mL). Sodium hydroxide (124.89 mg, 3.12 mmol, 8.0 eq.), pyridine (92.62 mg, 1.17 mmol, 3.0 eq.), and 4-dimethylaminopyridine (95.37 mg, 0.78 mmol, 2.0 eq.) were added sequentially in an ice bath. The mixture was stirred at room temperature for 3 h. After the reaction was completed, the reaction liquid was diluted with water (10 mL) and then extracted with ethyl acetate (3 × 50 mL). The organic phases were combined, washed with a saturated aqueous sodium chloride solution (3 × 50 mL), and dried over anhydrous sodium sulfate. The organic phase was collected by filtration, and the organic solvent was removed by using a rotary evaporator to give a crude product. The crude product was purified by high performance liquid chromatography (column: YMC-Actus Triart C8 20 × 150 mm, 5 µm; phase A: acetonitrile/water (10 mmol/L ammonium bicarbonate + 0.05% ammonia water), phase B: isopropanol/acetonitrile; flow rate: 20 mL/min; gradient: 75% B to 95% B, 9 min) to give compound 18 (71.4 mg) as a yellow oil.

**¹H NMR** (400 MHz, CD₃OD) δ: 0.86-0.95 (m, 15H), 1.26-1.43 (m, 45H), 1.59-1.61 (m, 14H), 2.26 (s, 6H), 2.29-2.39 (m, 6H), 2.90 (t, *J* = 6.8 Hz, 2H), 3.29-3.32 (m, 4H), 4.07-4.12 (m, 4H); **ESI-MS** m/z: 825.70 [M+H]⁺.

### Example 19: Synthesis of Compound 19

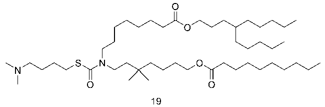

Compound 19 was prepared by referring to the method of Example 18 to give an oily product (72.0 mg).

**¹H NMR** (300 MHz, CD₃OD) δ: 0.90-0.92 (m, 15H), 1.28-1.34 (m, 41H), 1.43-1.61 (m, 16H), 2.25 (s, 6H), 2.29-2.39 (m, 6H), 2.90 (t, *J* = 6.6 Hz, 2H), 3.29-3.31 (m, 4H), 4.03-4.11 (m, 4H); **ESI-MS** m/z: 811.90

[M+H]⁺.

### Example 20: Synthesis of Compound 20

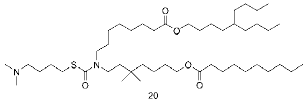

Compound 20 was prepared by referring to the method of Example 18 to give an oily product (74.5 mg).

**¹H NMR** (400 MHz, CD₃OD) δ: 0.88-0.92 (m, 15H), 1.21-1.35 (m, 40H), 1.45-1.62 (m, 15H), 2.27 (s, 6H), 2.29-2.40 (m, 6H), 2.90 (t, *J* = 6.4 Hz, 2H), 3.29-3.31 (m, 4H), 4.08 (q, *J =* 6.8 Hz, 4H); **ESI-MS** m/z: 797.70 [M+H]⁺.

### Example 21: Synthesis of Compound 21

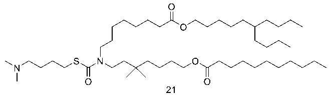

Compound 21 was prepared by referring to the method of Example 18 to give an oily product (77.1 mg).

**¹H NMR** (300 MHz, CDCl₃) δ: 0.82-0.95 (m, 15H), 1.14-1.40 (m, 41H), 1.45-1.85 (m, 18H), 2.26-2.69 (m, 12H), 2.90 (t, *J* = 6.9 Hz, 2H), 3.19-3.37 (m, 4H), 4.03-4.09 (m, 4H); **ESI-MS** m/z: 825.95 [M+H]⁺.

### Example 22: Synthesis of Compound 22

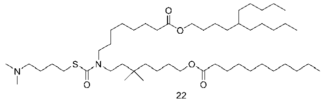

Compound 22 was prepared by referring to the method of Example 18 to give an oily product (98.9 mg).

**¹H NMR** (300 MHz, CD₃OD) δ: 0.85-0.92 (m, 15H), 1.21-1.62 (m, 61H), 2.25 (s, 6H), 2.29-2.38 (m, 6H), 2.90 (t, *J* = 6.9 Hz, 2H), 3.29-3.32 (m, 4H), 4.07 (dd, *J* = 6.3 Hz, 11.7 Hz, 4H); **ESI-MS** m/z: 839.95 [M+H]⁺.

### Example 23: Synthesis of Compound 23

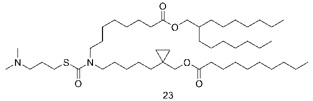

Compound 23 was prepared by referring to the method of Example 18 to give an oily product (130.5 mg). **¹H NMR** (300 MHz, CDCl₃) δ: 3.96 (d, *J* = 6.0 Hz, 2H), 3.89 (s, 2H), 3.35-3.18 (m, 4H), 2.91 (t, *J* = 7.2 Hz, 2H), 2.44-2.28 (m, 12H), 1.87-1.79 (m, 2H), 1.77-1.60 (m, 10H), 1.34-1.17 (m, 47H), 0.90-0.85 (m, 9H), 0.52-0.32 (m, 4H); **ESI-MS** m/z: 823.60 [M+H]⁺.

### Example 24: Synthesis of Compound 24

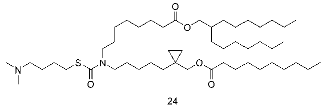

Compound 24 was prepared by referring to the method of Example 18 to give an oily product (79.4 mg).

**¹H NMR** (300 MHz, CDCl₃) δ: 3.95 (d, *J* = 5.7 Hz, 2H), 3.90 (s, 2H), 3.35-3.19 (m, 4H), 2.90 (t, *J* = 7.2 Hz, 2H), 2.67 (s, 4H), 2.34-2.27 (m, 4H), 1.72-1.51 (m, 16H), 1.29-1.21 (m, 49H), 0.90-0.83 (m, 9H), 0.46-0.36 (m, 4H); ESI-MS m/z: 837.75 [M+H]⁺.

### Example 25: Synthesis of Compound 25

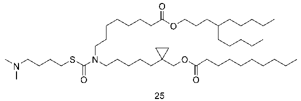

Compound 25 was prepared by referring to the method of Example 18 to give an oily product (67.0 mg).

**¹H NMR** (300 MHz, CD₃OD) δ: 4.05 (t, *J* = 6.9 Hz, 2H), 3.92 (s, 2H), 3.32 (s, 2H), 2.92-2.88 (m, 2H), 2.41-2.25 (m, 12H), 1.71-1.48 (m, 14H), 1.34-1.25 (m, 45H), 0.98-0.83 (m, 9H), 0.47 (m, 2H), 0.39 (m, 2H); **ESI-**MS m/z: 809.75 [M+H]⁺.

### Example 26: Synthesis of Compound 26

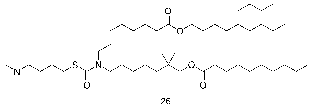

Compound 26 was prepared by referring to the method of Example 18 to give an oily product (33.5 mg).

**¹H NMR** (400 MHz, CD₃OD) δ: 4.07 (t, *J* = 6.4 Hz, 2H), 3.92 (s, 2H), 3.34 (s, 2H), 2.90 (t, *J* = 6.8 Hz, 2H), 2.40-2.32 (m, 6H), 2.27 (s, 6H), 1.60 (m, 16H), 1.40-1.18 (m, 41H), 0.93-0.88 (m, 9H), 0.48-0.46 (m, 2H), 0.43-0.39 (m, 2H); **ESI-MS** m/z: 795.70 [M+H]⁺.

### Example 27: Synthesis of Compound 27

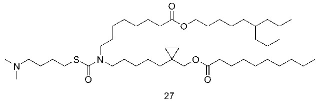

Compound 27 was prepared by referring to the method of Example 18 to give an oily product (32.0 mg).

**¹H NMR** (300 MHz, CDCl₃) δ: 4.05 (t, *J* = 6.6 Hz, 2H), 3.90 (s, 2H), 3.24 (s, 4H), 2.96-2.75 (m, 8H), 2.30 (q, *J* = 7.2 Hz, 4H), 1.74-1.62 (m, 18H), 1.32-1.18 (s, 37H), 0.93-0.82 (m, 9H), 0.46 (m, 2H), 0.36 (m, 2H); **ESI-MS** m/z: 781.65 [M+H]⁺.

### Example 28: Synthesis of Compound 28

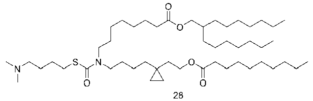

Compound 28 was prepared by referring to the method of Example 18 to give an oily product (41 mg).

**¹H NMR** (400 MHz, CDCl₃) δ: 4.13 (s, 2H), 3.96 (d, *J* = 6.0 Hz, 2H), 3.31-3.24 (m, 4H), 2.90 (t, *J=* 6.8 Hz, 2H), 2.54-2.26 (m, 10H), 1.67-1.45 (m, 18H), 1.29-1.21 (m, 45H), 0.90-0.82 (m, 9H), 0.32-0.29 (m, 4H); **ESI-MS** m/z: 837.70 [M+H]⁺.

### Example 29: Synthesis of Compound 29

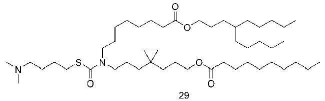

Compound 29 was prepared by referring to the method of Example 18 to give an oily product (68.8 mg).

**¹H NMR** (300 MHz, CD₃OD) δ: 0.26 (m, 4H), 0.86-0.96 (m, 9H), 1.24-1.41 (m, 41H), 1.61-1.74 (m, 16H), 2.27 (s, 6H), 2.29-2.41 (m, 6H), 2.90 (t, *J* = 6.6 Hz, 2H), 3.32-3.41 (m, 4H), 4.03-4.09 (m, 4H); **ESI-MS** m/z: 809.65 [M+H]⁺.

### Example 30: Synthesis of Compound 30

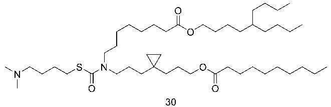

Compound 30 was prepared by referring to the method of Example 18 to give an oily product (86.5 mg).

**¹H NMR** (300 MHz, CD₃OD) δ: 0.21-0.28 (m, 4H), 0.86-0.95 (m, 9H), 1.21-1.45 (m, 39H), 1.61-1.74 (m, 16H), 2.22 (s, 6H), 2.25-2.39 (m, 6H), 2.90 (t, *J =* 6.6 Hz, 2H), 3.30-3.35 (m, 4H), 4.04-4.11 (m, 4H); **ESI-MS** m/z: 795.70 [M+H]⁺.

### Example 31: Synthesis of Compound 31

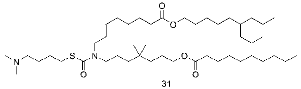

Compound 31 was prepared by referring to the method of Example 18 to give an oily product (86.5 mg).

**¹H NMR** (300 MHz, CD₃OD) δ: 0.27 (m, 4H), 0.81-0.96 (m, 9H), 1.27-1.34 (m, 37H), 1.59-1.74 (m, 16H), 2.25 (s, 6H), 2.28-2.38 (m, 6H), 2.90 (t, *J* = 6.6 Hz, 2H), 3.31-3.35 (m, 4H), 4.06 (t, *J* = 6.6 Hz, 4H); **ESI-MS** m/z: 781.85 [M+H]⁺.

### Example 32: Synthesis of Compound 32

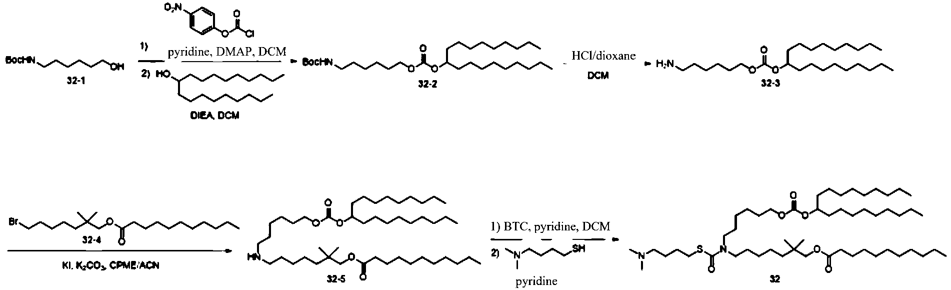

In a 100 mL three-necked round-bottom flask, compound 1 (1.0 g, 4.60 mmol, 1.0 eq.), pyridine (0.74 mL, 9.2 mmol, 2.0 eq.), DMAP (112.4 mg, 0.92 mmol, 0.2 eq.), and p-nitrophenyl chloroformate (1.11 g, 5.52 mmol, 1.2 eq.) were dissolved in dichloromethane (20 mL) at room temperature under nitrogen atmosphere. After the reaction system was stirred at room temperature for 1 h, 2-nonyl-1-decanol (3.93 g, 13.8 mmol, 3.0 eq.) and DIEA (2.40 mL, 13.8 mmol, 3.0 eq.) were added to the reaction system. The reaction system was stirred at room temperature overnight. The reaction was monitored by TLC, and a new spot of the product was found. The mixture was diluted with DCM (20 mL) and washed once with a saturated sodium bicarbonate solution (10 mL). The organic phase was washed 2 times with a saturated NaCl solution (40 mL), dried over anhydrous Na₂SO₄, filtered, and concentrated. The crude product was purified by silica gel column chromatography to give compound 32-2 (1.68 g) as a light yellow oil.

Compound 32 was prepared by referring to the method of Example 1 to give an oily product (78.5 mg).

**¹H NMR** (300 MHz, CDCl₃) δ: 4.68 (p, *J =* 6.0 Hz, 1H), 4.11 (t, *J =* 6.6 Hz, 2H), 3.78 (s, 2H), 3.36-3.22 (m, 4H), 2.90 (t, *J =* 6.9 Hz, 2H), 2.47-2.29 (m, 10H), 1.70-1.56 (m, 18H), 1.29-1.21 (m, 50H), 0.90-0.86 (m, 15H); **ESI-MS** m/z: 897.70 [M+H]⁺.

### Example 33: Synthesis of Compound 33

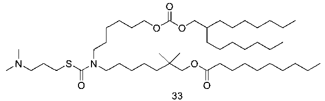

Compound 33 was prepared by referring to the method of Example 32 to give an oily product (189.8 mg). **¹H NMR** (400 MHz, CDCl₃) δ: 4.12 (t, *J =* 6.4 Hz, 2H), 4.02 (d, *J =* 5.6 Hz, 2H), 3.78 (s, 2H), 3.32-3.24 (m, 4H), 2.92 (t, *J =* 7.2 Hz, 2H), 2.47-2.30 (m, 10H), 1.85-1.84 (m, 2H), 1.66-1.40 (m, 7H), 1.26-1.18 (m, 48H), 0.93-0.88 (m, 15H); **ESI-MS** m/z: 827.55 [M+H]⁺.

### Example 34: Synthesis of Compound 34

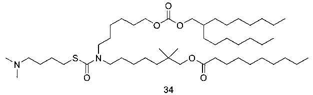

Compound 34 was prepared by referring to the method of Example 32 to give an oily product (129.4 mg). **¹H NMR** (300 MHz, CDCl₃) δ: 4.12 (t, *J* = 6.6 Hz, 2H), 4.02 (d, *J* = 5.7 Hz, 2H), 3.78 (s, 2H), 3.36-3.21 (m, 4H), 2.90 (t, *J =* 6.9 Hz, 2H), 2.36-2.26 (m, 10H), 1.63-1.41 (m, 13H), 1.26-1.18 (m, 46H), 0.89-0.86 (m, 15H); **ESI-MS** m/z: 841.65 [M+H]⁺.

### Example 35: Synthesis of Compound 35

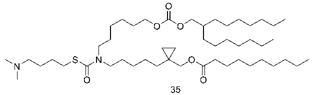

Compound 35 was prepared by referring to the method of Example 32 to give an oily product (135.6 mg). **¹H NMR** (300 MHz, CDCl₃) δ: 4.12 (t, *J* = 6.3 Hz, 2H), 4.02 (d, *J =* 5.7 Hz, 2H), 3.89 (s, 2H), 3.31-3.22 (m, 4H), 2.90 (t*, J* = 6.9 Hz, 2H), 2.34-2.26 (m, 10H), 1.65-1.48 (m, 13H), 1.27-1.19 (m, 46H), 0.90-0.86 (m, 9H), 0.45-0.35 (m, 4H); **ESI-MS** m/z: 839.50 [M+H]⁺.

### Example 36: Synthesis of Compound 36

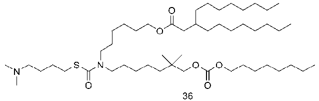

Compound 36 was prepared by referring to the method of Example 32 to give an oily product (123.1 mg). **¹H NMR** (300 MHz, CDCl₃) δ: 4.12 (t, *J =* 6.6 Hz, 2H), 4.02 (t, *J =* 6.6 Hz, 2H), 3.84 (s, 2H), 3.34-3.19 (m, 4H), 2.90 (t, *J* = 6.6 Hz, 2H), 2.46-2.20 (m, 10H), 1.84 (m, 2H), 1.65-1.44 (m, 13H), 1.27-1.19 (m, 46H), 0.92-0.86 (m, 15H); **ESI-MS** m/z: 855.60 [M+H]⁺.

### Example 37: Synthesis of Compound 37

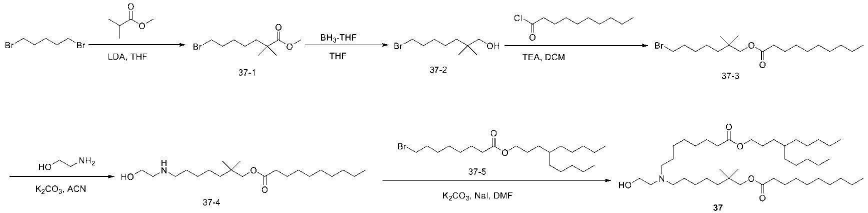

Methyl isobutyrate (21 g, 205.0 mmol, 1.0 eq.) was dissolved in anhydrous THF (200 mL), and the mixture was cooled to 0 °C. LDA (205 mL, 410.0 mmol, 2.0 eq.) was added to the reaction liquid under nitrogen atmosphere. The reaction system was heated to room temperature and stirred for 30 min, and then 1,5-dibromopentane (47 g, 205.0 mmol, 1.0 eq.) was added. After the reaction was completed as monitored by TLC, the reaction was quenched with a saturated aqueous ammonium chloride solution, and the mixture was extracted with DCM (3 × 300 mL). The organic phases were combined and dried over anhydrous sodium sulfate. The mixture was filtered, and the filtrate was concentrated to dryness to give a crude product, which was purified by silica gel column chromatography to give compound 37-1 (30 g) as a yellow oil.

Compound 37-1 (15.0 g, 40.0 mmol, 1.0 eq.) was dissolved in THF (30 mL), and the mixture was cooled to 0 °C. A borane-tetrahydrofuran solution (1 M, 100.0 mL) was added dropwise to the reaction system under nitrogen atmosphere. The mixture was heated to 75 °C and stirred for reaction for 3 h. After the reaction was completed, the mixture was cooled to room temperature. The reaction was quenched with a saturated aqueous ammonium chloride solution, and the mixture was extracted with DCM (3 × 300 mL). The organic phases were combined and dried over anhydrous sodium sulfate. The mixture was filtered, and the filtrate was concentrated to dryness to give a crude product of 37-2 (13.6 g), which was directly used in the next step without purification.

Decanoyl chloride (11.0 g, 58.0 mmol, 1.3 eq.) was added to a solution of compound 37-2 (10.0 g, 44.0 mmol, 1.0 eq.) in DCM (100 mL), and then triethylamine (13.5 g, 134.0 mmol, 3.0 eq.) was added to the reaction system. The mixture was reacted at room temperature for 3 h. The reaction liquid was poured into water (100 mL), and the mixture was extracted with DCM. The organic phases were combined and dried over anhydrous sodium sulfate. The mixture was filtered, and the filtrate was concentrated to dryness to give a crude product, which was purified by silica gel column chromatography to give compound 37-3 (10 g).

Potassium carbonate (1.5 g, 11.1 mmol, 3.0 eq.) and compound 37-3 (1.4 g, 3.7 mmol, 1.0 eq.) were added to a solution of ethanolamine (2.3 g, 37.2 mmol, 10.0 eq.) in acetonitrile (15.0 mL). The mixture was heated to 70 °C and stirred for reaction for 3 h. The reaction liquid was poured into water (30 mL), and the mixture was extracted with DCM. The organic phases were combined and dried over anhydrous sodium sulfate. The mixture was filtered, and the filtrate was concentrated to dryness to give a crude product, which was purified by silica gel column chromatography to give compound 37-4 (1.4 g).

37-5 (211.0 mg, 0.50 mmol, 1.2 eq.) and 37-4 (150.0 mg, 0.42 mmol, 1.0 eq.) were added to a solution of *N,N-*dimethylformamide (2 mL), and potassium carbonate (173.9 mg, 1.26 mmol, 3.0 eq.) and sodium iodide (157.5 mg, 1.05 mmol, 2.5 eq.) were added under nitrogen atmosphere. The mixture was stirred at 70 °C for 6 h. After the reaction was completed, the reaction liquid was cooled to room temperature, diluted with water (10 mL), and then extracted with dichloromethane (3 × 50 mL). The organic phases were combined, washed with a saturated aqueous sodium chloride solution (3 × 50 mL), and dried over anhydrous sodium sulfate. The organic phase was collected by filtration, and the organic solvent was removed by using a rotary evaporator to give a crude product. The crude product was purified by high performance liquid chromatography (column: Xselect CSH F-Phenyl OBD column 19 × 250 mm, 5 µmn; phase A: acetonitrile/water (10 mmol/L ammonium bicarbonate + 0.05% ammonia water), phase B: isopropanol/acetonitrile; flow rate: 20 mL/min; gradient: 75% B to 95% B, 9 min) to give compound 37 (82 mg) as a yellow oil.

**¹H NMR** (300 MHz, CDCl₃) δ: 0.83-0.94 (m, 15H), 1.18-1.42 (m, 43H), 1.48-1.67 (m, 10H), 2.28-2.35 (m, 4H), 2.46-2.52 (m, 4H), 2.61 (t, *J* = 6.6 Hz, 2H), 3.61 (t, *J* = 6.6 Hz, 2H), 3.80 (s, 2H), 4.05 (t, *J=* 6.6 Hz, 2H); **ESI-MS** m/z: 696.60 [M+H]⁺.

### Example 38: Synthesis of Compound 38

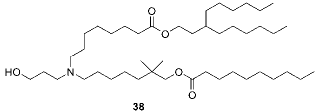

Compound 38 (138.0 mg) was prepared as an oily product by referring to the method of Example 37.

**¹H NMR** (300 MHz, CD₃OD) δ: 0.86-0.94 (m, 15H), 1.18-1.42 (m, 45H), 1.49-1.74 (m, 13H), 2.33 (q, *J* = 7.5 Hz, 4H), 2.47 (t, *J* = 7.5 Hz, 4H), 2.61 (t, *J* = 7.5Hz, 2H), 3.62 (t, *J* = 6.0 Hz, 2H), 3.80 (s, 2H), 4.10 (t, *J* = 6.6 Hz, 2H); **ESI-MS** m/z: 724.75 [M+H]⁺.

### Example 39: Synthesis of Compound 39

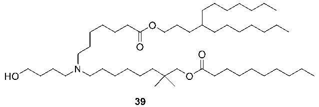

Compound 39 (113.5 mg) was prepared as an oily product by referring to the method of Example 37.

**¹H NMR** (300 MHz, CD₃OD) δ: 0.88-0.97 (m, 15H), 1.12-1.41 (m, 52H), 1.50-1.63 (m, 14H), 2.30-2.35 (m, 4H), 2.48-2.53 (m, 6H), 3.55 (t, *J =* 6.0 Hz, 2H), 3.80 (s, 2H), 4.06 (t, *J =* 6.6 Hz, 2H); **ESI-MS** m/z: 780.80 [M+H]⁺.

### Example 40: Synthesis of Compound 40

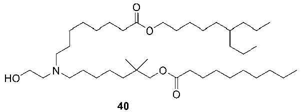

Compound 40 (90 mg) was prepared as an oily product by referring to the method of Example 37.

**¹H NMR** (300 MHz, CD₃OD) δ: 0.89-0.98 (m, 15H), 1.22-1.34 (m, 40H), 1.48-1.63 (m, 10H), 2.32 (q, *J* = 7.5 Hz, 4H), 2.50 (q, *J =* 7.5 Hz, 4H), 2.62 (t, *J =* 6.6 Hz, 2H), 3.62 (t, *J =* 6.6 Hz, 2H), 3.80 (s, 2H), 4.06 (t, *J* = 6.6 Hz, 2H); **ESI-MS** m/z: 668.70 [M+H]⁺.

### Example 41: Synthesis of Compound 41

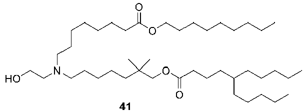

Compound 41 (174 mg) was prepared as an oily product by referring to the method of Example 37.

**¹H NMR** (400 MHz, CD₃OD) δ: 0.85-0.95 (m, 15H), 1.20-1.40 (m, 43H), 1.50-1.60 (m, 4H), 1.61-1.70 (m, 7H), 2.28-2.33 (m, 4H), 2.48-2.64 (m, 5H), 3.51-3.63 (m, 2H), 3.80 (s, 2H), 4.04-4.08 (m, 2H); **ESI-MS** m/z: 696.60 [M+H]⁺.

### Example 42: Synthesis of Compound 42

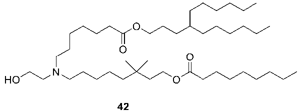

Compound 42 (93.6 mg) was prepared as an oily product by referring to the method of Example 37.

**¹H NMR** (300 MHz, CDCl₃) δ: 0.88-0.94 (m, 15H), 1.18-1.43 (m, 42H), 1.51-1.65 (m, 14H), 2.28-2.32 (m, 4H), 2.56-2.69 (m, 6H), 3.62 (m, 2H), 4.02-4.12 (m, 4H); **ESI-MS** m/z: 710.70 [M+H]⁺.

### Example 43: Synthesis of Compound 43

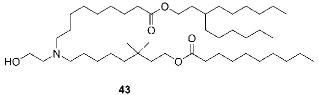

Compound 43 (185.7 mg) was prepared as an oily product by referring to the method of Example 37.

**¹H NMR** (300 MHz, CD₃OD) δ: 0.89-0.96 (m, 15H), 1.24-1.43 (m, 46H), 1.57-1.61 (m, 14H), 2.26-2.32 (m, 4H), 2.48-2.53 (m, 4H), 2.62 (t, *J =* 6.3 Hz, 2H), 3.61 (t, *J =* 6.6 Hz, 2H), 4.08-4.14 (m, 4H); **ESI-MS** m/z: 738.75 [M+H]⁺.

### Example 44: Synthesis of Compound 44

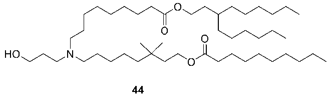

Compound 44 (177.7 mg) was prepared as an oily product by referring to the method of Example 37.

**¹H NMR** (300 MHz, CD₃OD) δ: 0.89-0.95 (m, 15H), 1.23-1.73 (m, 62H), 2.30 (dt, *J =* 4.2 Hz, 7.5 Hz, 4H), 2.43-2.56 (m, 4H), 2.61 (t, *J* = 6.9 Hz, 2H), 3.62 (t, *J* = 6.0 Hz, 2H), 4.08-4.14 (m, 4H); **ESI-MS** m/z: 752.70 [M+H]⁺.

### Example 45: Synthesis of Compound 45

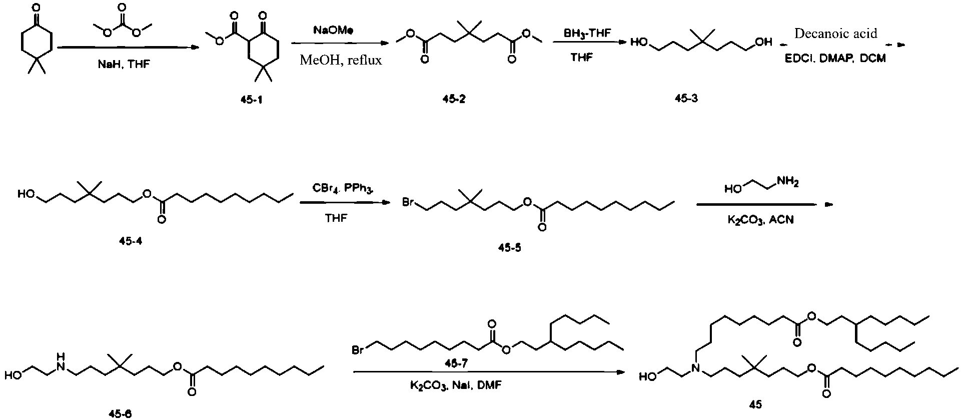

Dimethyl carbonate (10.7 g, 118.86 mmol, 3.0 eq.) was dissolved in a tetrahydrofuran solution (50 mL), and the reaction system was cooled to 0 °C. Sodium hydride (2.8 g, 118.86 mmol, 3.0 eq.) was added under nitrogen atmosphere. The mixture was stirred for reaction at 0 °C for 1 h. 4,4-Dimethylcyclohexanone (5.0 g, 39.62 mmol, 1.0 eq.) was then added. The mixture was stirred at reflux for reaction for 3 h. After the reaction was completed, the reaction liquid was cooled to room temperature, diluted with water (100 mL), and then extracted with dichloromethane (3 × 100 mL). The organic phases were combined, washed with a saturated aqueous sodium chloride solution (3 × 100 mL), and dried over anhydrous sodium sulfate. The organic phase was collected by filtration, and the organic solvent was removed by using a rotary evaporator to give a crude product. The crude product was separated and purified by silica gel column chromatography to give compound 45-1 (5.0 g) as a yellow oil.

45-1 (5.0 g, 27.139 mmol, 1.0 eq.) was dissolved in a methanol solution (50 mL), and sodium methoxide (4.4 g, 81.417 mmol, 3.0 eq.) was added under nitrogen atmosphere. The mixture was stirred at reflux for reaction for 12 h. After the reaction was completed, the reaction liquid was cooled to room temperature, diluted with water (100 mL), and extracted with dichloromethane (3 × 200 mL). The organic phases were combined, washed with a saturated aqueous sodium chloride solution (3 × 100 mL), and dried over anhydrous sodium sulfate. The organic phase was collected by filtration, and the organic solvent was removed by using a rotary evaporator to give compound 45-2 (5.0 g) as a yellow oil.

45-2 (5.0 g, 23.12 mmol, 1.0 eq.) was dissolved in a tetrahydrofuran solution (50 mL), and a borane-tetrahydrofuran solution (50.0 mL) was added under nitrogen atmosphere. The mixture was stirred for reaction at 75 °C for 3 h. After the reaction was completed, the reaction liquid was cooled to 0 °C, and methanol (10 mL) was added to quench the reaction. The organic solvent was removed by using a rotary evaporator to give a crude product, which was purified by silica gel column chromatography to give compound 45-3 (2.5 g) as a yellow oil.

45-3 (1.5 g, 9.36 mmol, 1.0 eq.) was dissolved in a dichloromethane solution (15 mL), and decanoic acid (1.8 g, 10.29 mmol, 1.1 eq.), EDCI (2.7 g, 14.04 mmol, 1.5 eq.), and N,N-dimethylaminopyridine (1.7 g, 14.04 mmol, 1.5 eq.) were added sequentially under nitrogen atmosphere. The mixture was stirred for reaction at room temperature for 12 h. After the reaction was completed, the reaction liquid was diluted with water (50 mL) and extracted with dichloromethane (3 × 50 mL). The organic phases were combined, washed with a saturated aqueous sodium chloride solution (3 × 50 mL), and dried over anhydrous sodium sulfate. The organic phase was collected by filtration, and the organic solvent was removed by using a rotary evaporator to give a crude product, which was purified by silica gel column chromatography to give compound 45-4 (1.5 g) as a yellow oil.

45-4 (1.5 g, 4.77 mmol, 1.0 eq.) was dissolved in a tetrahydrofuran solution (15 mL), and triphenylphosphine (3.7 g, 14.31 mmol, 3.0 eq.) and tetrabromomethane (4.7 g, 14.31 mmol, 3.0 eq.) were added under nitrogen atmosphere. The mixture was stirred for reaction at room temperature for 5 h. After the reaction was completed, the reaction liquid was diluted with water (50 mL) and extracted with dichloromethane (3 × 50 mL). The organic phases were combined, washed with a saturated aqueous sodium chloride solution (3 × 50 mL), and dried over anhydrous sodium sulfate. The organic phase was collected by filtration, and the organic solvent was removed by using a rotary evaporator to give a crude product, which was purified by silica gel column chromatography to give compound 45-5 (1.5 g) as a yellow oil.

Compound 45 (95 mg) was prepared as an oily product by referring to the method of Example 37.

**¹H NMR** (300 MHz, DMSO-*d₆*) δ: 0.89-0.98 (m, 15H), 1.18-1.55 (m, 46H), 1.57-1.61 (m, 8H), 2.28-2.33 (m, 4H), 2.45-2.64 (m, 6H), 3.59 (t, *J* = 6.6 Hz, 2H), 4.02-4.12 (m, 4H); **ESI-MS** m/z: 696.65 [M+H]⁺.

### Example 46: Synthesis of Compound 46

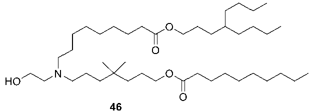

Compound 46 (99.3 mg) was prepared as an oily product by referring to the method of Example 45.

**¹H NMR** (400 MHz, CD₃OD) δ: 0.89-0.96 (m, 15H), 1.22-1.41 (m, 40H), 1.46-1.63 (m, 12H), 2.30 (t, *J* = 7.5 Hz, 4H), 2.44-2.52 (m, 4H), 2.61 (t, *J* = 6.6 Hz, 2H), 3.61 (t, *J* = 6.6 Hz, 2H), 4.02-4.07 (m, 4H); **ESI-MS** m/z: 682.65 [M+H]⁺.

### Example 47: Synthesis of Compound 47

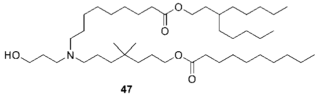

Compound 47 (92.2 mg) was prepared as an oily product by referring to the method of Example 45.

**¹H NMR** (400 MHz, CD₃OD) δ: 0.91-0.99 (m, 15H), 1.17-1.70 (m, 56H), 2.30-2.34 (m, 4H), 2.42-2.63 (m, 6H), 3.63 (t, *J* = 6.6 Hz, 2H), 4.03-4.13 (m, 4H); **ESI-MS** m/z: 710.65 [M+H]⁺.

### Example 48: Synthesis of Compound 48

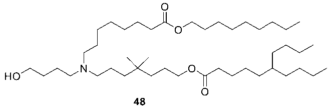

Compound 48 (94 mg) was prepared as an oily product by referring to the method of Example 45.

**¹H NMR** (400 MHz, CD₃OD) δ: 0.93-0.99 (m, 15H), 1.19-1.64 (m, 56H), 2.29-2.34 (m, 4H), 2.42-2.51 (m, 6H), 3.55 (t, *J* = 5.7 Hz, 2H), 4.02-4.08 (m, 4H); **ESI-MS** m/z: 710.75 [M+H]⁺.

### Example 49: Synthesis of Compound 49

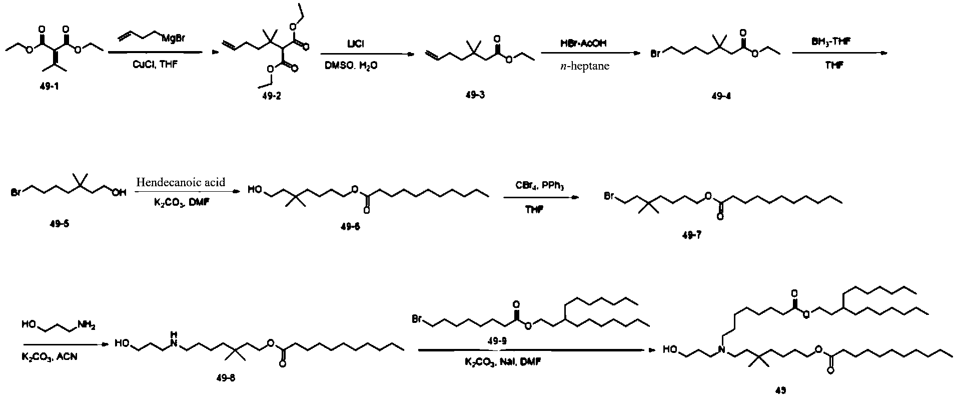

Diethyl isopropylidenemalonate (10.0 g, 49.94 mmol, 1.0 eq.) was dissolved in a tetrahydrofuran solution (50 mL), and cuprous chloride (500 mg, 5.0 mmol, 0.1 eq.) and 3-butenylmagnesium bromide (76.0 mL, 74.92 mmol, 1.5 eq.) were added at -30 °C under nitrogen atmosphere. The mixture was stirred for reaction at room temperature for 12 h. After the reaction was completed, the reaction liquid was diluted with ammonium chloride (100 mL) and extracted with dichloromethane (3 × 100 mL). The organic phases were combined, washed with a saturated aqueous sodium chloride solution (3 × 100 mL), and dried over anhydrous sodium sulfate. The organic phase was collected by filtration, and the organic solvent was removed by using a rotary evaporator to give a crude product, which was purified by silica gel column chromatography to give compound 49-2 (8.0 g) as a yellow oil.

49-2 (8.0 g, 31.21 mmol, 1.0 eq.) was dissolved in a mixed solution of dimethyl sulfoxide (100 mL) and water (1 mL), and lithium chloride (130.0 mg, 3.12 mmol, 0.1 eq.) was added under nitrogen atmosphere. The mixture was stirred for reaction at 180 °C for 8 h. After the reaction was completed, the reaction liquid was cooled to room temperature, diluted with water (100 mL), and extracted with dichloromethane (3 × 100 mL). The organic phases were combined, washed with a saturated aqueous sodium chloride solution (3 × 100 mL), and dried over anhydrous sodium sulfate. The organic phase was collected by filtration, and the organic solvent was removed by using a rotary evaporator to give a crude product, which was purified by silica gel column chromatography to give compound 49-3 (4.2 g) as a yellow oil.

49-3 (3.9 g, 21.16 mmol, 1.0 eq.) was dissolved in an n-heptane solution (40 mL), and a solution of hydrobromic acid in acetic acid (10.3 g, 42.33 mmol, 2.0 eq.) was added at 0 °C under nitrogen atmosphere. The mixture was stirred for reaction at room temperature for 2 h. After the reaction was completed, the reaction liquid was diluted with water (100 mL) and extracted with dichloromethane (3 × 100 mL). The organic phases were combined, washed with a saturated aqueous sodium chloride solution (3 × 100 mL), and dried over anhydrous sodium sulfate. The organic phase was collected by filtration, and the organic solvent was removed by using a rotary evaporator to give a crude product, which was purified by silica gel column chromatography to give compound 49-4 (3.1 g) as a yellow oil.

49-4 (3.0 g, 11.31 mmol, 1.0 eq.) was dissolved in a tetrahydrofuran solution (10 mL), and a borane-tetrahydrofuran solution (30.0 mL) was added at 0 °C under nitrogen atmosphere. The mixture was heated to 75 °C and stirred for reaction for 5 h. After the reaction was completed, the reaction liquid was cooled to 0 °C, and methanol (30 mL) was added to quench the reaction. The organic solvent was removed by using a rotary evaporator to give compound 49-5 (2.0 g) as a yellow oil.

49-5 (2.0 g, 8.96 mmol, 1.0 equiv.) was dissolved in DMF (20.0 mL), and potassium carbonate (3.7 g, 26.88 mmol, 3.0 eq.) and undecanoic acid (1.83 g, 9.85 mmol, 1.1 eq.) were added under nitrogen atmosphere. The mixture was stirred at 70 °C for 2 h. After the reaction was completed, the reaction liquid was cooled to room temperature, diluted with water (50 mL), and then extracted with ethyl acetate (3 × 50 mL). The organic phases were combined, washed with a saturated aqueous sodium chloride solution (3 × 50 mL), and dried over anhydrous sodium sulfate. The organic phase was collected by filtration, and the organic solvent was removed by using a rotary evaporator to give a crude product. The crude product was separated and purified by silica gel column chromatography to give compound 49-6 (2.4 g) as a yellow oil.

Compound 49 (91 mg) was prepared as an oily product by referring to the method of Example 45.

**¹H NMR** (300 MHz, CD₃OD) δ: 0.88-0.94 (m, 15H), 1.22-1.74 (m, 64H), 2.30 (t, *J* = 7.2 Hz, 4H), 2.49-2.52 (m, 4H), 2.60 (t, *J* = 7.2 Hz, 2H), 3.62 (t, *J* = 6.0 Hz, 2H), 4.06-4.12 (m, 4H); **ESI-MS** m/z: 766.70 [M+H]⁺.

### Example 50: Synthesis of Compound 50

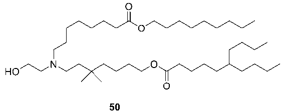

Compound 50 (77 mg) was prepared as an oily product by referring to the method of Example 49.

**¹H NMR** (300 MHz, CD₃OD) δ: 0.87-0.93 (m, 15H), 1.21-1.63 (m, 52H), 2.28-2.34 (m, 4H), 2.50-2.66 (m, 6H), 3.62 (t, *J* = 6.3 Hz, 2H), 4.04-4.10 (m, 4H); **ESI-MS** m/z: 682.70 [M+H]⁺.

### Example 51: Synthesis of Compound 51

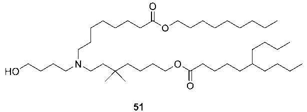

Compound 51 (90 mg) was prepared as an oily product by referring to the method of Example 49.

**¹H NMR** (400 MHz, CD₃OD) δ: 0.90-0.96 (m, 15H), 1.22-1.63 (m, 56H), 2.29-2.33 (m, 4H), 2.46-2.52 (m, 6H), 3.55 (t, *J* = 6.0 Hz, 2H), 4.04-4.10 (m, 4H); **ESI-MS** m/z: 710.90 [M+H]⁺.

### Example 52: Synthesis of Compound 52

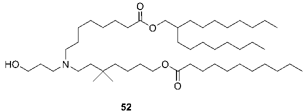

Compound 52 (95.1 mg) was prepared as an oily product by referring to the method of Example 49.

**¹H NMR** (400 MHz, CD₃OD) δ: 0.90-0.94 (m, 15H), 1.25-1.36 (m, 47H), 1.40-1.73 (m, 19H), 2.30-2.35 (m, 4H), 2.48-2.54 (m, 4H), 2.63 (t, *J* = 7.2 Hz, 2H), 3.64 (t, *J* = 6.0 Hz, 2H), 4.00 (d, *J* = 5.6 Hz, 2H), 4.10 (t, *J* = 6.4 Hz, 2H); **ESI-MS** m/z: 780.95 [M+H]⁺.

### Example 53: Synthesis of Compound 53

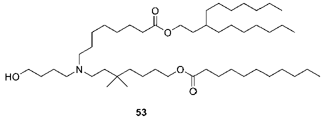

Compound 53 (76 mg) was prepared as an oily product by referring to the method of Example 49.

**¹H NMR** (300 MHz, CD₃OD) δ: 0.88-0.93 (m, 15H), 1.21-1.63 (m, 66H), 2.30 (t, *J* = 7.5 Hz, 4H), 2.46-2.53 (m, 6H), 3.55 (t, *J* = 6.0 Hz, 2H), 4.00 (d, *J* = 5.6 Hz, 2H), 4.06-4.12 (m, 4H); **ESI-MS** m/z: 780.80 [M+H]⁺.

### Example 54: Synthesis of Compound 54

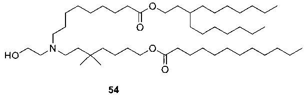

Compound 54 (90.5 mg) was prepared as an oily product by referring to the method of Example 49.

**¹H NMR** (300 MHz, CD₃OD) δ: 0.88-0.93 (m, 15H), 1.21-1.63 (m, 66H), 2.28-2.33 (m, 4H), 2.50-2.66 (m, 6H), 3.63 (t, *J* = 6.3 Hz, 2H), 4.10 (dd, *J* = 6.6 Hz, 12.6 Hz, 4H); **ESI-MS** m/z: 780.75 [M+H]⁺.

### Example 55: Synthesis of Compound 55

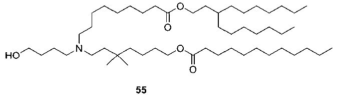

Compound 55 (75.7 mg) was prepared as an oily product by referring to the method of Example 49.

**¹H NMR** (300 MHz, CD₃OD) δ: 0.89-0.97 (m, 15H), 1.23-1.63 (m, 70H), 2.28-2.33 (m, 4H), 2.46-2.53 (m, 6H), 3.55 (t, *J* = 5.7 Hz, 2H), 4.10 (dd, *J* = 6.3 Hz, 12.6 Hz, 4H); **ESI-MS** m/z: 808.75 [M+H]⁺.

### Example 56: Synthesis of Compound 56

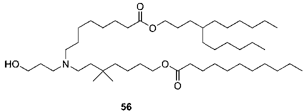

Compound 56 (94.1 mg) was prepared as an oily product by referring to the method of Example 49.

**¹H NMR** (400 MHz, CD₃OD) δ: 0.88-0.92 (m, 15H), 1.23-1.69 (m, 62H), 2.29-2.33 (m, 4H), 2.46-2.52 (m, 4H), 2.61 (t, *J* = 7.2 Hz, 2H), 3.62 (t, *J* = 6.0 Hz, 2H), 4.07 (dd, *J* = 6.4 Hz, 12.0 Hz, 4H); **ESI-MS** m/z: 752.90 [M+H]⁺.

### Example 57: Synthesis of Compound 57

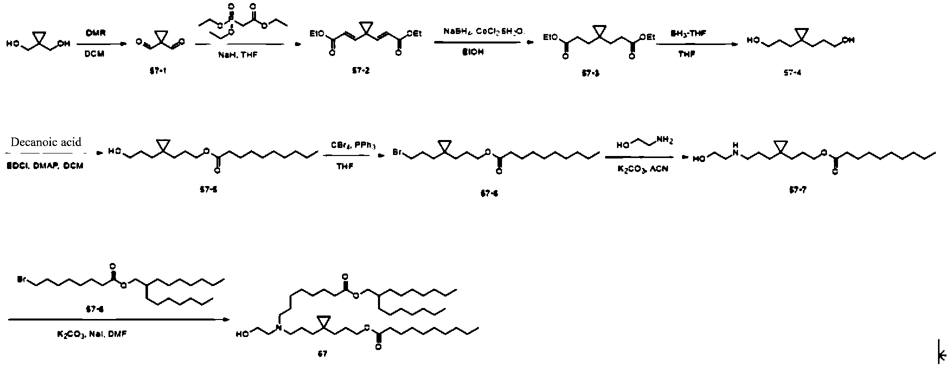

1,1-Cyclopropanedimethanol (6.0 g, 58.75 mmol, 1.0 eq.) was dissolved in a dichloromethane solution (60 mL), and Dess-Martin periodinane (74.8 g, 176.24 mmol, 3.0 eq.) was added at 0 °C under nitrogen atmosphere. The mixture was stirred for reaction at room temperature for 5 h. After the reaction was completed, the reaction liquid was diluted with water (50 mL), and the pH was adjusted to 7 with saturated sodium bicarbonate. The mixture was extracted with dichloromethane (3 × 100 mL). The organic phases were combined, washed with a saturated aqueous sodium chloride solution (3 × 100 mL), and dried over anhydrous sodium sulfate. The organic phase was collected by filtration, and the organic solvent was removed by using a rotary evaporator to give compound 57-1 (3.5 g) as a yellow oil.

A solution of sodium hydride (2.6 g, 61.2 mmol, 3.0 eq.) in tetrahydrofuran (30 mL) was cooled to 0 °C, and triethyl phosphorylacetate (13.7 g, 61.2 mmol, 3.0 eq.) was slowly added under nitrogen atmosphere. The mixture was stirred for reaction at room temperature for 1 h. The reaction system was then cooled to 0 °C, and 57-1 (2.0 g, 20.4 mmol, 1.0 eq.) was added to the reaction liquid. The mixture was stirred for reaction at room temperature for 12 h. After the reaction was completed, the reaction liquid was diluted with water (100 mL) and extracted with dichloromethane (3 × 100 mL). The organic phases were combined, washed with a saturated aqueous sodium chloride solution (3 × 100 mL), and dried over anhydrous sodium sulfate. The organic phase was collected by filtration, and the organic solvent was removed by using a rotary evaporator to give a crude product, which was purified by silica gel column chromatography to give compound 57-2 (2.4 g) as a yellow oil.

57-2 (2.4 g, 10.08 mmol, 1.0 eq.) was dissolved in an ethanol solution (20 mL), and cobalt chloride hexahydrate (476.4 mg, 2.01 mmol, 0.2 eq.) and sodium borohydride (1.5 g, 40.34 mmol, 4.0 eq.) were added at 0 °C under nitrogen atmosphere. The mixture was stirred for reaction at room temperature for 2 h. After the reaction was completed, the reaction liquid was diluted with water (50 mL) and extracted with dichloromethane (3 × 50 mL). The organic phases were combined, washed with a saturated aqueous sodium chloride solution (3 × 50 mL), and dried over anhydrous sodium sulfate. The organic phase was collected by filtration, and the organic solvent was removed by using a rotary evaporator to give a crude product, which was purified by silica gel column chromatography to give compound 57-3 (1.8 g) as a yellow oil.

57-3 (1.8 g, 7.43 mmol, 1 eq.) was dissolved in a tetrahydrofuran solution (10 mL), and a borane-tetrahydrofuran solution (18.0 mL) was added at 0 °C under nitrogen atmosphere. The mixture was stirred for reaction at 75 °C for 3 h. After the reaction was completed, the reaction liquid was cooled to room temperature, and methanol (10 mL) was added to quench the reaction. The organic solvent was removed by using a rotary evaporator to give compound 57-4 (1.0 g, 85%) as a yellow oil.

57-4 (500.0 mg, 3.16 mmol, 1.0 eq.) was dissolved in a dichloromethane solution (5 mL), and decanoic acid (653.2 mg, 3.79 mmol, 1.2 eq.), EDCI (908.6 mg, 4.74 mmol, 1.5 eq.), and DMPA (579.0 mg, 4.74 mmol, 1.5 eq.) were added sequentially under nitrogen atmosphere. The mixture was stirred for reaction at room temperature for 5 h. After the reaction was completed, the reaction liquid was diluted with water (50 mL) and extracted with dichloromethane (3 × 50 mL). The organic phases were combined, washed with a saturated aqueous sodium chloride solution (3 × 50 mL), and dried over anhydrous sodium sulfate. The organic phase was collected by filtration, and the organic solvent was removed by using a rotary evaporator to give a crude product, which was purified by silica gel column chromatography to give compound 57-5 (500 mg) as a yellow oil.

Compound 57 (77.7 mg) was prepared as an oily product by referring to the method of Example 45.

**¹H NMR** (400 MHz, CD₃OD) δ: 0.16 (m, 4H), 0.79-0.82 (m, 9H), 1.13-1.38 (m, 46H), 1.40-1.64 (m, 12H), 2.18-2.24 (m, 4H), 2.37-2.52 (m, 4H), 2.51 (t, *J* = 6.4 Hz, 2H), 3.51 (t, *J* = 6.4 Hz, 2H), 3.89 (d, *J* = 5.6 Hz, 2H), 4.00 (t, *J* = 6.4 Hz, 2H); **ESI-MS** m/z: 722.45 [M+H]⁺.

### Example 58: Synthesis of Compound 58

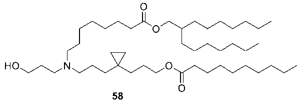

Compound 58 (85 mg) was prepared as an oily product by referring to the method of Example 57.

**¹H NMR** (300 MHz, CD₃OD) δ: 0.24-0.26 (m, 4H), 0.91-0.97 (m, 9H), 1.24-1.75 (m, 60H), 2.27-2.34 (m, 4H), 2.43-2.50 (m, 4H), 2.60 (t, *J =* 7.5 Hz, 2H), 3.62 (t, *J* = 6.0 Hz, 2H), 3.98 (d, *J* = 5.4 Hz, 2H), 4.06 (t, *J* = 6.6 Hz, 2H); **ESI-MS** m/z: 736.65 [M+H]⁺.

### Example 59: Synthesis of Compound 59

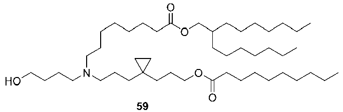

Compound 59 (90.2 mg) was prepared as an oily product by referring to the method of Example 57.

**¹H NMR** (300 MHz, CD₃OD) δ: 0.22 (m, 4H), 0.89-0.96 (m, 9H), 1.22-1.75 (m, 62H), 2.28-2.34 (m, 4H), 2.43-2.50 (m, 6H), 3.55 (t, *J* = 6.3 Hz, 2H), 3.98 (d, *J* = 5.7 Hz, 2H), 4.06 (t, *J* = 6.6 Hz, 2H); ESI-MS m/z: 750.55 [M+H]⁺.

### Example 60: Synthesis of Compound 60

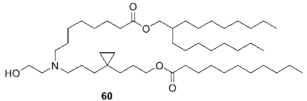

Compound 60 (80 mg) was prepared as an oily product by referring to the method of Example 57.

**¹H NMR** (300 MHz, CD₃OD) δ: 0.26-0.28 (m, 4H), 0.91-0.97 (m, 9H), 1.22-1.75 (m, 64H), 2.32 (dt, *J* = 5.1 Hz, 7.8 Hz, 4H), 2.47-2.53 (m, 4H), 2.62 (t, *J* = 6.6 Hz, 2H), 3.61 (t, *J* = 6.6 Hz, 2H), 3.98 (d, *J* = 5.7 Hz, 2H), 4.06 (t, *J* = 6.6 Hz, 2H); **ESI-MS** m/z: 764.75 [M+H]⁺.

### Example 61: Synthesis of Compound 61

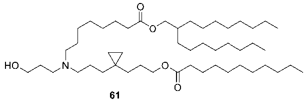

Compound 61 (85 mg) was prepared as an oily product by referring to the method of Example 57.

**¹H NMR** (500 MHz, CD₃OD) δ: 0.28-0.29 (m, 4H), 0.91-0.94 (m, 9H), 1.26-1.37 (m, 52H), 1.50-1.74 (m, 14H), 2.31-2.36 (m, 4H), 2.47-2.51 (m, 4H), 2.63 (t, *J* = 7.0 Hz, 2H), 3.65 (t, *J* = 6.5 Hz, 2H), 4.01 (d, *J* = 5.5 Hz, 2H), 4.10 (t, *J* = 6.5 Hz, 2H); **ESI-MS** m/z: 778.70 [M+H]⁺.

### Example 62: Synthesis of Compound 62

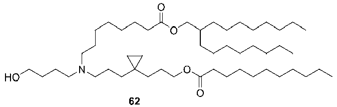

Compound 62 (90 mg) was prepared as an oily product by referring to the method of Example 57.

**¹H NMR** (300 MHz, CD₃OD) δ: 0.24-0.26 (m, 4H), 0.88-0.92 (m, 9H), 1.25-1.41 (m, 52H), 1.52-1.73 (m, 16H), 2.28-2.34 (m, 4H), 2.48-2.54 (m, 6H), 3.55 (t, *J* = 6.0 Hz, 2H), 4.00 (d, *J* = 5.7 Hz, 2H), 4.07 (t, *J* = 6.6 Hz, 2H); **ESI-MS** m/z: 792.65 [M+H]⁺.

### Example 63: Synthesis of Compound 63

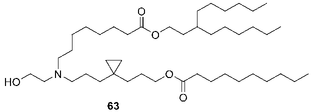

Compound 63 (89.6 mg) was prepared as an oily product by referring to the method of Example 57.

**¹H NMR** (400 MHz, CD₃OD) δ: 0.25-0.26 (m, 4H), 0.88-0.92 (m, 9H), 1.23-1.34 (m, 41H), 1.40-1.72 (m, 15H), 2.30 (t, *J =* 7.6 Hz, 4H), 2.47-2.52 (m, 4H), 2.61 (t, *J* = 6.4 Hz, 2H), 3.61 (t, *J* = 6.4 Hz, 2H), 4.05-4.11 (m, 4H); **ESI-MS** m/z: 708.75 [M+H]⁺.

### Example 64: Synthesis of Compound 64

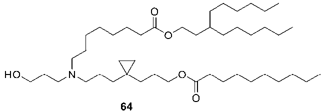

Compound 64 (79.7 mg) was prepared as an oily product by referring to the method of Example 57.

**¹H NMR** (400 MHz, CD₃OD) δ: 0.25-0.26 (m, 4H), 0.88-0.92 (m, 9H), 1.23-1.72 (m, 58H), 2.28-2.32 (dt, *J* = 1.6Hz, 7.6 Hz, 4H), 2.44-2.49 (m, 4H), 2.60 (t, *J* = 7.2 Hz, 2H), 3.62 (t, *J* = 6.0 Hz, 2H), 4.05-4.12 (m, 4H); **ESI-MS** m/z: 722.70 [M+H]⁺.

### Example 65: Synthesis of Compound 65

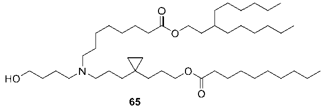

Compound 65 (101.4 mg) was prepared as an oily product by referring to the method of Example 57.

**¹H NMR** (300 MHz, CD₃OD) δ: 0.25-0.26 (m, 4H), 0.88-0.92 (m, 9H), 1.23-1.75 (m, 60H), 2.30 (t, *J* = 7.2 Hz, 4H), 2.44-2.50 (m, 6H), 3.55 (t, *J* = 6.0 Hz, 2H), 4.04-4.12 (m, 4H); **ESI-MS** m/z: 736.85 [M+H]⁺.

### Example 66: Synthesis of Compound 66

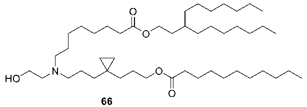

Compound 66 (106.4 mg) was prepared as an oily product by referring to the method of Example 57.

**¹H NMR** (300 MHz, CD₃OD) δ: 0.28-0.29 (m, 4H), 0.89-0.93 (m, 9H), 1.26-1.75 (m, 62H), 2.30 (t, *J* = 7.5 Hz, 4H), 2.48-2.54 (m, 4H), 2.63 (t, *J* = 6.3 Hz, 2H), 3.61 (t, *J* = 6.3 Hz, 2H), 4.08 (t, *J =* 6.6 Hz, 2H), 4.10 (t, *J* = 6.6 Hz, 2H); **ESI-MS** m/z: 750.70 [M+H]⁺.

### Example 67: Synthesis of Compound 67

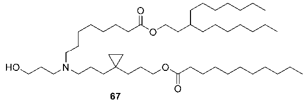

Compound 67 (85 mg) was prepared as an oily product by referring to the method of Example 57.

**¹H NMR** (500 MHz, CD₃OD) δ: 0.28-0.29 (m, 4H), 0.91-0.94 (m, 9H), 1.26-1.74 (m, 64H), 2.32 (dt, *J =* 2.5Hz, 7.5 Hz, 4H), 2.48-2.52 (m, 4H), 2.64 (t, *J* = 7.5 Hz, 2H), 3.65 (t, *J* = 7.0 Hz, 2H), 4.09 (t, *J* = 6.5 Hz, 2H), 4.12 (t, *J* = 6.5 Hz, 2H); **ESI-MS** m/z: 764.65 [M+H]⁺.

### Example 68: Synthesis of Compound 68

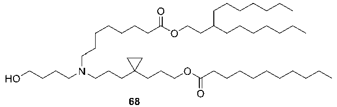

Compound 68 (86.8 mg) was prepared as an oily product by referring to the method of Example 57.

**¹H NMR** (400 MHz, CD₃OD) δ: 0.25-0.26 (m, 4H), 0.91-0.94 (m, 9H), 1.24-1.74 (m, 66H), 2.30 (dt, *J* = 1.6 Hz, 7.6 Hz, 4H), 2.45-2.50 (m, 6H), 3.55 (t, *J* = 5.6 Hz, 2H), 4.05-4.12 (m, 4H); **ESI-MS** m/z: 778.80 [M+H]⁺.

### Example 69: Synthesis of Compound 69

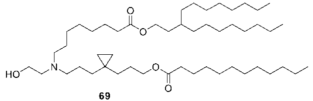

Compound 69 (80 mg) was prepared as an oily product by referring to the method of Example 57.

**¹H NMR** (300 MHz, CD₃OD) δ: 0.25-0.26 (m, 4H), 0.89-0.94 (m, 9H), 1.26-1.73 (m, 68H), 2.30 (t, *J* = 7.5 Hz, 4H), 2.47-2.54 (m, 4H), 2.62 (t, *J* = 6.6 Hz, 2H), 3.61 (t, *J* = 6.3 Hz, 2H), 4.04-4.12 (m, 4H); **ESI-MS** m/z: 792.80 [M+H]⁺.

### Example 70: Synthesis of Compound 70

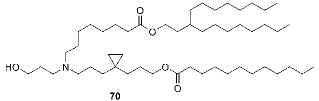

Compound 70 (87 mg) was prepared as an oily product by referring to the method of Example 57.

**¹H NMR** (300 MHz, CD₃OD) δ: 0.26-0.27 (m, 4H), 0.88-0.92 (m, 9H), 1.26-1.73 (m, 70H), 2.30 (t, *J* = 7.5 Hz, 4H), 2.43-2.49 (m, 4H), 2.59 (t, *J* = 7.5 Hz, 2H), 3.62 (t, *J* = 6.0 Hz, 2H), 4.04-4.12 (m, 4H); **ESI-MS** m/z: 806.80 [M+H]⁺.

### Example 71: Synthesis of Compound 71

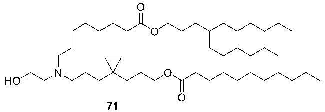

Compound 71 (97 mg) was prepared as an oily product by referring to the method of Example 57.

**¹H NMR** (500 MHz, CD₃OD) δ: 0.28-0.29 (m, 4H), 0.94-0.98 (m, 9H), 1.29-1.75 (m, 60H), 2.33 (dt, *J* = 5.5 Hz, 7.5 Hz, 4H), 2.50-2.54 (m, 4H), 2.64 (t, *J* = 6.5 Hz, 2H), 3.63 (t, *J* = 6.5 Hz, 2H), 4.07 (q, *J* = 6.5 Hz, 4H); **ESI-MS** m/z: 736.70 [M+H]⁺.

### Example 72: Synthesis of Compound 72

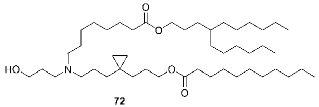

Compound 72 (83.3 mg) was prepared as an oily product by referring to the method of Example 57.

**¹H NMR** (500 MHz, CD₃OD) δ: 0.28-0.29 (m, 4H), 0.91-0.94 (m, 9H), 1.26-1.38 (m, 47H), 1.50-1.73 (m, 15H), 2.31-2.35 (m, 4H), 2.46-2.50 (m, 4H), 2.62 (t, *J* = 7.0 Hz, 2H), 3.65 (t, *J* = 6.0 Hz, 2H), 4.08 (q, *J* = 7.0 Hz, 4H); **ESI-MS** m/z: 750.65 [M+H]⁺.

### Example 73: Synthesis of Compound 73

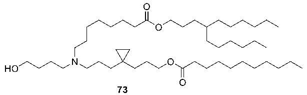

Compound 73 (99 mg) was prepared as an oily product by referring to the method of Example 57.

**¹H NMR** (400 MHz, CD₃OD) δ: 0.26-0.27 (m, 4H), 0.88-0.92 (m, 9H), 1.16-1.40 (m, 46H), 1.45-1.74 (m, 18H), 2.28-2.33 (m, 4H), 2.44-2.49 (m, 6H), 3.55 (t, *J =* 5.6 Hz, 2H), 4.06 (q, *J =* 6.4 Hz, 4H); **ESI-MS** m/z: 765.00 [M+H]⁺.

### Example 74: Synthesis of Compound 74

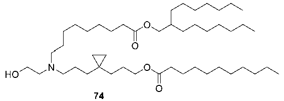

Compound 74 (80 mg) was prepared as an oily product by referring to the method of Example 57.

**¹H NMR** (300 MHz, CD₃OD) δ: 0.26-0.28 (m, 4H), 0.91-0.97 (m, 9H), 1.23-1.75 (m, 62 H), 2.31 (dt, *J* = 4.2 Hz, 7.2 Hz, 4H), 2.47-2.53 (m, 4H), 2.62 (t, *J* = 6.3 Hz, 2H), 3.61 (t, *J* = 6.6 Hz, 2H), 4.00 (d, *J =* 6.0 Hz, 2H), 4.06 (t, *J* = 6.6 Hz, 2H); **ESI-MS** m/z: 750.75 [M+H]⁺.

### Example 75: Synthesis of Compound 75

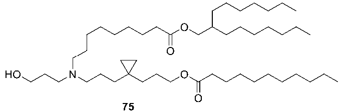

Compound 75 (83 mg) was prepared as an oily product by referring to the method of Example 57.

**¹H NMR** (300 MHz, CD₃OD) δ: 0.26-0.27 (m, 4H), 0.91-0.94 (m, 9H), 1.22-1.40 (m, 50H), 1.45-1.75 (m, 14H), 2.27-2.34 (m, 4H), 2.45-2.51 (m, 4H), 2.61 (t, *J* = 6.9 Hz, 2H), 3.63 (t, *J* = 6.0 Hz, 2H), 3.98 (d, *J* = 5.4 Hz, 2H), 4.06 (t, *J* = 6.6 Hz, 2H); **ESI-MS** m/z: 764.70 [M+H]⁺.

### Example 76: Synthesis of Compound 76

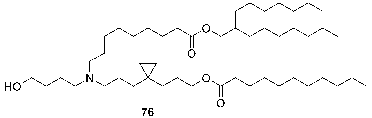

Compound 76 (100.4 mg) was prepared as an oily product by referring to the method of Example 57.

**¹H NMR** (400 MHz, CD₃OD) δ: 0.28-0.29 (m, 4H), 0.93-0.97 (m, 9H), 1.25-1.42 (m, 50H), 1.47-1.74 (m, 16H), 2.28-2.33 (m, 4H), 2.49-2.52 (m, 6H), 3.55 (t, *J* = 6.0 Hz, 2H), 3.98 (d, *J* = 5.6 Hz, 2H), 4.06 (t, *J* = 6.8 Hz, 2H); **ESI-MS** m/z: 778.80 [M+H]⁺.

### Example 77: Synthesis of Compound 77

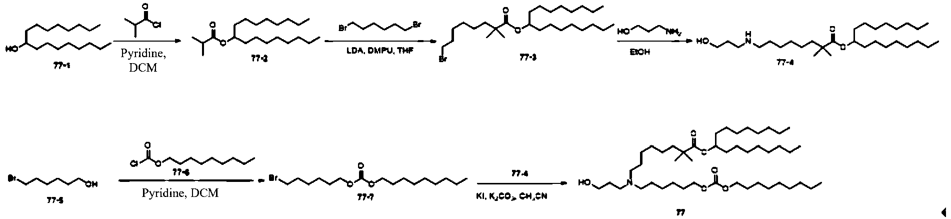

In a 250 mL round-bottom flask, 9-heptadecanol (10.0 g, 39.0 mmol, 1.0 eq.) and pyridine (6.17 g, 78.0 mmol, 2.0 eq.) were dissolved in dichloromethane (100 mL), and then the reaction system was cooled to 0 °C. Isobutyryl chloride (10.39 g, 97.5 mmol, 2.5 eq.) was slowly added to the reaction liquid, and then the mixture was stirred at room temperature for 2 h. Water was added to quench the reaction at 0 °C, and the mixture was extracted with dichloromethane. The organic phases were combined, dried over anhydrous Na₂SO₄, and filtered to remove the drying agent, and the solvent was removed by rotary evaporation. The resulting crude product was purified by silica gel column chromatography to give compound 77-2 (10.4 g) as a yellow oil. Compound 77-2 (10.0 g, 30.62 mmol, 1.0 eq.) was dissolved in anhydrous THF (100 mL), and the reaction system was cooled to -40 °C. LDA (15.3 mL, 30.6 mmol, 1.0 eq.) was added to the reaction liquid under nitrogen atmosphere. After the mixture was stirred for reaction at -40 °C for 1 h, 1,6-dibromohexane (14.9 g, 61.2 mmol, 2.0 eq.) and DMPU (471 mg, 3.7 mmol, 0.12 eq.) were added at the same temperature. The reaction system was slowly warmed to room temperature and then reacted overnight. After the reaction was completed, the reaction liquid was added to a saturated NH₄Cl solution, and the mixture was extracted with dichloromethane. The organic phases were combined, dried over anhydrous Na₂SO₄, and filtered to remove the drying agent, and the solvent was removed by rotary evaporation to give a crude product of compound 77-3, which was directly used in the next step without purification.

Compound 77-3 (15 g, 30.6 mmol, 1.0 eq.) and 3-hydroxypropylamine (45.0 g, 612.0 mmol, 20.0 equiv) were dissolved in ethanol (80 mL), and the reaction system was heated to 60 °C and reacted for 2 h. After the reaction was completed, the reaction system was cooled to room temperature, and the ethanol solvent was removed by rotary evaporation. The crude product was dissolved in ethyl acetate, and then a saturated sodium chloride solution was added. After extraction, the organic phases were combined, dried over anhydrous Na₂SO₄, and filtered to remove the drying agent, and the solvent was removed by rotary evaporation. The resulting crude product was purified by silica gel column chromatography to give compound 77-4 (12.6 g) as a yellow oil.

In a 50 mL reaction flask, 6-bromo-1-hexanol (1.5 g, 8.3 mmol, 1.0 eq.) and pyridine (1.31 g, 16.6 mmol, 2.0 eq.) were dissolved in dichloromethane (15 mL), and n-nonyl chloroformate (1.88 g, 9.1 mmol, 1.1 equiv) was added dropwise within 15 min in an ice bath. The mixture was left to stand at room temperature overnight. A saturated aqueous ammonium chloride solution was added to quench the reaction, and the mixture was extracted with dichloromethane. The organic phases were combined, dried over anhydrous Na₂SO₄, and filtered to remove the drying agent, and the solvent was removed by rotary evaporation. The resulting crude product was purified by silica gel column chromatography to give compound 77-7 (2.5 g) as a yellow oil.

In an 8 mL sealed tube, compound 77-7 (200 mg, 0.57 mmol, 1.0 eq.), compound 77-4 (304.8 mg, 0.63 mmol, 1.1 eq.), KI (113.4 mg, 0.68 mmol, 1.2 eq.), K₂CO₃ (236.0 mg, 1.71 mmol, 3.0 eq.), and anhydrous acetonitrile (5.0 mL) were added. The mixture was heated to 80 °C, stirred for reaction overnight, cooled to room temperature, and filtered. After the filter cake was washed with acetonitrile, the organic phases were combined, and the solvent was removed by rotary evaporation to give a reaction crude product. The reaction crude product was purified by preparative liquid chromatography to give 77 (93.9 mg).

**¹H NMR** (300 MHz, CDCl₃) δ: 4.85 (p, *J =* 6.6 Hz, 1H), 4.12 (t, *J =* 6.6 Hz, 4H), 3.79 (t, *J =* 5.1 Hz, 2H), 2.65 (m, 2H), 2.42 (m, 4H), 1.77-1.64 (m, 6H), 1.51 (t, *J* = 6.0 Hz, 10H), 1.28 (d, *J* = 4.5 Hz, 47H), 1.17 (s, 6H), 0.88 (t, *J* = 6.6 Hz, 9H); **ESI-MS** m/z: 754.60 [M+H]⁺.

The compounds in Table 3 were synthesized using the methods of the above examples, or using similar methods of the corresponding intermediates.

**Table 3**

| | |
|---|---|
| 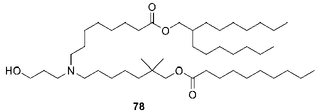 Example 78 | 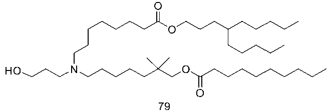 Example 79 |
| [M+H]⁺: 738.75 | [M+H]⁺: 710.65 |
| 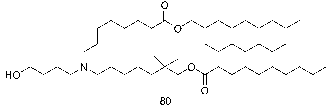 Example 80 | 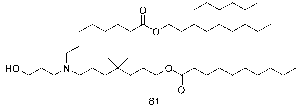 Example 81 |
| [M+H]⁺: 752.90 | [M+H]⁺: 724.70 |
| 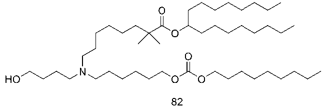 Example 82 | 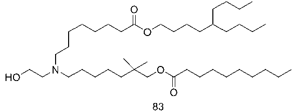 Example 83 |
| [M+H]⁺: 768.65 | [M+H]⁺: 704.80 |
| 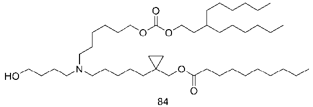 Example 84 | 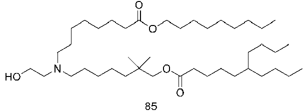 Example 85 |
| [M+H]⁺: 738.85 | [M+H]⁺: 682.55 |
| 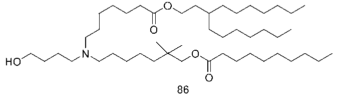 Example 86 | 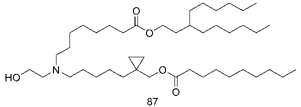 Example 87 |
| [M+H]⁺: 752.75 | |
| | [M+H]⁺: 730.65 |
| 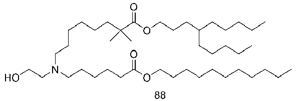 Example 88 | 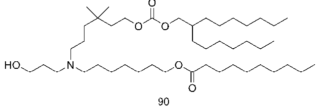 Example 90 |
| [M+H]⁺: 696.55 | [M+H]⁺: 740.65 |
| 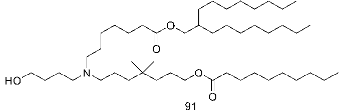 Example 91 | 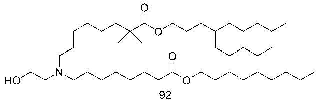 Example 92 |
| [M+H]⁺: 766.65 | [M+H]⁺: 696.50 |
| 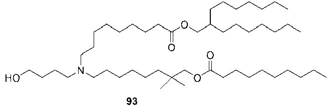 Example 93 | 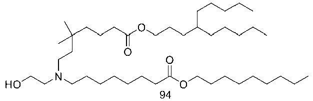 Example 94 |
| [M+H]⁺: 780.65 | [M+H]⁺: 682.85 |
| 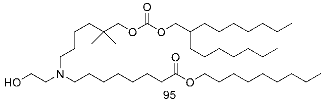 Example 95 | 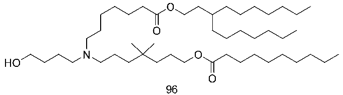 Example 96 |
| [M+H]⁺: 726.80 | [M+H]⁺: 752.70 |

### Pharmacological Experiments

### Experimental Example 1: Preparation of Nanoparticle

Materials used for lipid nanoparticle assembly were as follows: (1) an ionizable lipid compound: the synthesized ionizable lipid as designed in the present disclosure or SM102 (purchased from AVT/MCE) as a control group; (2) a structural lipid: such as cholesterol (purchased from Sigma-Aldrich); (3) a phospholipid: such as DSPC (1,2-distearoyl-SN-glycero-3-phosphocholine or distearoylphosphatidylcholine, purchased from AVT); (4) a PEGylated lipid compound: such as DSPE-PEG2000 (distearoyl phosphatidylethanolamine-polyethylene glycol 2000 or distearoyl phosphoethanolamine-PEG2000, purchased from AVT/SINOPEG); and (5) a nucleic acid fragment (active ingredient): such as luciferase mRNA (Trilink, L-7202). The names and structural formulas of the materials for lipid nanoparticle assembly are shown in Table 4.

**Table 4**

| No. | Name of structure | Structural formula |
|---|---|---|
| 1 | SM102 | 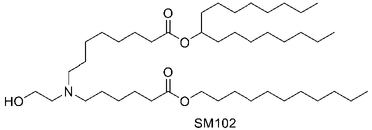 |
| 2 | Cholesterol | 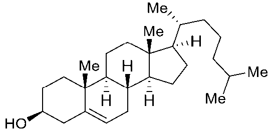 |
| 3 | DSPC | 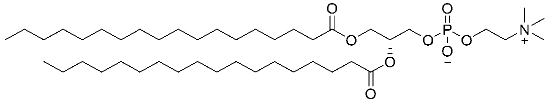 |
| 4 | DSPE-PEG2000 | 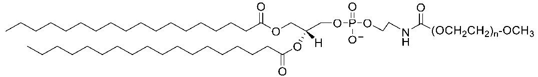 |

The lipid nanoparticle was prepared as follows: (1) the ionizable lipid compound, cholesterol, phospholipid, and PEGylated lipid were sequentially dissolved and mixed in ethanol according to a (mol%) ratio of 50%:38.5%:10%:1.5%; (2) the mRNA active ingredient was dissolved in 10 mM-25 mM citric acid buffer (pH = 4.0-4.5); (3) the organic phase containing the lipid mixture and the aqueous phase containing the mRNA component were mixed according to a flow rate ratio of 1:3 by using an automatic high-throughput microfluidic system, wherein the mixing speed was 5 mL/min to 20 mL/min; (4) the prepared lipid nanoparticle was diluted with a phosphate buffered saline, and the nanoparticle solution was ultrafiltered to the original preparation volume using an ultrafiltration tube (purchased from Millipore) with a cut-off molecular weight of 100 kDa; and (5) the resulting nanoparticle was stored in a sealed EP tube at 4 °C.

### Experimental Example 2: Physical Property Characterization of Lipid Nanoparticle

The particle size and particle size dispersity index (PDI) of the prepared lipid nanoparticle were measured using Zetasizer Pro (purchased from Malvern Instruments Ltd.) and DynaPro NanoStar (purchased from Wyatt) dynamic light scattering instruments. The encapsulation degree of RNA in the lipid nanoparticle was characterized by the encapsulation efficiency% (EE%), which represents the combination degree of the lipid nanoparticle and the RNA fragment. This coefficient was measured by the method of Quant-it^{™} RiboGreen RNA Assay (purchased from Invitrogen). Lipid nanoparticle samples were diluted in a TE buffer (10 mM Tris-HCl, 1 mM EDTA, pH = 7.5). A portion of the sample solution was taken and 0.5% Triton (Triton X-100) was added. The mixture was let to stand at 37 °C for 30 min. After the RIBOGREEN^{®} reaction liquid was added, the fluorescence value was immediately read in a Varioskan LUX multifunctional microplate reader (purchased from Thermofisher) under a condition of an absorption light at 485 nm and an emission light at 528 nm, so as to give the encapsulation efficiency.

### Experimental Example 3: Animal Experiment

The delivery effect and safety of a luciferase mRNA-encapsulated nanoparticle were assessed in mice. The test mice were SPF-grade female C57BL/6 mice aged 6-8 weeks and weighing 18-22 g, which were purchased from SPF (Beijing) Biotechnology Co., Ltd. All animals were acclimatized for 7 days or more before the test, and were given free access to food and water during the test. The mice were kept in 12/12-h light/dark cycles at an ambient temperature of 20-26 °C and with a humidity of 40%-70%. The mice were randomized. The luciferase mRNA-encapsulated lipid nanoparticle prepared above was injected into the mice (n = 3) at a single dose of 2 mg/kg mRNA by intravenous administration, and the mice were subjected to *in vivo* bioluminescence assay on a small animal *in vivo* imaging system (IVIS LUMINA III, purchased from PerkinElmer) 6 h after the administration. The mice were shaved for skin preparation before the imaging. The specific procedures of the test were as follows: a D-fluorescein solution at a concentration of 20 mg/mL was prepared in normal saline, and the substrate was given by intraperitoneal injection to 3 mice. The mice were placed in an anesthesia box for anesthesia with 2.5% isoflurane after the substrate was given for 10 min. The anesthetized mice were placed in the IVIS and subjected to fluorescence imaging to obtain *in vivo* muscle signals. After the *in vivo* imaging, the mice were sacrificed by cervical dislocation, and livers were collected from each mouse for *ex vivo* imaging to obtain *ex vivo* liver signals.

The *in vivo* delivery efficiency of the lipid nanoparticle carrier was expressed as the mean photon flux [p/s/cm²/sr] of the fluorescence intensity of different animals in the same test group, as shown in Table 5. The region of interest (ROI) was divided by IVIS software, the muscle expression was represented by leg muscles, the leg muscles were circled, the *ex vivo* liver was circled, and the mean value was calculated. A higher average photon count indicates a higher *in vivo* delivery efficiency of the lipid nanoparticle for the mRNA fragment. The data indicate that the lipid nanoparticle containing the cationic lipid of the present disclosure has good *in vivo* muscle-specific delivery efficiency.

**Table 5**

| **Compound** | **Particle size (nm)** | **Particle size Distribution (PDI)** | **Encapsulation efficiency (%)** | **Mean fluorescence intensity of muscle *in vivo* (p/s/cm²/sr)** | **Mean fluorescence intensity of liver *ex vivo* (p/s/cm²/sr)** | **Muscle/liver Ratio** |
|---|---|---|---|---|---|---|
| 1 | 115.5 | 0.096 | 98.9 | 5.11E+07 | 2.90E+07 | 1.76 |
| 4 | 115.0 | 0.196 | 97.9 | 7.47E+07 | 2.64E+07 | 2.83 |
| 8 | 106.2 | 0.072 | 98.2 | 1.14E+08 | 3.83E+07 | 2.98 |
| 10 | 116.7 | 0.211 | 97.7 | 8.56E+07 | 5.84E+07 | 1.47 |
| 15 | 98.0 | 0.161 | 98.5 | 1.41E+08 | 9.89E+07 | 1.43 |
| 24 | 102.0 | 0.101 | 98.9 | 5.26E+07 | 9.48E+07 | 0.55 |
| 25 | 116.5 | 0.106 | 97.8 | 1.14E+08 | 2.34E+07 | 4.87 |
| 34 | 131.8 | 0.150 | 99.0 | 3.32E+07 | 4.79E+07 | 0.69 |
| 36 | 109.0 | 0.180 | 99.3 | 3.80E+07 | 6.67E+07 | 0.57 |
| 37 | 134.1 | 0.250 | 97.8 | 1.36E+08 | 7.35E+07 | 1.85 |
| 38 | 139.5 | 0.100 | 89.6 | 1.46E+08 | 1.49E+08 | 0.98 |
| 39 | 143.7 | 0.080 | 87.7 | 1.40E+08 | 3.91E+08 | 0.36 |
| 41 | 121.3 | 0.216 | 96.1 | 1.54E+08 | 7.44E+07 | 2.06 |
| 44 | 137.3 | 0.106 | 83.5 | 7.68E+07 | 5.13E+07 | 1.50 |
| 52 | 110.2 | 0.064 | 82.9 | 1.39E+08 | 1.82E+08 | 0.76 |
| 56 | 132.5 | 0.113 | 87.0 | 2.45E+08 | 9.26E+07 | 2.64 |
| 58 | 116.9 | 0.093 | 88.3 | 1.97E+08 | 1.80E+08 | 1.09 |
| 61 | 109.5 | 0.126 | 88.9 | 1.77E+08 | 1.34E+08 | 1.33 |
| 62 | 146.9 | 0.084 | 81.1 | 9.28E+07 | 1.15E+08 | 0.81 |
| 63 | 117.5 | 0.188 | 91.1 | 6.28E+07 | 5.53E+07 | 1.14 |
| 64 | 134.7 | 0.143 | 94.7 | 1.79E+08 | 1.27E+08 | 1.41 |
| 67 | 118.1 | 0.174 | 88.1 | 2.66E+08 | 1.89E+08 | 1.40 |
| 70 | 114.3 | 0.093 | 90.3 | 1.78E+08 | 1.03E+08 | 1.72 |
| 71 | 108.1 | 0.114 | 89.4 | 1.41E+08 | 1.60E+08 | 0.88 |
| 72 | 119.2 | 0.115 | 89.6 | 2.31E+08 | 1.01E+08 | 2.29 |
| 75 | 123.1 | 0.091 | 86.2 | 6.96E+07 | 8.79E+07 | 0.79 |
| 77 | 123.3 | 0.205 | 95.8 | 1.66E+08 | 4.68E+07 | 3.54 |
| 78 | 132.3 | 0.173 | 90.8 | 1.90E+08 | 1.59E+08 | 1.19 |
| 79 | 141.1 | 0.089 | 88.9 | 1.51E+08 | 8.08E+07 | 1.87 |
| 80 | 130.1 | 0.116 | 85.1 | 1.32E+08 | 3.56E+08 | 0.37 |
| 81 | 175.5 | 0.153 | 87.7 | 1.21E+08 | 9.07E+07 | 1.33 |
| 82 | 134.1 | 0.200 | 95.4 | 1.09E+08 | 1.01E+08 | 1.08 |
| 83 | 135.6 | 0.175 | 84.8 | 1.05E+08 | 3.29E+07 | 3.19 |
| 84 | 125.8 | 0.110 | 97.4 | 8.81E+07 | 2.55E+07 | 3.46 |
| 85 | 125.2 | 0.152 | 91.7 | 8.71E+07 | 1.34E+08 | 0.65 |
| 87 | 149.3 | 0.226 | 96.3 | 8.03E+07 | 3.91E+07 | 2.06 |
| 88 | 114.0 | 0.212 | 97.9 | 7.86E+07 | 6.27E+07 | 1.25 |
| 90 | 135.5 | 0.129 | 93.4 | 7.18E+07 | 8.46E+07 | 0.85 |
| 91 | 142.4 | 0.121 | 87.3 | 6.15E+07 | 3.18E+07 | 1.93 |
| 92 | 133.1 | 0.143 | 93.2 | 5.65E+07 | 1.14E+08 | 0.50 |
| 93 | 117.0 | 0.058 | 87.6 | 4.94E+07 | 6.14E+07 | 0.80 |
| 94 | 125.1 | 0.313 | 97.6 | 4.79E+07 | 2.69E+07 | 1.78 |
| 95 | 144.2 | 0.251 | 92.4 | 4.72E+07 | 4.97E+07 | 0.95 |
| 96 | 216.2 | 0.179 | 69.5 | 4.25E+07 | 4.14E+07 | 1.03 |
| SM102 | 100.9 | 0.226 | 97.9 | 2.64E+07 | 1.63E+08 | 0.25 |

Although the present disclosure has been comprehensively illustrated by means of embodiments thereof, it should be noted that various changes and modifications will be apparent to those skilled in the art. Such changes and modifications shall fall within the scope of the appended claims.

## Claims

1. A compound of formula (I), or an isotopic variant, tautomer, or stereoisomer thereof, or a pharmaceutically acceptable salt thereof: 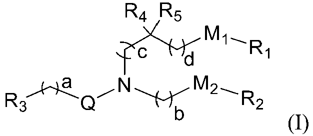 wherein,
a = 1, 2, 3, 4, 5, or 6;
b = 4, 5, 6, 7, 8, 9, or 10;
c = 1, 2, 3, 4, 5, or 6;
d = 0, 1, 2, 3, 4, or 5;
c + d = 3, 4, 5, 6, 7, 8, or 9;
M₁ and M₂ are independently selected from -C(O)O-, -OC(O)-, -O-, -SC(O)O-, -OC(O)NR-, -NRC(O)NR-, - OC(O)S-, -OC(O)O-, -NRC(O)O-, -SC(O)-, -C(O)S-, -NR-, -C(O)NR-, -NRC(O)-, -NRC(O)S-, -SC(O)NR-, -C(O)-, -OC(S)-, -C(S)O-, -OC(S)NR-, -NRC(S)O-, -S-S-, and -S(O)₀₋₂-;
Q is selected from a chemical bond, -C(O)O-, -O-, -SC(O)O-, -OC(O)NRₐ-, -NRₐC(O)NR'ₐ-, -OC(O)S-, - OC(O)O-, -NRₐC(O)O-, -OC(O)-, -SC(O)-, -C(O)S-, -NRₐ-, -C(O)NRₐ-, -NRₐC(O)-, -NRₐC(O)S-, - SC(O)NRₐ-, -C(O)-, -OC(S)-, -C(S)O-, -OC(S)NRₐ-, -NRₐC(S)O-, -S-S-, and -S(O)₀₋₂-;
Rₐ and R'ₐ are independently selected from H, C₁₋₁₀ alkyl, C₃₋₁₄ cycloalkyl, and 3- to 14-membered heterocyclyl;
R₁ and R₂ are independently selected from C₄₋₂₀ alkyl, C₄₋₂₀ alkenyl, and C₄₋₂₀ alkynyl, which are optionally substituted with one or more Rₛ and in which one or more methylene units are optionally and independently replaced by -NR'-;
the substitution site of Rₛ on R₁ or R₂ is separated from M₁ or M₂ by 0-12 carbon atoms;
R and R' are each independently selected from H and C₁₋₂₀ alkyl;
Rₛ is independently selected from H, C₁₋₂₀ alkyl, -L_{b}-OR_{b}, -L_{b}-SR_{b}, and -L_{b}-NR_{b}R'_{b};
L_{b} is independently selected from a chemical bond and C₁₋₂₀ alkylene;
R_{b} and R'_{b} are independently selected from H, C₁₋₂₀ alkyl, C₃₋₁₄ cycloalkyl, and 3- to 14-membered heterocyclyl; R₃ is selected from CN, -OR_{c}, -C(O)R_{c}, -OC(O)R_{c}, -NR"C(O)R_{c}, -NRₑR'_{c}, -NR"C(O)NR_{c}R'_{c}, -NR"C(O)R_{c}, - NR"S(O)₂R_{c}, -OC(O)NR_{c}R'_{c}, -NR"C(O)OR_{c}, -N(OR_{c})C(O)R_{c}, -N(OR_{c})S(O)₂R_{c}, -N(OR_{c})C(O)OR_{c}, - N(OR_{c})C(O)R_{c}R'_{c}, 3- to 14-membered heterocyclyl, and 5- to 14-membered heteroaryl;
R_{c} and R'_{c} are independently selected from H, C₁₋₁₀ alkyl, C₃₋₁₀ cycloalkyl, and 3- to 10-membered heterocyclyl;
R" is independently selected from H and C₁₋₆ alkyl;
R₄ and R₅ are independently C₁₋₅ alkyl, which is optionally substituted with one or more R*;
or R₄ and R₅, together with the carbon atom to which they are connected, form C₃₋₁₄ cycloalkylene or 3- to 14-membered heterocyclylene, which is optionally substituted with one or more R*;
R* is independently selected from H, halogen, cyano, C₁₋₈ alkyl, C₁₋₅ haloalkyl, -L_{d}-OR_{d}, -L_{d}-SR_{d}, and -L_{d}-NR_{d}R'_{d};
L_{d} is independently selected from a chemical bond and C₁₋₈ alkylene;
R_{d} and R'_{d} are independently selected from H, C₁₋₅ alkyl, C₃₋₁₄ cycloalkyl, and 3- to 14-membered heterocyclyl.

2. The compound of formula (I), or the isotopic variant, tautomer, or stereoisomer thereof, or the pharmaceutically acceptable salt thereof according to claim 1, wherein a = 2, 3, 4, 5, or 6; preferably, a = 2, 3, 4, or 5; preferably, a = 2, 3, or 4; preferably, a = 2 or 3; preferably, a = 3 or 4; preferably, a = 2; preferably, a = 3; preferably, a = 4.

3. The compound of formula (I), or the isotopic variant, tautomer, or stereoisomer thereof, or the pharmaceutically acceptable salt thereof according to claim 1 or 2, wherein b = 4, 5, 6, 7, 8, or 9; preferably, b = 5, 6, 7, or 8; preferably, b = 6, 7, or 8; preferably, b = 6 or 7; preferably, b = 6 or 8; preferably, b = 7 or 8; preferably, b = 7.

4. The compound of formula (I), or the isotopic variant, tautomer, or stereoisomer thereof, or the pharmaceutically acceptable salt thereof according to any one of claims 1-3, wherein c = 2, 3, 4, 5, or 6; preferably, c = 2, 3, 5, or 6; preferably, c = 3, 4, 5, or 6; preferably, c = 2, 3, 4, or 5; preferably, c = 3, 5, or 6; preferably, c = 2, 4, or 5; preferably, c = 2 or 4; preferably, c = 2 or 6; preferably, c = 5 or 6; preferably, c = 2; preferably, c = 3; preferably, c = 5; preferably, c = 6.

5. The compound of formula (I), or the isotopic variant, tautomer, or stereoisomer thereof, or the pharmaceutically acceptable salt thereof according to any one of claims 1-4, wherein d = 1, 2, 3, 4, or 5; preferably, d = 0, 1, 2, 3, or 4; preferably, d = 1, 2, 3, or 4; preferably, d = 0, 1, 2, or 3; preferably, d = 1, 2, or 4; preferably, d = 0, 1, or 2; preferably, d = 0 or 1; preferably, d = 2 or 4; preferably, d = 0 or 3; preferably, d = 0; preferably, d = 1; preferably, d = 3; preferably, d = 4.

6. The compound of formula (I), or the isotopic variant, tautomer, or stereoisomer thereof, or the pharmaceutically acceptable salt thereof according to any one of claims 1-5, wherein c + d = 5, 6, or 7; preferably, c + d = 6 or 7; preferably, c + d = 5 or 6; preferably, c + d = 6.

7. The compound of formula (I), or the isotopic variant, tautomer, or stereoisomer thereof, or the pharmaceutically acceptable salt thereof according to any one of claims 1-6, wherein M₁ and M₂ are independently selected from -C(O)O-, -C(O)S-, -OC(O)-, -SC(O)-, -OC(O)O-, -C(O)NR-, and -NRC(O)-, preferably selected from -C(O)O-, -C(O)S-, -OC(O)-, -SC(O)-, and -OC(O)O-, and preferably selected from - C(O)O-, -C(O)S-, -OC(O)-, and -SC(O)-;
preferably, M₁ and M₂ are independently selected from -C(O)O-, -OC(O)-, and -OC(O)O-; preferably, they are not both -OC(O)O-; preferably, they are not both -OC(O)-; preferably, they are selected from -C(O)O- and -OC(O)-;
preferably, M₁ is -C(O)O- or -OC(O)-, preferably -C(O)O-; M₂ is -C(O)O-;
preferably, M₁ is -OC(O)- or -SC(O)-; M₂ is -C(O)O- or -C(O)S-;
preferably, M₁ is -OC(O)-; M₂ is -C(O)O-;
preferably, M₁ and M₂ are independently selected from -C(O)O- and -C(O)S-, preferably -C(O)O-;
preferably, one of M₁ and M₂ is -OC(O)O-, and the other is selected from -C(O)O-, -C(O)S-, -OC(O)-, and - SC(O)-, preferably -C(O)O- and -OC(O)-; preferably, M₁ is selected from -C(O)O- and OC(O)-, preferably - C(O)O-, and M₂ is -OC(O)O-.

8. The compound of formula (I), or the isotopic variant, tautomer, or stereoisomer thereof, or the pharmaceutically acceptable salt thereof according to any one of claims 1-7, wherein Q is selected from a chemical bond, -OC(O)-, and -SC(O)-, preferably a chemical bond or -SC(O)-, and preferably -SC(O)- or - OC(O)-.

9. The compound of formula (I), or the isotopic variant, tautomer, or stereoisomer thereof, or the pharmaceutically acceptable salt thereof according to any one of claims 1-8, wherein Rₐ and R'ₐ are independently selected from H, C₁₋₆ alkyl, C₃₋₁₀ cycloalkyl, and 3- to 10-membered heterocyclyl, preferably selected from H and C₁₋₆ alkyl, and preferably selected from H and C₁₋₄ alkyl.

10. The compound of formula (I), or the isotopic variant, tautomer, or stereoisomer thereof, or the pharmaceutically acceptable salt thereof according to any one of claims 1-9, wherein R₁ and R₂ are independently selected from C₆₋₁₄ alkyl, C₆₋₁₄ alkenyl, and C₆₋₁₄ alkynyl, preferably selected from C₆₋₁₄ alkyl; preferably, R₁ and R₂ are independently selected from C₈₋₁₂ alkyl, C₈₋₁₂ alkenyl, and C₈₋₁₂ alkynyl, preferably C₈₋₁₀ alkyl, C₈₋₁₀ alkenyl, and C₈₋₁₀ alkynyl;
preferably, R₁ and R₂ are independently C₈₋₁₂ alkyl, preferably C₈₋₁₁ alkyl, preferably C₉₋₁₁ alkyl, preferably C₈₋₁₀ alkyl, preferably C₉₋₁₀ alkyl, and preferably C₉ alkyl;
preferably, R₁ and R₂ are optionally substituted with 1, 2, 3, or 4 Rₛ, and preferably, optionally substituted with 1 Rₛ;
preferably, R₁ and R₂ are not both substituted; preferably, one of R₁ and R₂ is substituted, and the other is unsubstituted; preferably, R₁ is unsubstituted, and R₂ is substituted; preferably, R₁ is substituted, and R₂ is unsubstituted;
preferably, R₁ and R₂ are independently selected from the following groups: -(CH₂)₇CH₃, -(CH₂)₈CH₃, - (CH₂)₉CH₃, -(CH₂)₁₀CH₃, 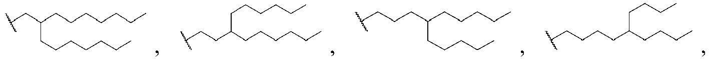 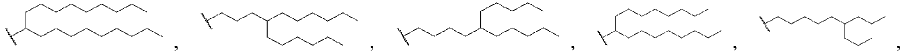 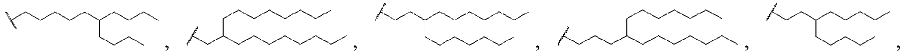 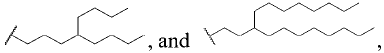
preferably selected from -(CH₂)₇CH₃, -(CH₂)₈CH₃, -(CH₂)₉CH₃, - (CH₂)₁₀CH₃, 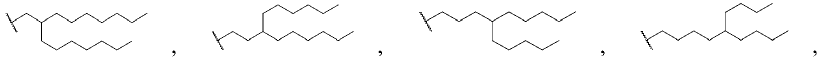 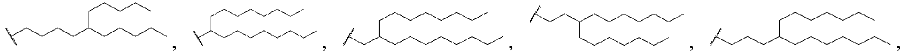
and

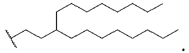

11. The compound of formula (I), or the isotopic variant, tautomer, or stereoisomer thereof, or the pharmaceutically acceptable salt thereof according to any one of claims 1-10, wherein R and R' are each independently selected from H and C₁₋₁₄ alkyl, preferably selected from H and C₁₋₁₀ alkyl, and preferably selected from H and C₁₋₆ alkyl; preferably, R is H.

12. The compound of formula (I), or the isotopic variant, tautomer, or stereoisomer thereof, or the pharmaceutically acceptable salt thereof according to any one of claims 1-11, wherein Rₛ is independently selected from H, C₁₋₁₄ alkyl, -L_{b}-OR_{b}, and -L_{b}-NR_{b}R'_{b}, preferably H and C₁₋₁₄ alkyl, preferably H and C₁₋₁₂ alkyl, preferably H and C₁₋₁₀ alkyl, preferably H and C₁₋₉ alkyl, preferably H and C₃₋₉ alkyl, preferably H and C₁₋₈ alkyl, preferably H and C₃₋₈ alkyl, preferably H and C₄₋₈ alkyl, preferably H and C₅₋₈ alkyl, preferably H and C₆₋₈ alkyl, preferably H and C₁₋₇ alkyl, preferably H and C₃₋₇ alkyl, preferably H and C₄₋₇ alkyl, preferably H and C₅₋₇ alkyl, preferably H and C₁₋₆ alkyl, preferably H and C₅₋₆ alkyl, preferably H and C₁₋₅ alkyl, preferably H and C₄₋₅ alkyl, and preferably H and C₅ alkyl;
preferably, Rₛ is independently preferably selected from C₁₋₁₄ alkyl, -L_{b}-OR_{b}, and -L_{b}-NR_{b}R'_{b}, preferably C₁₋₁₄ alkyl, preferably C₁₋₁₂ alkyl, preferably C₁₋₁₀ alkyl, preferably C₁₋₉ alkyl, preferably C₃₋₉ alkyl, preferably C₁₋₈ alkyl, preferably C₃₋₈ alkyl, preferably C₄₋₈ alkyl, preferably C₅₋₈ alkyl, preferably C₆₋₈ alkyl, preferably C₁₋₇ alkyl, preferably C₃₋₇ alkyl, preferably C₄₋₇ alkyl, preferably C₅₋₇ alkyl, preferably C₁₋₆ alkyl, preferably C₅₋₆ alkyl, preferably C₁₋₅ alkyl, preferably C₄₋₅ alkyl, and preferably C₅ alkyl.

13. The compound of formula (I), or the isotopic variant, tautomer, or stereoisomer thereof, or the pharmaceutically acceptable salt thereof according to any one of claims 1-12, wherein L_{b} is independently selected from a chemical bond and C₁₋₁₄ alkylene, preferably selected from C₁₋₁₀ alkylene, and preferably selected from C₁₋₆ alkylene;
preferably, R_{b} and R'_{b} are independently selected from H and C₁₋₁₄ alkyl, preferably selected from H and C₁₋₁₀ alkyl, and preferably selected from H and C₁₋₆ alkyl.

14. The compound of formula (I), or the isotopic variant, tautomer, or stereoisomer thereof, or the pharmaceutically acceptable salt thereof according to any one of claims 1-13, wherein R₃ is selected from CN, -OR_{c}, and -NR_{c}R'_{c}, preferably selected from -OR_{c} and -NR_{c}R'_{c}, preferably -NR_{c}R'_{c}, preferably -N(CH₃)₂, preferably -OR_{c}, and preferably OH.

15. The compound of formula (I), or the isotopic variant, tautomer, or stereoisomer thereof, or the pharmaceutically acceptable salt thereof according to any one of claims 1-14, wherein R_{c} and R'_{c} are independently selected from H, C₁₋₁₀ alkyl, C₃₋₇ cycloalkyl, and 3- to 7-membered heterocyclyl, preferably selected from H and C₁₋₁₀ alkyl, preferably selected from H and C₁₋₆ alkyl, and preferably selected from H and C₁₋₄ alkyl (e.g., methyl).

16. The compound of formula (I), or the isotopic variant, tautomer, or stereoisomer thereof, or the pharmaceutically acceptable salt thereof according to any one of claims 1-15, wherein R₄ and R₅ are independently C₁₋₆ alkyl, preferably C₁₋₃ alkyl, preferably C₁₋₂ alkyl, and preferably methyl;
preferably, R₄ and R₅ are optionally substituted with 1, 2, or 3 R*;
preferably, R₄ and R₅, together with the carbon atom to which they are connected, form C₃₋₁₀ cycloalkylene or 3- to 10-membered heterocyclylene, preferably form C₃₋₆ cycloalkylene or 3- to 6-membered heterocyclylene, preferably form C₃₋₆ cycloalkylene (e.g., cyclopropylene, cyclobutylene, cyclopentylene, or cyclohexylene), preferably form C₃₋₄ cycloalkylene, and preferably form cyclopropylene;
preferably, the ring formed by R₄, R₅, and the carbon atom to which they are connected is optionally substituted with 1, 2, or 3 R*.

17. The compound of formula (I), or the isotopic variant, tautomer, or stereoisomer thereof, or the pharmaceutically acceptable salt thereof according to any one of claims 1-16, wherein R* is independently selected from H, halogen, cyano, C₁₋₆ alkyl, C₁₋₆ haloalkyl, -L_{d}-OR_{d}, -L_{d}-SR_{d}, and -L_{d}-NR_{d}R'_{d}, preferably selected from H, halogen, cyano, C₁₋₆ alkyl, and C₁₋₆ haloalkyl, and preferably selected from H, C₁₋₃ alkyl, and C₁₋₃ haloalkyl.

18. The compound of formula (I), or the isotopic variant, tautomer, or stereoisomer thereof, or the pharmaceutically acceptable salt thereof according to any one of claims 1-17, wherein L_{d} is independently selected from a chemical bond and C₁₋₆ alkylene, preferably selected from a chemical bond and C₁₋₃ alkylene; preferably, R_{d} and R'_{d} are independently selected from H, C₁₋₆ alkyl, C₃₋₁₀ cycloalkyl, and 3- to 10-membered heterocyclyl, preferably selected from H and C₁₋₆ alkyl.

19. The compound of formula (I), or the isotopic variant, tautomer, or stereoisomer thereof, or the pharmaceutically acceptable salt thereof according to any one of claims 1-18, wherein the substitution site of Rₛ on R₁ or R₂ is separated from M₁ or M₂ by 0-10 carbon atoms, preferably 0-6 carbon atoms, preferably 0-5 carbon atoms, preferably 0-4 carbon atoms, preferably 0-3 carbon atoms, and preferably 0-2 carbon atoms; preferably, the substitution site of Rₛ on R₁ or R₂ is separated from M₁ or M₂ by at least 2 carbon atoms, preferably 2-4 carbon atoms, preferably 2-3 carbon atoms, and preferably 3-4 carbon atoms;
preferably, the substitution site of Rₛ on R₁ or R₂ is separated from M₁ or M₂ by at least 1 carbon atom, preferably 1-5 carbon atoms, preferably 1-4 carbon atoms, preferably 1-3 carbon atoms, and preferably 3 carbon atoms.

20. The compound of formula (I), or the isotopic variant, tautomer, or stereoisomer thereof, or the pharmaceutically acceptable salt thereof according to any one of claims 1-19: 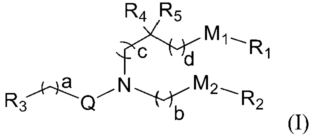 wherein,
a = 1, 2, 3, 4, 5, or 6;
b = 4, 5, 6, 7, 8, 9, or 10;
c = 1, 2, 3, 4, 5, or 6;
d = 1, 2, 3, 4, or 5;
c + d = 3, 4, 5, 6, 7, 8, or 9;
M₁ is -OC(O)- or -SC(O)-;
M₂ is -C(O)O- or -C(O)S-;
Q is selected from a chemical bond, -OC(O)-, and -SC(O)-;
R₁ and R₂ are independently selected from C₆₋₁₄ alkyl, C₆₋₁₄ alkenyl, and C₆₋₁₄ alkynyl;
one of R₁ and R₂ is substituted with 1, 2, 3, or 4 Rₛ, and the other is unsubstituted;
the substitution site of Rₛ on R₁ or R₂ is separated from M₁ or M₂ by at least 1 carbon atom;
Rₛ is independently selected from C₁₋₁₄ alkyl, -L_{b}-OR_{b}, and -L_{b}-NR_{b}R'_{b}, preferably C₁₋₁₄ alkyl;
L_{b} is independently selected from a chemical bond and C₁₋₁₄ alkylene;
R_{b} and R'_{b} are independently selected from H and C₁₋₁₄ alkyl;
R₃ is selected from -OR_{c} and -NR_{c}R'_{c};
R_{c} and R'_{c} are independently selected from H or C₁₋₆ alkyl;
R₄ and R₅ are independently C₁₋₆ alkyl;
or R₄ and R₅, together with the carbon atom to which they are connected, form C₃₋₆ cycloalkylene or 3- to 6-membered heterocyclylene.

21. The compound of formula (I), or the isotopic variant, tautomer, or stereoisomer thereof, or the pharmaceutically acceptable salt thereof according to claim 20, wherein
a = 2, 3, 4, or 5;
b = 5, 6, 7, or 8;
c = 1, 2, 3, 4, 5, or 6;
d = 1, 2, 3, 4, or 5;
c + d = 5, 6, or 7;
M₁ is -OC(O)- or -SC(O)-;
M₂ is -C(O)O- or -C(O)S-;
Q is selected from a chemical bond, -OC(O)-, and -SC(O)-;
R₁ and R₂ are independently selected from C₈₋₁₂ alkyl, C₈₋₁₂ alkenyl, C₈₋₁₂ alkynyl, and preferably C₈₋₁₂ alkyl;
one of R₁ and R₂ is substituted with 1 Rₛ, and the other is unsubstituted;
the substitution site of Rₛ on R₁ or R₂ is separated from M₁ or M₂ by at least 1 carbon atom;
Rₛ is C₁₋₁₂ alkyl;
R₃ is selected from -OR_{c} and -NR_{c}R'_{c};
R_{c} and R'_{c} are independently H and C₁₋₆ alkyl;
R₄ and R₅ are independently C₁₋₃ alkyl;
or R₄ and R₅, together with the carbon atom to which they are connected, form C₃₋₆ cycloalkylene.

22. The compound of formula (I), or the isotopic variant, tautomer, or stereoisomer thereof, or the pharmaceutically acceptable salt thereof according to claim 20 or 21, wherein
a = 2, 3, or 4;
b = 6, 7, or 8;
c = 2, 3, 4, 5, or 6;
d = 1, 2, 3, 4, or 5;
c + d = 6 or 7;
M₁ is -OC(O)-; M₂ is -C(O)O-;
Q is a chemical bond or -SC(O)-;
R₁ and R₂ are independently C₈₋₁₂ alkyl, preferably C₈₋₁₁ alkyl, and preferably C₉₋₁₁ alkyl;
one of R₁ and R₂ is substituted with 1 Rₛ, and the other is unsubstituted;
the substitution site of Rₛ on R₁ or R₂ is separated from M₁ or M₂ by at least 1 carbon atom, preferably 1-4 carbon atoms;
Rₛ is C₁₋₁₀ alkyl, preferably C₁₋₈ alkyl, preferably C₃₋₈ alkyl, and preferably C₄₋₈ alkyl;
R₃ is selected from -OR_{c} and -NR_{c}R'_{c}, preferably -OH and -N(CH₃)₂;
R_{c} and R'_{c} are independently H and C₁₋₄ alkyl, preferably H and methyl;
R₄ and R₅ are independently C₁₋₃ alkyl, preferably methyl;
or R₄ and R₅, together with the carbon atom to which they are connected, form C₃₋₄ cycloalkylene, preferably cyclopropylene;
preferably, one of R₁ and R₂ is selected from -(CH₂)₇CH₃, -(CH₂)₈CH₃, -(CH₂)₉CH₃, and -(CH₂)₁₀CH₃,
preferably -(CH₂)₈CH₃, -(CH₂)₉CH₃, and -(CH₂)₁₀CH₃; the other is selected from: 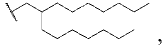 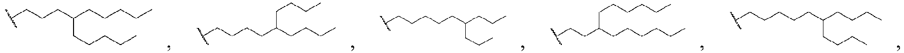 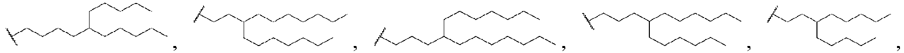 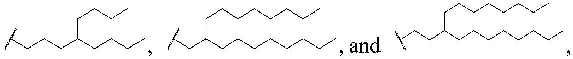 preferably selected from:

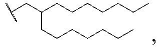

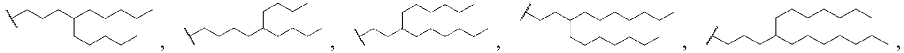

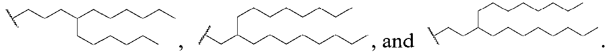

23. The compound of formula (I), or the isotopic variant, tautomer, or stereoisomer thereof, or the pharmaceutically acceptable salt thereof according to any one of claims 1-22, having a structure of formula (II) or formula (III): 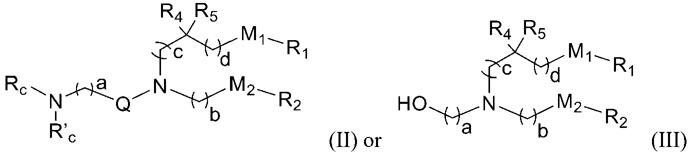 wherein the variables are as defined in any one of claims 1-22.

24. The compound of formula (II), or the isotopic variant, tautomer, or stereoisomer thereof, or the pharmaceutically acceptable salt thereof according to claim 23, 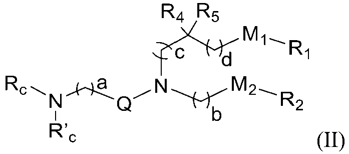 wherein,
a = 2, 3, 4, 5, or 6; preferably, a = 2, 3, 4, or 5;
b = 4, 5, 6, 7, 8, or 9; preferably, b = 5, 6, 7, or 8;
c = 1, 2, 3, 4, 5, or 6;
d = 1, 2, 3, 4, or 5;
c + d = 5, 6, or 7;
M₁ and M₂ are independently selected from -C(O)O-, -C(O)S-, -OC(O)-, -SC(O)-, and -OC(O)O-, preferably selected from -C(O)O-, -C(O)S-, -OC(O)-, and -SC(O)-;
Q is -SC(O)- or -OC(O)-;
R₁ and R₂ are independently C₆₋₁₄ alkyl, which is optionally substituted with 1, 2, 3, or 4 Rₛ;
the substitution site of Rₛ on R₁ or R₂ is separated from M₁ or M₂ by 0-5 carbon atoms;
Rₛ is independently selected from C₁₋₁₄ alkyl, -L_{b}-OR_{b}, and -L_{b}-NR_{b}R'_{b}, preferably C₁₋₁₄ alkyl;
L_{b} is independently selected from a chemical bond and C₁₋₁₄ alkylene;
R_{b} and R'_{b} are independently selected from H and C₁₋₁₄ alkyl;
R_{c} and R'_{c} are independently C₁₋₆ alkyl;
R₄ and R₅ are independently C₁₋₆ alkyl;
or R₄ and R₅, together with the carbon atom to which they are connected, form C₃₋₆ cycloalkylene or 3- to 6-membered heterocyclylene.

25. The compound of formula (II), or the isotopic variant, tautomer, or stereoisomer thereof, or the pharmaceutically acceptable salt thereof according to claim 24, wherein
a = 3 or 4;
b = 6 or 7;
c = 2, 3, 4, or 5; preferably, c = 2, 4, or 5;
d = 1, 2, 3, or 4; preferably, d = 1, 2, or 4;
c + d = 6;
M₁ and M₂ are independently selected from -C(O)O-, -OC(O)-, and -OC(O)O-; preferably, they are not both - OC(O)O-; preferably, they are not both -OC(O)-; preferably, they are selected from -C(O)O- and -OC(O)-;
Q is -SC(O)-;
R₁ and R₂ are independently C₈₋₁₂ alkyl, preferably C₈₋₁₀ alkyl;
one of R₁ and R₂ is substituted with 1 Rₛ, and the other is unsubstituted;
the substitution site of Rₛ on R₁ or R₂ is separated from M₁ or M₂ by 0-4 carbon atoms;
Rₛ is C₁₋₁₂ alkyl, preferably C₁₋₉ alkyl, preferably C₃₋₉ alkyl, preferably C₁₋₈ alkyl, and preferably C₅₋₈ alkyl;
R_{c} and R'_{c} are independently C₁₋₄ alkyl, preferably methyl;
R₄ and R₅ are independently C₁₋₃ alkyl, preferably methyl;
or R₄ and R₅, together with the carbon atom to which they are connected, form C₃₋₆ cycloalkylene, preferably C₃₋₄ cycloalkylene, e.g., cyclopropylene;
preferably, R₁ and R₂ are independently selected from: -(CH₂)₇CH₃, -(CH₂)₈CH₃, -(CH₂)₉CH₃, 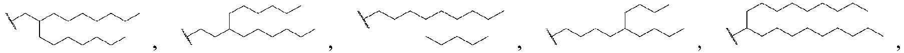 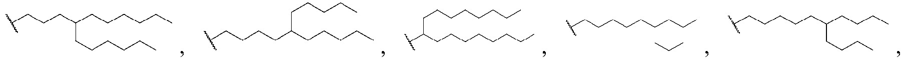 and 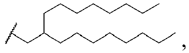 preferably selected from -(CH₂)₇CH₃, -(CH₂)₈CH₃, -(CH₂)₉CH₃, 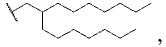 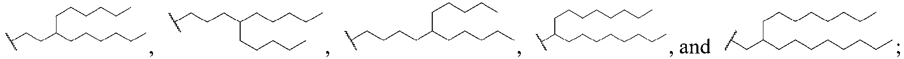
preferably, R₁ is unsubstituted, and R₂ is substituted.

26. The compound of formula (II), or the isotopic variant, tautomer, or stereoisomer thereof, or the pharmaceutically acceptable salt thereof according to claim 24, wherein
a = 3 or 4; preferably, a = 4;
b = 6 or 7; preferably, b = 7;
c = 2 or 4, preferably, c = 2;
d = 2 or 4; preferably, d = 4;
c + d = 6;
M₁ is -C(O)O- or -OC(O)-, preferably -C(O)O-; M₂ is -C(O)O-;
Q is -SC(O)-;
R₁ is selected from C₈₋₁₂ linear alkyl, preferably C₉₋₁₀ linear alkyl;
R₂ is selected from C₈₋₁₂ alkyl, preferably C₉₋₁₀ alkyl, which is substituted with 1 Rₛ;
the substitution site of Rₛ on R₂ is separated from M₂ by at least 2 carbon atoms;
Rₛ is C₁₋₆ alkyl, preferably C₅₋₆ alkyl, preferably C₁₋₅ alkyl, and preferably C₅ alkyl;
R_{c} and R'_{c} are independently C₁₋₄ alkyl, preferably methyl;
R₄ and R₅ are independently C₁₋₃ alkyl, preferably methyl;
preferably, the substitution site of Rₛ on R₂ is separated from M₂ by 2-4 carbon atoms, preferably 3-4 carbon atoms;
preferably, R₂ is selected from: 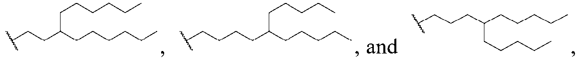 preferably

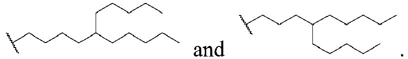

27. The compound of formula (II), or the isotopic variant, tautomer, or stereoisomer thereof, or the pharmaceutically acceptable salt thereof according to claim 24, wherein
a = 3 or 4; preferably, a = 4;
b = 7;
c = 5;
d = 1;
M₁ is -OC(O)-; M₂ is -C(O)O-;
Q is -SC(O)-;
R₁ is selected from C₈₋₁₂ linear alkyl, preferably C₉₋₁₀ linear alkyl, and preferably C₉ linear alkyl;
R₂ is selected from C₈₋₁₂ alkyl, preferably C₉₋₁₀ alkyl, and preferably C₉ alkyl, which is substituted with 1 Rₛ; the substitution site of Rₛ on R₂ is separated from M₂ by 0-3 carbon atoms;
Rₛ is C₁₋₈ alkyl, preferably C₅₋₈ alkyl;
R_{c} and R'_{c} are independently C₁₋₄ alkyl, preferably methyl;
R₄ and R₅ are independently C₁₋₃ alkyl, preferably methyl;
or R₄ and R₅, together with the carbon atom to which they are connected, form C₃₋₄ cycloalkylene, e.g., cyclopropylene;
preferably, R₂ is selected from:

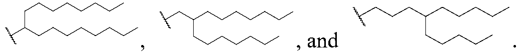

28. The compound of formula (III), or the isotopic variant, tautomer, or stereoisomer thereof, or the pharmaceutically acceptable salt thereof according to claim 23, 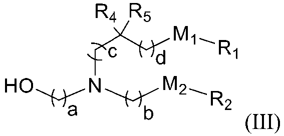 wherein,
a = 2, 3, 4, 5, or 6; preferably, a = 2, 3, 4, or 5;
b = 4, 5, 6, 7, 8, or 9; preferably, b = 5, 6, 7, or 8;
c = 1, 2, 3, 4, 5, or 6;
d = 1, 2, 3, 4, or 5;
c + d = 5, 6, or 7;
M₁ is -OC(O)- or -SC(O)-; M₂ is -C(O)O- or -C(O)S-;
R₁ and R₂ are independently C₆₋₁₄ alkyl, which is optionally substituted with 1, 2, 3, or 4 Rₛ;
the substitution site of Rₛ on R₁ or R₂ is separated from M₁ or M₂ by at least 1 carbon atom, preferably 1-5 carbon atoms;
Rₛ is independently selected from H, C₁₋₁₄ alkyl, -L_{b}-OR_{b}, and -L_{b}-NR_{b}R'_{b}, preferably selected from H and C₁₋₁₄ alkyl;
L_{b} is independently selected from a chemical bond and C₁₋₁₄ alkylene;
R_{b} and R'_{b} are independently H and C₁₋₁₄ alkyl;
R₄ and R₅ are independently C₁₋₆ alkyl;
or R₄ and R₅, together with the carbon atom to which they are connected, form C₃₋₆ cycloalkylene or 3- to 6-membered heterocyclylene.

29. The compound of formula (III), or the isotopic variant, tautomer, or stereoisomer thereof, or the pharmaceutically acceptable salt thereof according to claim 28, wherein
a = 2, 3, or 4;
b = 6, 7, or 8;
c = 2, 3, 4, 5, or 6; preferably, c = 2, 3, 5, or 6;
d = 1, 2, 3, or 4;
c + d = 6 or 7;
M₁ is -OC(O)-; M₂ is -C(O)O-;
R₁ and R₂ are independently C₈₋₁₂ alkyl, preferably C₈₋₁₁ alkyl, and preferably C₉₋₁₁ alkyl;
one of R₁ and R₂ is substituted with 1 Rₛ, and the other is unsubstituted;
the substitution site of Rₛ on R₁ or R₂ is separated from M₁ or M₂ by 1-5 carbon atoms, preferably 1-4 carbon atoms;
Rₛ is C₁₋₁₂ alkyl, preferably C₁₋₁₀ alkyl, preferably C₁₋₈ alkyl, preferably C₃₋₈ alkyl, and preferably C₄₋₈ alkyl; R₄ and R₅ are independently C₁₋₃ alkyl, preferably methyl;
or R₄ and R₅, together with the carbon atom to which they are connected, form C₃₋₆ cycloalkylene, preferably C₃₋₄ cycloalkylene, e.g., cyclopropylene;
preferably, R₁ and R₂ are independently selected from: -(CH₂)₇CH₃, -(CH₂)₈CH₃, -(CH₂)₉CH₃, -(CH₂)₁₀CH₃, 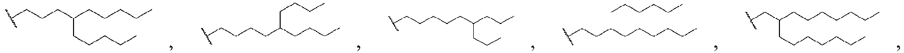 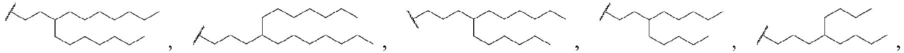 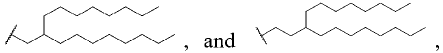 preferably selected from -(CH₂)₈CH₃, -(CH₂)₉CH₃, - (CH₂)₁₀CH₃, 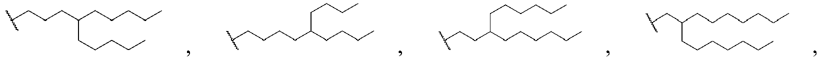 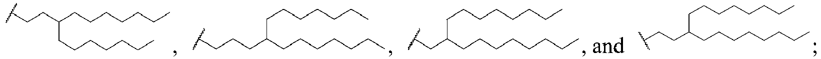
preferably, R₁ is unsubstituted, and R₂ is substituted.

30. The compound of formula (III), or the isotopic variant, tautomer, or stereoisomer thereof, or the pharmaceutically acceptable salt thereof according to claim 28, wherein
a = 2, 3, or 4; preferably, a = 2 or 3;
b = 6 or 7; preferably, b = 7;
c = 5;
d = 1;
M₁ is -OC(O)-; M₂ is -C(O)O-;
R₁ and R₂ are independently C₈₋₁₂ alkyl, preferably C₈₋₁₀ alkyl, preferably C₉₋₁₀ alkyl, and preferably C₉ alkyl; one of R₁ and R₂ is substituted with 1 Rₛ, and the other is unsubstituted;
the substitution site of Rₛ on R₁ or R₂ is separated from M₁ or M₂ by at least 1 carbon atom;
Rₛ is C₁₋₁₂ alkyl, preferably C₁₋₁₀ alkyl, preferably C₁₋₇ alkyl, preferably C₃₋₇ alkyl, preferably C₄₋₇ alkyl, and preferably C₅₋₇ alkyl;
R₄ and R₅ are independently C₁₋₃ alkyl, preferably methyl;
or R₄ and R₅, together with the carbon atom to which they are connected, form C₃₋₆ cycloalkylene, preferably C₃₋₄ cycloalkylene, e.g., cyclopropylene; preferably, R₄ and R₅, together with the carbon atom to which they are connected, do not form a ring;
preferably, the substitution site of Rₛ on R₁ or R₂ is separated from M₁ or M₂ by 1-4 carbon atoms, preferably 1-3 carbon atoms;
preferably, one of R₁ and R₂ is selected from C₉₋₁₀ linear alkyl, preferably C₉ linear alkyl, and the other is selected from: 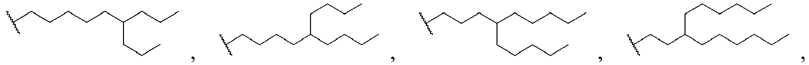 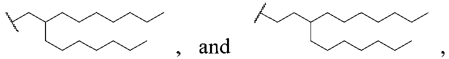 preferably 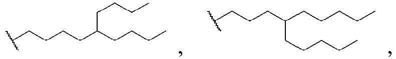 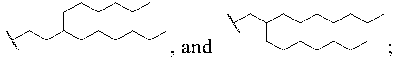
preferably,
R₁ is unsubstituted, and R₂ is substituted;
R₁ is selected from C₈₋₁₀ linear alkyl, preferably C₉₋₁₀ linear alkyl, and preferably C₉ linear alkyl;
R₂ is selected from C₈₋₁₀ alkyl, preferably C₉₋₁₀ alkyl, and preferably C₉ alkyl, which is substituted with 1 Rₛ; preferably,
R₁ is substituted, and R₂ is unsubstituted;
R₁ is selected from C₈₋₁₀ alkyl, preferably C₉₋₁₀ alkyl, and preferably C₉ alkyl, which is substituted with 1 Rₛ;
R₂ is selected from C₈₋₁₀ linear alkyl, preferably C₉₋₁₀ linear alkyl, and preferably C₉ linear alkyl;
Rₛ is C₁₋₁₀ alkyl, preferably C₁₋₅ alkyl, preferably C₄₋₅ alkyl, and preferably C₅ alkyl.

31. The compound of formula (III), or the isotopic variant, tautomer, or stereoisomer thereof, or the pharmaceutically acceptable salt thereof according to claim 28, wherein
a = 2, 3, or 4; preferably, a = 3 or 4;
b = 6, 7, or 8; preferably, b = 6 or 8;
c = 5 or 6;
d = 1 or 2;
c + d = 7;
M₁ is -OC(O)-; M₂ is -C(O)O-;
R₁ is selected from C₈₋₁₂ linear alkyl, preferably C₈₋₁₀ linear alkyl, preferably C₈₋₉ linear alkyl, and preferably C₉ linear alkyl;
R₂ is selected from C₈₋₁₂ alkyl, preferably C₉₋₁₁ alkyl, which is substituted with 1 Rₛ;
the substitution site of Rₛ on R₁ or R₂ is separated from M₁ or M₂ by at least 2 carbon atoms, preferably 2-3 carbon atoms;
Rₛ is C₁₋₁₂ alkyl, preferably C₁₋₁₀ alkyl, preferably C₁₋₇ alkyl, and preferably C₆₋₇ alkyl;
R₄ and R₅ are independently C₁₋₃ alkyl, preferably methyl;
or R₄ and R₅, together with the carbon atom to which they are connected, form C₃₋₆ cycloalkylene, preferably C₃₋₄ cycloalkylene, e.g., cyclopropylene; preferably, R₄ and R₅, together with the carbon atom to which they are connected, do not form a ring;
preferably, R₂ is selected from:

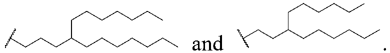

32. The compound of formula (III), or the isotopic variant, tautomer, or stereoisomer thereof, or the pharmaceutically acceptable salt thereof according to claim 28, wherein
a = 2, 3, or 4; preferably, a = 3;
b = 7 or 8; preferably, b = 7;
c = 3;
d = 3;
M₁ is -OC(O)-; M₂ is -C(O)O-;
R₁ is selected from C₈₋₁₂ linear alkyl, preferably C₉₋₁₁ linear alkyl;
R₂ is selected from C₈₋₁₂ alkyl, preferably C₉₋₁₁ alkyl, which is substituted with 1 Rₛ;
the substitution site of Rₛ on R₂ is separated from M₂ by at least 1 carbon atoms;
Rₛ is C₁₋₁₂ alkyl, preferably C₁₋₁₀ alkyl, preferably C₁₋₈ alkyl, and preferably C₆₋₈ alkyl;
R₄ and R₅ are independently C₁₋₃ alkyl, preferably methyl;
or R₄ and R₅, together with the carbon atom to which they are connected, form C₃₋₆ cycloalkylene, preferably C₃₋₄ cycloalkylene, e.g., cyclopropylene;
preferably, the substitution site of Rₛ on R₂ is separated from M₂ by 1-3 carbon atoms;
preferably, R₂ is selected from:

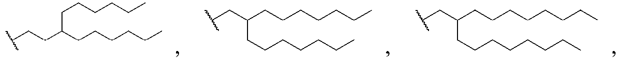

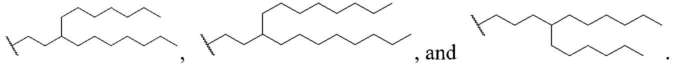

33. The compound of formula (III), or the isotopic variant, tautomer, or stereoisomer thereof, or the pharmaceutically acceptable salt thereof according to claim 28, wherein
a = 2, 3, or 4; preferably, a = 3;
b = 7 or 8; preferably, b = 7;
c=2;
d=4;
M₁ is -OC(O)-; M₂ is -C(O)O-;
R₁ is selected from C₈₋₁₂ linear alkyl, preferably C₉₋₁₁ linear alkyl, and preferably C₁₀ linear alkyl;
R₂ is selected from C₈₋₁₂ alkyl, preferably C₉₋₁₁ alkyl, and preferably C₁₀ alkyl, which is substituted with 1 Rₛ; the substitution site of Rₛ on R₂ is separated from M₂ by at least 1 carbon atoms;
Rₛ is C₁₋₁₂ alkyl, preferably C₁₋₁₀ alkyl, preferably C₁₋₈ alkyl, and preferably C₆₋₈ alkyl;
R₄ and R₅ are independently C₁₋₃ alkyl, preferably methyl;
or R₄ and R₅, together with the carbon atom to which they are connected, form C₃₋₆ cycloalkylene, preferably C₃₋₄ cycloalkylene, e.g., cyclopropylene; preferably, R₄ and R₅, together with the carbon atom to which they are connected, do not form a ring;
preferably, the substitution site of Rₛ on R₂ is separated from M₂ by 1-3 carbon atoms;
preferably, R₂ is selected from: 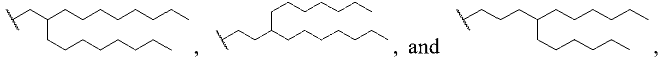
preferably

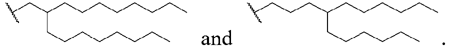

34. The compound of formula (III), or the isotopic variant, tautomer, or stereoisomer thereof, or the pharmaceutically acceptable salt thereof according to claim 23, 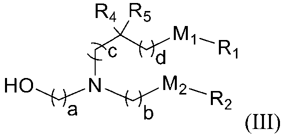 wherein,
a = 2, 3, 4, 5, or 6; preferably, a = 2, 3, or 4;
b = 4, 5, 6, 7, 8, or 9; preferably, b = 5, 6, 7, or 8;
c = 1, 2, 3, 4, 5, or 6;
d = 0, 1, 2, 3, 4, or 5;
c + d = 5, 6, or 7;
M₁ and M₂ are independently selected from -C(O)O- and -C(O)S-, preferably -C(O)O-; or one of M₁ and M₂ is -OC(O)O-, and the other is selected from -C(O)O-, -C(O)S-, -OC(O)-, and -SC(O)-, preferably -C(O)O- and -OC(O)-;
R₁ and R₂ are independently C₆₋₁₄ alkyl, which is optionally substituted with 1, 2, 3, or 4 Rₛ;
the substitution site of Rₛ on R₁ or R₂ is separated from M₁ or M₂ by 0-4 carbon atoms;
Rₛ is independently selected from H, C₁₋₁₄ alkyl, -L_{b}-OR_{b}, and -L_{b}-NR_{b}R'_{b}, preferably selected from H and C₁₋₁₄ alkyl;
L_{b} is independently selected from a chemical bond and C₁₋₁₄ alkylene;
R_{b} and R'_{b} are independently selected from H and C₁₋₁₄ alkyl;
R₄ and R₅ are independently C₁₋₆ alkyl;
or R₄ and R₅, together with the carbon atom to which they are connected, form C₃₋₆ cycloalkylene or 3- to 6-membered heterocyclylene.

35. The compound of formula (III), or the isotopic variant, tautomer, or stereoisomer thereof, or the pharmaceutically acceptable salt thereof according to claim 34, wherein
a = 2, 3, or 4; preferably, a = 2;
b = 5, 6, or 7; preferably, b = 5 or 7;
c = 2, 3, 4, 5, or 6; preferably, c = 2 or 6; preferably, c = 6;
d = 0, 1, 2, or 3; preferably, d = 0 or 3; preferably, d = 0;
c + d = 5 or 6; preferably, c + d = 6;
M₁ and M₂ are -C(O)O-;
R₁ is selected from C₈₋₁₂ alkyl, preferably C₈₋₁₀ alkyl, preferably C₉₋₁₀ alkyl, and preferably C₉ alkyl, which is substituted with 1 Rₛ;
the substitution site of Rₛ on R₁ is separated from M₁ by at least 1 carbon atoms;
R₂ is selected from C₈₋₁₂ linear alkyl, preferably C₉₋₁₁ linear alkyl;
Rₛ is C₁₋₁₂ alkyl, preferably C₁₋₁₀ alkyl, preferably C₁₋₅ alkyl, and preferably C₅ alkyl;
R₄ and R₅ are independently C₁₋₃ alkyl, preferably methyl;
or R₄ and R₅, together with the carbon atom to which they are connected, form C₃₋₆ cycloalkylene, preferably C₃₋₄ cycloalkylene, e.g., cyclopropylene; preferably, R₄ and R₅, together with the carbon atom to which they are connected, do not form a ring;
preferably, the substitution site of Rₛ on R₁ is separated from M₁ by 1-3 carbon atoms, preferably 3 carbon atoms;
preferably, R₁ is

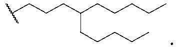

36. The compound of formula (III), or the isotopic variant, tautomer, or stereoisomer thereof, or the pharmaceutically acceptable salt thereof according to claim 34, wherein
a = 2, 3, or 4; preferably, a = 3 or 4;
b = 5, 6, or 7; preferably, b = 6 or 7;
c = 3, 4, 5, or 6; preferably, c = 3, 5, or 6; preferably, c = 5 or 6; preferably, c = 6;
d = 0, 1, or 2; preferably, d = 0 or 1; preferably, d = 0;
c + d = 5 or 6; preferably, c + d = 6;
one of M₁ and M₂ is -OC(O)O-, and the other is selected from -C(O)O- and -OC(O)-; preferably, M₁ is selected from -C(O)O- and OC(O)-, preferably -C(O)O-, and M₂ is -OC(O)O-;
R₁ and R₂ are independently C₈₋₁₂ alkyl, preferably C₈₋₁₀ alkyl, preferably C₉₋₁₀ alkyl, and preferably C₉ alkyl; one of R₁ and R₂ is substituted with 1 Rₛ, and the other is unsubstituted;
the substitution site of Rₛ on R₁ or R₂ is separated from M₁ or M₂ by 0-4 carbon atoms;
Rₛ is C₁₋₁₂ alkyl, preferably C₁₋₁₀ alkyl, preferably C₁₋₈ alkyl, and preferably C₆₋₈ alkyl;
R₄ and R₅ are independently C₁₋₃ alkyl, preferably methyl;
or R₄ and R₅, together with the carbon atom to which they are connected, form C₃₋₆ cycloalkylene, preferably C₃₋₄ cycloalkylene, e.g., cyclopropylene; preferably, R₄ and R₅, together with the carbon atom to which they are connected, do not form a ring;
preferably, the substitution site of Rₛ on R₁ or R₂ is separated from M₁ or M₂ by 0-3 carbon atoms, preferably 0-2 carbon atoms;
preferably, one of R₁ and R₂ is C₉ linear alkyl, and the other is selected from: 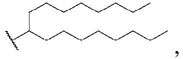 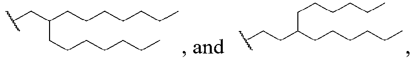 preferably

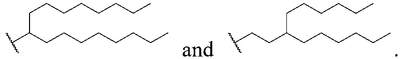

37. The compound of formula (I), or the isotopic variant, tautomer, or stereoisomer thereof, or the pharmaceutically acceptable salt thereof according to claim 1, wherein the compound is selected from compounds in Table 1.

38. A pharmaceutical composition, comprising the compound, or the isotopic variant, tautomer, or stereoisomer thereof, or the pharmaceutically acceptable salt thereof according to any one of claims 1-37, and a pharmaceutically acceptable auxiliary material.

39. A nanoparticle composition, comprising a lipid component, and optionally a load, wherein the lipid component comprises the compound, or the isotopic variant, tautomer, or stereoisomer thereof, or the pharmaceutically acceptable salt thereof according to any one of claims 1-37, and the load is selected from one or more of a therapeutic agent, a prophylactic agent, and a diagnostic agent;
preferably, the lipid component comprises the following components in molar percentage:
20 mol%-85 mol% of the compound according to any one of claims 1-37;
10 mol%-75 mol% of a structural lipid;
1.0 mol%-30 mol% of a neutral lipid;
0.25 mol%-10 mol% of a polymeric lipid;
preferably, the lipid component comprises the following components in molar percentage:
50 mol% of the compound according to any one of claims 1-37;
10 mol% of a neutral lipid;
38.5 mol% of a structural lipid;
1.5 mol% of a polymeric lipid.

40. Use of the compound, or the isotopic variant, tautomer, or stereoisomer thereof, or the pharmaceutically acceptable salt thereof according to any one of claims 1-37, or the pharmaceutical composition according to claim 38, or the nanoparticle composition according to claim 39 in the manufacture of a medicament for treating, diagnosing, or preventing a disease, wherein preferably, the medicament for treating, diagnosing, or preventing a disease is a therapeutic or prophylactic mRNA vaccine.

41. Use of the compound, or the isotopic variant, tautomer, or stereoisomer thereof, or the pharmaceutically acceptable salt thereof according to any one of claims 1-37, or the pharmaceutical composition according to claim 38, or the nanoparticle composition according to claim 39 in the manufacture of a medicament for delivering a load, wherein the load is selected from one or more of a therapeutic agent, a prophylactic agent, and a diagnostic agent.

42. A method for treating, diagnosing, or preventing a disease in a subject, comprising administering to the subject the pharmaceutical composition according to claim 38 or the nanoparticle composition according to claim 39.

43. The compound, or the isotopic variant, tautomer, or stereoisomer thereof, or the pharmaceutically acceptable salt thereof according to any one of claims 1-37, or the pharmaceutical composition according to claim 38, or the nanoparticle composition according to claim 39 for use in treating, diagnosing, or preventing a disease.

44. A method for delivering a load into a subject's body, comprising administering to the subject the pharmaceutical composition according to claim 38 or the nanoparticle composition according to claim 39, wherein the load is selected from one or more of a therapeutic agent, a prophylactic agent, and a diagnostic agent.

45. The compound, or the isotopic variant, tautomer, or stereoisomer thereof, or the pharmaceutically acceptable salt thereof according to any one of claims 1-37, or the pharmaceutical composition according to claim 38, or the nanoparticle composition according to claim 39 for use in delivering a load,
wherein the load is selected from one or more of a therapeutic agent, a prophylactic agent, and a diagnostic agent.

46. The nanoparticle composition according to claim 39, or the use according to claim 41 or 45, or the method according to claim 44, wherein the therapeutic agent, the prophylactic agent, or the diagnostic agent is a nucleic acid;
preferably, the nucleic acid is selected from one or more of ASO, RNA, and DNA;
preferably, the RNA is selected from one or more of a small interfering RNA (siRNA), a short hairpin RNA (shRNA), an antisense RNA (aRNA), a messenger RNA (mRNA), a long non-coding RNA (lncRNA), a micro RNA (miRNA), a small activating RNA (saRNA), a multimeric coding nucleic acid (MCNA), a polymeric coding nucleic acid (PCNA), a guide RNA (gRNA), a CRISPR RNA (crRNA), and a ribozyme, preferably an mRNA, and more preferably a modified mRNA.

47. A method for preparing a compound of formula (II), comprising:
reacting a compound of formula (IIA) with a compound of formula (IIB) to give the compound of formula (II), 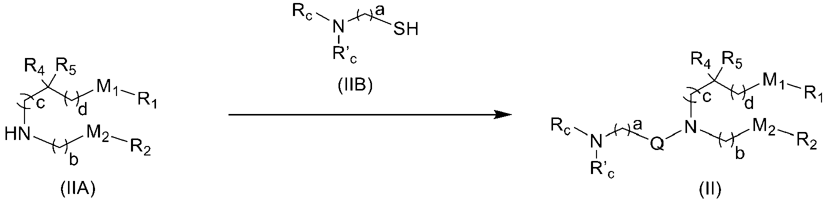
wherein the variables are as defined in any one of claims 1-37.

48. A method for preparing a compound of formula (III), comprising:
reacting a compound of formula (IIIA) with a compound of formula (IIIB) to give the compound of formula (III); 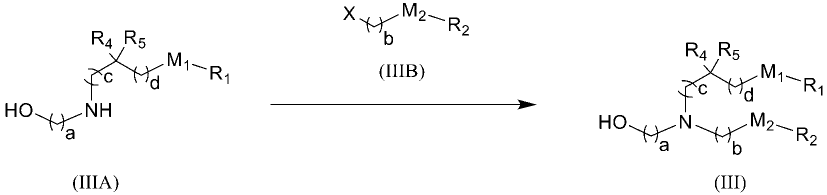
or reacting a compound of formula (IIIC) with a compound of formula (IIID) to give the compound of formula (II), 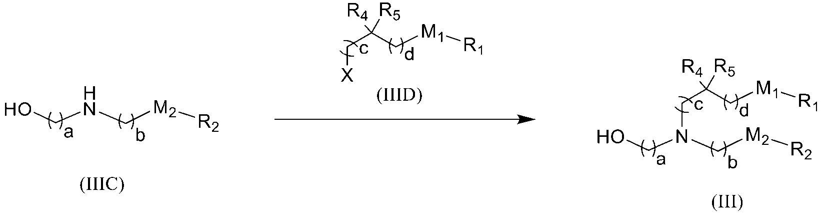
wherein X is halogen, and the other variables are as defined in any one of claims 1-37.
